# EUROPEAN PATENT SPECIFICATION

(11) **EP 3 010 919 B1**
(45) Date of publication and mention of the grant of the patent: **12.04.2017**
(21) Application number: 14733588.9
(22) Date of filing: 20.06.2014
(51) Int. Cl.: C07D 417/10, C07D 417/12, C07D 471/04, C07D 279/02, A61K 31/541, A61P 37/00, A61P 19/02

(54) **ARYL SULTAM DERIVATIVES AS ROR-C MODULATORS**
ARYLSULTAMDERIVATE ALS ROR-C-MODULATOREN
DÉRIVÉS D'ARYLE SULTAME UTILISÉS EN TANT QUE MODULATEURS DE ROR-C

(30) Priority: 21.06.2013 US 201361837757 P
(43) Date of publication of application: 27.04.2016
(73) Proprietor: F. Hoffmann-La Roche AG, 4070 Basel (CH)
(72) Inventor: FAUBER, Benjamin, South San Francisco, California 94080 (US); RENE, Olivier, South San Francisco, California 94080 (US)
(74) Representative: Bernard, Guillaume
(86) International application number: PCT/EP2014/062983
(87) International publication number: WO 2014/202741

(56) References cited:
- WO-A1-2013/064231
- WO-A2-2012/064744

## Description

### CROSS REFERENCE TO RELATED APPLICATIONS

This application claims the benefit of priority of U.S. Provisional Patent Application Serial No. 61/837,757 filed on June 21, 2013.

### FIELD OF THE INVENTION

The invention pertains to compounds that modulate the function of retinoid-receptor related orphan receptor RORc (RORγ) and use of such compounds for treatment of autoimmune diseases

### BACKGROUND OF THE INVENTION

T helper 17 cells (Th17) are interleukin (IL)-17 secreting CD4+ T cells involved in pathogenesis of autoimmune diseases such as rheumatoid arthritis, irritable bowel disease, psoriasis, psoriatic arthritis and spondyloarthridities. The retinoic acid-related orphan receptor γ (RORγ or RORc) is recognized as a transcription factor necessary for Th17 cell differentiation. RORc is an orphan member of the nuclear hormone receptor subfamily that includes RORα (RORa) and RORβ (RORb). RORc controls gene transcription by binding to DNA as a monomer. Selective modulation of RORc has been proposed as a route to discovery and development of Th17 cell-associated autoimmune diseases. WO2012064744 discloses tetrahydroquinoline and related bicyclic compounds for inhibition of RORγ activity.

There is accordingly a need for compounds that inhibit RORc for use in treatment of autoimmune diseases such as rheumatoid arthritis, irritable bowel disease, psoriasis, psoriatic arthritis and spondyloarthridities.

### SUMMARY OF THE INVENTION

The invention provides compounds of the formula I: 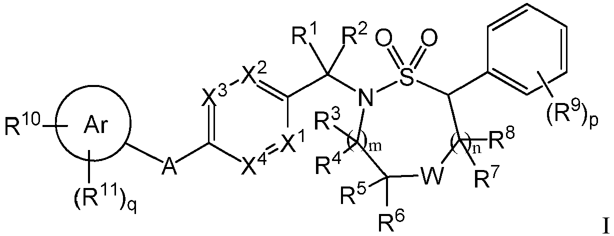 or a pharmaceutically acceptable salt thereof,
wherein:
m is 0 or 1;
n is 0 or 1;
p is from 0 to 3;
q is from 0 to 2;
t is from 0 to 4;
v is 0 or 1,
w is from 0 to 2;
Ar is mono- or bicyclic aryl or heteroaryl;
A is: a bond; -(CR^{j}R^{k})ₜ-; -C(O)-(CR^{j}R^{k})ₜ-; -(CR^{j}R^{k})ₜ-C(O)-; -NR^{a}-(CR^{j}R^{k})ₜ-; -(CR^{j}R^{k})ₜ-NR^{a}-; -C(O)NR^{a}-(CR^{j}R^{k})ₜ-; -(CR^{j}R^{k})ₜ-NR^{a}C(O)-; -O-(CR^{j}R^{k})ₜ-; -(CR^{j}R^{k})ₜ-O-; -S-(CR^{j}R^{k})ₜ; -(CR^{j}R^{k})ₜ-S-; -SO₂-(CR^{j}R^{k})ₜ-; or -(CR^{j}R^{k})ₜ-SO₂-;
W is: -CR^{b}R^{c}-; -O-; -S-; -SO₂-; or -NR^{d}-;
one of X¹, X², X³ and X⁴ is N and the others are CR^{e}; or two of X¹, X², X³ and X⁴ are N and the others are CR^{e}; or three of X¹, X², X³ and X⁴ are N and the other is CR^{e}; or each of X¹, X², X³ and X⁴ is CR^{e};
R¹, R², R³, R⁴, R⁵, R⁶, R⁷ and R⁸ each independently is: hydrogen; or C₁₋₆alkyl which may be unsubstituted or substituted one or more times with halo;
or R³ and R⁴ together with the atom to which they are attached may form an ethylene group;
or R³ and R⁴ together with the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
or R⁵ and R⁶ together with the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
or R⁷ and R⁸ together with the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
or one of R³ and R⁴ together with one of R⁵ and R⁶ and the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
or one of R⁵ and R⁶ together with one of R⁷ and R⁸ and the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
each R⁹ is independently: C₁₋₆alkyl; halo; C₁₋₆alkoxy; or cyano; wherein the C₁₋₆alkyl moieties may be unsubstituted or substituted one or more times with halo;
R¹⁰ is: hydrogen; C₁₋₆alkyl; cyano; -(CH₂)ᵥ-NR^{f}R^{g}; -(CH₂)ᵥ-S(O)_{w}-R^{h}; -(CH₂)ᵥ-C(O)-NR^{f}R^{g}; -(CH₂)ᵥ-S(O)_{w}-NR^{f}R^{g}; -(CH₂)ᵥ-NR^{f}-C(O)-R^{h}; -(CH₂)ᵥ-NR^{f}-C(O)-NR^{f}R^{g}; or -(CH₂)ᵥ-NR^{f}- S(O)_{w}-R^{h};
each R¹¹ is independently: C₁₋₆alkyl; halo; C₁₋₆alkoxy; cyano; halo-C₁₋₆alkyl; hydroxy-C₁₋₆alkyl; halo-C₁₋₆alkoxy; or C₁₋₆alkylsulfonyl;
R^{a}, R^{b}, R^{c}, R^{d} each independent is: hydrogen; or C₁₋₆alkyl which may be unsubstituted or substituted one or more times with halo;
or R^{b} and R^{c} together with the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
or one of R^{b} and R^{c} together with one of R⁷ and R⁸ and the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
or one of R^{b} and R^{c} together with one of R⁵ and R⁶ and the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
each R^{e} is independently: hydrogen; C₁₋₆alkyl; halo; C₁₋₆alkoxy; or cyano; wherein the C₁₋₆alkyl moieties may be unsubstituted or substituted one or more times with halo;
R^{f} and R^{g} each independently is: hydrogen; or C₁₋₆alkyl which may be unsubstituted or substituted one or more times with halo;
R^{h} is: C₁₋₆alkyl; C₃₋₆cycloalkyl; or C₃₋₆cycloalkyl-C₁₋₆alkyl, each of which may be unsubstituted or substituted one or more times with halo;
Rⁱ is: C₁₋₆alkyl; halo; oxo; hydroxy; acetyl; or C₁₋₆alkoxy; wherein the C₁₋₆alkyl moieties may be unsubstituted or substituted one or more times with halo; and
R^{j} and R^{k} each independent is: hydrogen; or C₁₋₆alkyl which may be unsubstituted or substituted one or more times with halo;
provided that when Ar is imidazolyl substituted with methyl, A is a bond, and each of X¹, X², X³ and X⁴ is CR^{e}, then R^{e} is not methoxy.

The invention also provides and pharmaceutical compositions comprising the compounds, methods of using the compounds, and methods of preparing the compounds.

### DETAILED DESCRIPTION OF THE INVENTION

### Definitions

Unless otherwise stated, the following terms used in this Application, including the specification and claims, have the definitions given below. It must be noted that, as used in the specification and the appended claims, the singular forms "a", "an," and "the" include plural referents unless the context clearly dictates otherwise.

"Alkyl" means the monovalent linear or branched saturated hydrocarbon moiety, consisting solely of carbon and hydrogen atoms, having from one to twelve carbon atoms. "Lower alkyl" refers to an alkyl group of one to six carbon atoms, i.e. C₁-C₆alkyl. Examples of alkyl groups include, but are not limited to, methyl, ethyl, propyl, isopropyl, isobutyl, sec-butyl, tert-butyl, pentyl, n-hexyl, octyl, dodecyl, and the like.

"Alkenyl" means a linear monovalent hydrocarbon radical of two to six carbon atoms or a branched monovalent hydrocarbon radical of three to six carbon atoms, containing at least one double bond, *e.g*., ethenyl, propenyl, and the like.

"Alkynyl" means a linear monovalent hydrocarbon radical of two to six carbon atoms or a branched monovalent hydrocarbon radical of three to six carbon atoms, containing at least one triple bond, *e.g*., ethynyl, propynyl, and the like.

"Alkylene" means a linear saturated divalent hydrocarbon radical of one to six carbon atoms or a branched saturated divalent hydrocarbon radical of three to six carbon atoms, e.g., methylene, ethylene, 2,2-dimethylethylene, propylene, 2-methylpropylene, butylene, pentylene, and the like. "Alkoxy" and "alkyloxy", which may be used interchangeably, mean a moiety of the formula-OR, wherein R is an alkyl moiety as defined herein. Examples of alkoxy moieties include, but are not limited to, methoxy, ethoxy, isopropoxy, and the like.

"Alkoxyalkyl" means a moiety of the formula R^{a}-O-R^{b}-, where R^{a} is alkyl and R^{b} is alkylene as defined herein. Exemplary alkoxyalkyl groups include, by way of example, 2-methoxyethyl, 3-methoxypropyl, 1-methyl-2-methoxyethyl, 1-(2-methoxyethyl)-3-methoxypropyl, and 1-(2-methoxyethyl)-3-methoxypropyl.

"Alkoxyalkoxy' means a group of the formula -O-R-R' wherein R is alkylene and R' is alkoxy as defined herein.

"Alkylcarbonyl" means a moiety of the formula -C(O)-R, wherein R is alkyl as defined herein. "Alkoxycarbonyl" means a group of the formula -C(O)-R wherein R is alkoxy as defined herein. "Alkylcarbonylalkyl" means a group of the formula -R-C(O)-R wherein R is alkylene and R' is alkyl as defined herein.

"Alkoxyalkylcarbonyl" means a moiety of the formula -C(O)-R-R', wherein R is alkylene and R' is alkoxy as defined herein.

"Alkoxycarbonylalkyl" means a group of the formula -R-C(O)-R wherein R is alkylene and R' is alkoxy as defined herein.

"Alkoxycarbonylalkoxy"means a group of the formula -O-R-C(O)-R' wherein R is alkylene and R' is alkoxy as defined herein.

"Hydroxycarbonylalkoxy" means a group of the formula -O-R-C(O)-OH wherein R is alkylene as defined herein.

"Alkylaminocarbonylalkoxy" means a group of the formula -O-R-C(O)-NHR' wherein R is alkylene and R' is alkyl as defined herein.

"Dialkylaminocarbonylalkoxy" means a group of the formula -O-R-C(O)-NR'R" wherein R is alkylene and R' and R" are alkyl as defined herein.

"Alkylaminoalkoxy" means a group of the formula -O-R-NHR' wherein R is alkylene and R' is alkyl as defined herein.

"Dialkylaminoalkoxy" means a group of the formula -O-R-NR'R' wherein R is alkylene and R' and R" are alkyl as defined herein.

"Alkylsulfonyl" means a moiety of the formula - SO₂-R, wherein R is alkyl as defined herein. "Alkylsulfonylalkyl means a moiety of the formula -R'-SO₂-R" where where R' is alkylene and R" is alkyl as defined herein.

"Alkylsulfonylalkoxy" means a group of the formula -O-R-SO₂-R' wherein R is alkylene and R' is alkyl as defined herein.

"Amino means a moiety of the formula -NRR' wherein R and R' each independently is hyrdogen or alkyl as defined herein. "Amino thus includes "alkylamino (where one of R and R' is alkyl and the other is hydrogen) and "dialkylamino (where R and R' are both alkyl. "Aminocarbonyl" means a group of the formula -C(O)-R wherein R is amino as defined herein. "N-hydroxy-aminocarbonyl" means a group of the formula -C(O)-NR-OH wherein R is hydrogen or alkyl as defined herein.

"N-alkoxy-aminocarbonyl" means a group of the formula -C(O)-NR-R' wherein R is hydrogen or alkyl and R' is alkoxy as defined herein.

"N-alkyl-aminocarbonyl means a group of the formula -C(O)-NH-R wherein R is alkyl as defined herein.

"N-hydroxy-N-alkylaminocarbonyl means a group of the formula -C(O)-NRR' wherein R is alkyl as defined herein and R' is hydroxy.

"N-alkoxy-N-alkylaminocarbonyl" means a group of the formula -C(O)-NRR' wherein R is alkyl and R' is alkoxy as defined herein.

"N,N-di-C₁₋₆alkyl-aminocarbonyl" means a group of the formula -C(O)-NRR' wherein R and R' are alkyl as defined herein.

"Aminosulfonyl" means a group of the formula -SO₂-NH₂.

"N-alkylaminosulfonyl" means a group of the formula -SO₂-NHR wherein R is alkyl as defined herein.

"N,N-dialkylaminosulfonyl" means a group of the formula -SO₂-NRR' wherein R and R' are alkyl as defined herein.

"Alkylsulfonylamino" means a group of the formula -NR'-SO₂-R wherein R id alkyl and R' is hydrogen or alkyl as defined herein.

"N-(alkylsulfonyl)-aminoalkyl" means a group of the formula -R-NH-SO₂-R' wherein R is alkylene and R' is alkyl as defined herein.

"N-(Alkylsulfonyl)aminocarbonyl" means a group of the formula -C(O)-NH-SO₂-R wherein wherein R is alkyl as defined herein.

"N-(Alkylsulfonyl)-N-alkylaminocarbonyl" means a group of the formula -C(O)-NR-SO₂-R' wherein wherein R and R' are alkyl as defined herein.

"N-Alkoxyalkyl-aminocarbonyl" means a group of the formula -C(O)-NR-R'-OR" wherein R is hydrogen or alkyl, R' is alkylene, and R" is alkyl as defined herein.

"N-Hydroxyalkyl-aminocarbonyl" means a group of the formula -C(O)-NR-R'-OH" wherein R is hydrogen or alkyl and R' is alkylene as defined herein.

"Alkoxyamino" means a moiety of the formula -NR-OR' wherein R is hydrogen or alkyl and R' is alkyl as defined herein.

"Alkylsulfanyl" means a moiety of the formula -SR wherein R is alkyl as defined herein. "Aminoalkyl" means a group -R-R' wherein R' is amino and R is alkylene as defined herein. "Aminoalkyl" includes aminomethyl, aminoethyl, 1-aminopropyl, 2-aminopropyl, and the like. The amino moiety of "aminoalkyl" may be substituted once or twice with alkyl to provide "alkylaminoalkyl" and "dialkylaminoalkyl" respectively. "Alkylaminoalkyl" includes methylaminomethyl, methylaminoethyl, methylaminopropyl, ethylaminoethyl and the like. "Dialkylaminoalkyl" includes dimethylaminomethyl, dimethylaminoethyl, dimethylaminopropyl, N-methyl-N-ethylaminoethyl, and the like.

"Aminoalkoxy" means a group -OR-R' wherein R' is amino and R is alkylene as defined herein. "Alkylsulfonylamido" means a moiety of the formula -NR'SO₂-R wherein R is alkyl and R' is hydrogen or alkyl.

"Aminocarbonyloxyalkyl" or "carbamylalkyl" means a group of the formula -R-O-C(O)-NR'R" wherein R is alkylene and R', R" each independently is hydrogen or alkyl as defined herein. "Alkynylalkoxy" means a group of the formula -O-R-R' wherein R is alkylene and R' is alkynyl as defined herein.

"Aryl" means a monovalent cyclic aromatic hydrocarbon moiety consisting of a mono-, bi- or tricyclic aromatic ring. The aryl group can be optionally substituted as defined herein. Examples of aryl moieties include, but are not limited to, phenyl, naphthyl, phenanthryl, fluorenyl, indenyl, pentalenyl, azulenyl, oxydiphenyl, biphenyl, methylenediphenyl, aminodiphenyl, diphenylsulfidyl, diphenylsulfonyl, diphenylisopropylidenyl, benzodioxanyl, benzofuranyl, benzodioxylyl, benzopyranyl, benzoxazinyl, benzoxazinonyl, benzopiperadinyl, benzopiperazinyl, benzopyrrolidinyl, benzomorpholinyl, methylenedioxyphenyl, ethylenedioxyphenyl, and the like, of which may be optionally substituted as defined herein. "Arylalkyl" and "Aralkyl", which may be used interchangeably, mean a radical-R^{a}R^{b} where R^{a} is an alkylene group and R^{b} is an aryl group as defined herein; *e.g*., phenylalkyls such as benzyl, phenylethyl, 3-(3-chlorophenyl)-2-methylpentyl, and the like are examples of arylalkyl. "Arylsulfonyl means a group of the formula -SO₂-R wherein R is aryl as defined herein. "Aryloxy" means a group of the formula -O-R wherein R is aryl as defined herein. "Aralkyloxy" means a group of the formula -O-R-R" wherein R is alkylene and R' is aryl as defined herein.

"Carboxy" or "hydroxycarbonyl", which may be used interchangeably, means a group of the formula -C(O)-OH.

"Cyanoalkyl" " means a moiety of the formula -R'-R", where R' is alkylene as defined herein and R" is cyano or nitrile.

"Cycloalkyl" means a monovalent saturated carbocyclic moiety consisting of mono- or bicyclic rings. Particular cycloalkyl are unsubstituted or substituted with alkyl. Cycloalkyl can optionally be substituted as defined herein. Unless defined otherwise, cycloalkyl may be optionally substitued with one or more substituents, wherein each substituent is independently hydroxy, alkyl, alkoxy, halo, haloalkyl, amino, monoalkylamino, or dialkylamino. Examples of cycloalkyl moieties include, but are not limited to, cyclopropyl, cyclobutyl, cyclopentyl, cyclohexyl, cycloheptyl, and the like, including partially unsaturated (cycloalkenyl) derivatives thereof.

"Cycloalkenyl" means a cycloalkyl as defined herein that includes at least one double bond or unsaturation. Exemplary cycloalkenyl include cyclohexenyl, cyclopentenyl, cyclobutenyl and the like.

"Cycloalkylalkyl" means a moiety of the formula -R'-R", where R' is alkylene and R" is cycloalkyl as defined herein.

"Cycloalkylalkoxy" means a group of the formula -O-R-R' wherein R is alkylene and R' is cycloalkyl as defined herein.

"Cycloalkylcarbonyl" means a moiety of the formula -C(O)-R, wherein R is cycloalkyl as defined herein.

"C₃₋₆cycloalkyl-C₁₋₆alkyl-carbonyl" means a moiety of the formula -C(O)-R, wherein R is cycloalkylalkyl as defined herein.

"Cyanoalkylcarbonyl" means a moiety of the formula -C(O)-R-R', wherein R is alkylene as defined herein and R' is cyano or nitrile.

"N-Cyano-aminocarbonyl" means a moiety of the formula -C(O)-NHR, wherein R is cyano or nitrile.

"N-Cyano-N-alkyl-aminocarbonyl" means a moiety of the formula -C(O)-NRR'-R, wherein R' is alkyl as defined herein and R is cyano or nitrile.

"Cycloalkylsulfonyl" means a group of the formula -SO₂-R wherein R is cycloalkyl as defined herein.

"Cycloalkylalkylsulfonyl" means a group of the formula -SO₂-R wherein R is cycloalkylalkyl as defined herein.

"Formyl" means a moiety of the formula -C(O)-H.

"Heteroaryl" means a monocyclic or bicyclic radical of 5 to 12 ring atoms having at least one aromatic ring containing one, two, or three ring heteroatoms selected from N, O, or S, the remaining ring atoms being C, with the understanding that the attachment point of the heteroaryl radical will be on an aromatic ring. The heteroaryl ring may be optionally substituted as defined herein. Examples of heteroaryl moieties include, but are not limited to, optionally substituted imidazolyl, oxazolyl, isoxazolyl, thiazolyl, isothiazolyl, oxadiazolyl, thiadiazolyl, pyrazinyl, thienyl, benzothienyl, thiophenyl, furanyl, pyranyl, pyridyl, pyrrolyl, pyrazolyl, pyrimidyl, quinolinyl, isoquinolinyl, benzofuryl, benzothiophenyl, benzothiopyranyl, benzimidazolyl, benzooxazolyl, benzooxadiazolyl, benzothiazolyl, benzothiadiazolyl, benzopyranyl, indolyl, isoindolyl, triazolyl, triazinyl, quinoxalinyl, purinyl, quinazolinyl, quinolizinyl, naphthyridinyl, pteridinyl, carbazolyl, azepinyl, diazepinyl, acridinyl and the like, each of which may be optionally substituted as defined herein.

Heteroarylalkyl" or "heteroaralkyl" means a group of the formula -R-R' wherein R is alkylene and R' is heteroaryl as defined herein.

"Heteroarylsulfonyl means a group of the formula -SO₂-R wherein R is heteroaryl as defined herein.

"Heteroaryloxy" means a group of the formula -O-R wherein R is heteroaryl as defined herein. "Heteroaralkyloxy" means a group of the formula -O-R-R" wherein R is alkylene and R' is heteroaryl as defined herein.

The terms "halo", "halogen" and "halide", which may be used interchangeably, refer to a substituent fluoro, chloro, bromo, or iodo.

"Haloalkyl" means alkyl as defined herein in which one or more hydrogen has been replaced with same or different halogen. Exemplary haloalkyls include -CH₂Cl, -CH₂CF₃, -CH₂CCl₃, perfluoroalkyl (e.g., -CF₃), and the like.

"Haloalkoxy" means a moiety of the formula -OR, wherein R is a haloalkyl moiety as defined herein. An exemplary haloalkoxy is difluoromethoxy.

"Heterocycloamino" means a saturated ring wherein at least one ring atom is N, NH or N-alkyl and the remaining ring atoms form an alkylene group.

"Heterocyclyl" means a monovalent saturated moiety, consisting of one to three rings, incorporating one, two, or three or four heteroatoms (chosen from nitrogen, oxygen or sulfur). The heterocyclyl ring may be optionally substituted as defined herein. Examples of heterocyclyl moieties include, but are not limited to, optionally substituted piperidinyl, piperazinyl, morpholinyl, thiomorpholinyl, azepinyl, pyrrolidinyl, azetidinyl, tetrahydropyranyl, tetrahydrofuranyl, oxetanyl and the like. Such heterocyclyl may be optionally substituted as defined herein.

"Heterocyclylalkyl" means a moiety of the formula -R-R' wherein R is alkylene and R' is heterocyclyl as defined herein.

"Heterocyclyloxy" means a moiety of the formula -OR wherein R is heterocyclyl as defined herein.

"Heterocyclylalkoxy" means a moiety of the formula -OR-R' wherein R is alkylene and R' is heterocyclyl as defined herein.

"Hydroxyalkoxy" means a moiety of the formula -OR wherein R is hydroxyalkyl as defined herein.

"Hydroxyalkylamino" means a moiety of the formula -NR-R' wherein R is hydrogen or alkyl and R' is hydroxyalkyl as defined herein.

"Hydroxyalkylaminoalkyl" means a moiety of the formula -R-NR'-R" wherein R is alkylene, R' is hydrogen or alkyl, and R" is hydroxyalkyl as defined herein.

"Hydroxycarbonylalkyl" or "carboxyalkyl" means a group of the formula -R-(CO)-OH where R is alkylene as defined herein.

"Hydroxycarbonylalkoxy" means a group of the formula -O-R-C(O)-OH wherein R is alkylene as defined herein.

"Hydroxyalkylcarbonyl" means a moiety of the formula -C(O)-R-R', wherein R is alkylene as defined herein and R' is hydroxy.

"Hydroxyalkyloxycarbonylalkyl" or "hydroxyalkoxycarbonylalkyl" means a group of the formula -R-C(O)-O-R-OH wherein each R is alkylene and may be the same or different. "Hydroxyalkyl" means an alkyl moiety as defined herein, substituted with one or more, for example, one, two or three hydroxy groups, provided that the same carbon atom does not carry more than one hydroxy group. Representative examples include, but are not limited to, hydroxymethyl, 2-hydroxyethyl, 2-hydroxypropyl, 3-hydroxypropyl, 1-(hydroxymethyl)-2-methylpropyl, 2-hydroxybutyl, 3-hydroxybutyl, 4-hydroxybutyl, 2,3-dihydroxypropyl, 2-hydroxy-1-hydroxymethylethyl, 2,3-dihydroxybutyl, 3,4-dihydroxybutyl and 2-(hydroxymethyl)-3-hydroxypropyl

"Hydroxycycloalkyl" means a cycloalkyl moiety as defined herein wherein one, two or three hydrogen atoms in the cycloalkyl radical have been replaced with a hydroxy substituent. Representative examples include, but are not limited to, 2-, 3-, or 4-hydroxycyclohexyl, and the like.

"Oxo" means a group of the formula =O (i.e., an oxygen with a double bond). Thus, for example, a 1-oxo-ethyl group is an acetyl group.

"Alkoxy hydroxyalkyl" and "hydroxy alkoxyalkyl", which may be used interchangeably, means an alkyl as defined herein that is substituted at least once with hydroxy and at least once with alkoxy. "Alkoxy hydroxyalkyl" and "hydroxy alkoxyalkyl" thus encompass, for example, 2-hydroxy-3-methoxy-propan-1-yl and the like.

"Urea"or "ureido" means a group of the formula -NR'-C(O)-NR"R"' wherein R', R" and R'" each independently is hydrogen or alkyl.

"Carbamate" means a group of the formula -O-C(O)-NR'R" wherein R' and R" each independently is hydrogen or alkyl.

"Carboxy" means a group of the formula -O-C(O)-OH.

"Sulfonamido" means a group of the formula -SO₂-NR'R" wherein R', R" and R'" each independently is hydrogen or alkyl.

"Optionally substituted" when used in association with an "aryl", phenyl", "heteroaryl" "cycloalkyl" or "heterocyclyl" moiety means that such moiety may be unsubstituted (i.e., all open valencies are occupied by a hydrogen atom) or substituted with specific groups as related herein.

"Leaving group" means the group with the meaning conventionally associated with it in synthetic organic chemistry, i.e., an atom or group displaceable under substitution reaction conditions. Examples of leaving groups include, but are not limited to, halogen, alkane- or arylenesulfonyloxy, such as methanesulfonyloxy, ethanesulfonyloxy, thiomethyl, benzenesulfonyloxy, tosyloxy, and thienyloxy, dihalophosphinoyloxy, optionally substituted benzyloxy, isopropyloxy, acyloxy, and the like.

"Modulator" means a molecule that interacts with a target. The interactions include, but are not limited to, agonist, antagonist, and the like, as defined herein.

"Optional" or "optionally" means that the subsequently described event or circumstance may but need not occur, and that the description includes instances where the event or circumstance occurs and instances in which it does not.

"Disease" and "Disease state" means any disease, condition, symptom, disorder or indication. "Inert organic solvent" or "inert solvent" means the solvent is inert under the conditions of the reaction being described in conjunction therewith, including for example, benzene, toluene, acetonitrile, tetrahydrofuran, N,N-dimethylformamide, chloroform, methylene chloride or dichloromethane, dichloroethane, diethyl ether, ethyl acetate, acetone, methyl ethyl ketone, methanol, ethanol, propanol, isopropanol, *tert*-butanol, dioxane, pyridine, and the like. Unless specified to the contrary, the solvents used in the reactions of the present invention are inert solvents.

"Pharmaceutically acceptable" means that which is useful in preparing a pharmaceutical composition that is generally safe, non-toxic, and neither biologically nor otherwise undesirable and includes that which is acceptable for veterinary as well as human pharmaceutical use. "Pharmaceutically acceptable salts" of a compound means salts that are pharmaceutically acceptable, as defined herein, and that possess the desired pharmacological activity of the parent compound.

It should be understood that all references to pharmaceutically acceptable salts include solvent addition forms (solvates) or crystal forms (polymorphs) as defined herein, of the same acid addition salt.

"Protective group" or "protecting group" means the group which selectively blocks one reactive site in a multifunctional compound such that a chemical reaction can be carried out selectively at another unprotected reactive site in the meaning conventionally associated with it in synthetic chemistry. Certain processes of this invention rely upon the protective groups to block reactive nitrogen and/or oxygen atoms present in the reactants. For example, the terms "amino-protecting group" and "nitrogen protecting group" are used interchangeably herein and refer to those organic groups intended to protect the nitrogen atom against undesirable reactions during synthetic procedures. Exemplary nitrogen protecting groups include, but are not limited to, trifluoroacetyl, acetamido, benzyl (Bn), benzyloxycarbonyl (carbobenzyloxy, CBZ), p-methoxybenzyloxycarbonyl, p-nitrobenzyloxycarbonyl, *tert*-butoxycarbonyl (BOC), and the like. The artisan in the art will know how to chose a group for the ease of removal and for the ability to withstand the following reactions.

"Solvates" means solvent additions forms that contain either stoichiometric or non stoichiometric amounts of solvent. Some compounds have a tendency to trap a fixed molar ratio of solvent molecules in the crystalline solid state, thus forming a solvate. If the solvent is water the solvate formed is a hydrate, when the solvent is alcohol, the solvate formed is an alcoholate. Hydrates are formed by the combination of one or more molecules of water with one of the substances in which the water retains its molecular state as H₂O, such combination being able to form one or more hydrate.

"Arthritis" means a disease or condition that causes damage to joints of the body and pain associated with such joint damage. Arthritis includes rheumatoid arthritis, osteoarthritis, psoriatic arthritis, septic arthritis, spondyloarthropathies, gouty arthritis, systemic lupus erythematosus and juvenile arthritis, osteoarthritis, and other arthritic conditions.

"Respiratory disorder" refers to, without limitation, chronic obstructive pulmonary disease (COPD), asthma, bronchospasm, and the like.

"Gastrointestinal disorder" ("GI disorder") refers to, without limitation, Irritable Bowel Syndrome (IBS), Inflammatory Bowel Disease (IBD), biliary colic and other biliary disorders, renal colic, diarrhea-dominant IBS, pain associated with GI distension, and the like.

"Pain" includes, without limitation, inflammatory pain; surgical pain; visceral pain; dental pain; premenstrual pain; central pain; pain due to burns; migraine or cluster headaches; nerve injury; neuritis; neuralgias; poisoning; ischemic injury; interstitial cystitis; cancer pain; viral, parasitic or bacterial infection; post-traumatic injury; or pain associated with irritable bowel syndrome. "Subject" means mammals and non-mammals. Mammals means any member of the mammalia class including, but not limited to, humans; non-human primates such as chimpanzees and other apes and monkey species; farm animals such as cattle, horses, sheep, goats, and swine; domestic animals such as rabbits, dogs, and cats; laboratory animals including rodents, such as rats, mice, and guinea pigs; and the like. Examples of non-mammals include, but are not limited to, birds, and the like. The term "subject" does not denote a particular age or sex.

"Therapeutically effective amount" means an amount of a compound that, when administered to a subject for treating a disease state, is sufficient to effect such treatment for the disease state. The "therapeutically effective amount" will vary depending on the compound, disease state being treated, the severity or the disease treated, the age and relative health of the subject, the route and form of administration, the judgment of the attending medical or veterinary practitioner, and other factors.

The terms "those defined above" and "those defined herein" when referring to a variable incorporates by reference the broad definition of the variable as well as particular definitions, if any.

"Treating" or "treatment" of a disease state includes, inter alia, inhibiting the disease state, *i.e.*, arresting the development of the disease state or its clinical symptoms, and/or relieving the disease state , *i.e.*, causing temporary or permanent regression of the disease state or its clinical symptoms.

The terms "treating", "contacting" and "reacting" when referring to a chemical reaction means adding or mixing two or more reagents under appropriate conditions to produce the indicated and/or the desired product. It should be appreciated that the reaction which produces the indicated and/or the desired product may not necessarily result directly from the combination of two reagents which were initially added, i.e., there may be one or more intermediates which are produced in the mixture
which ultimately leads to the formation of the indicated and/or the desired product.

### Nomenclature and Structures

In general, the nomenclature and chemical names used in this Application are based on ChembioOffice^{™} by CambridgeSoft^{™}. Any open valency appearing on a carbon, oxygen sulfur or nitrogen atom in the structures herein indicates the presence of a hydrogen atom unless indicated otherwise. Where a nitrogen-containing heteroaryl ring is shown with an open valency on a nitrogen atom, and variables such as R^{a}, R^{b} or R^{c} are shown on the heteroaryl ring, such variables may be bound or joined to the open valency nitrogen. Where a chiral center exists in a structure but no specific stereochemistry is shown for the chiral center, both enantiomers associated with the chiral center are encompassed by the structure. Where a structure shown herein may exist in multiple tautomeric forms, all such tautomers are encompassed by the structure. The atoms represented in the structures herein are intended to encompass all naturally occurring isotopes of such atoms. Thus, for example, the hydrogen atoms represented herein are meant to include deuterium and tritium, and the carbon atoms are meant to include C¹³ and C¹⁴ isotopes. One or more carbon atom(s) of a compound of the invention may be replaced by a silicon atom(s), and it is contemplated that one or more oxygen atom(s) of a compound of the invention may be replaced by a sulfur or selenium atom(s).

### Compounds of the Invention

The invention provides compounds of the formula I: 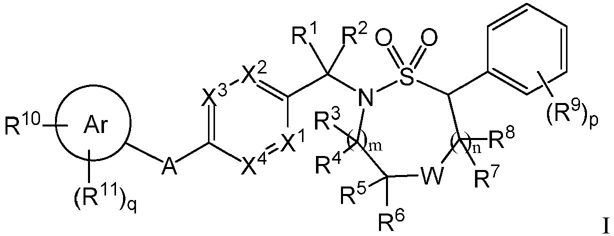 or a pharmaceutically acceptable salt thereof,
wherein:
m is 0 or 1;
n is 0 or 1;
p is from 0 to 3;
q is from 0 to 2;
t is from 0 to 4;
v is 0 or 1,
w is from 0 to 2;
Ar is mono- or bicyclic aryl or heteroaryl;
A is: a bond; -(CR^{j}R^{k})ₜ-; -C(O)-(CR^{j}R^{k})ₜ-; -(CR^{j}R^{k})ₜ-C(O)-; -NR^{a}-(CR^{j}R^{k})ₜ-; -(CR^{j}R^{k})ₜ-NR^{a}-; -C(O)NR^{a}-(CR^{j}R^{k})ₜ-; -(CR^{j}R^{k})ₜ-NR^{a}C(O)-; -O-(CR^{j}R^{k})ₜ-; -(CR^{j}R^{k})ₜ-O-; -S-(CR^{j}R^{k})ₜ; -(CR^{j}R^{k})ₜ-S-; -SO₂-(CR^{j}R^{k})ₜ-; or -(CR^{j}R^{k})ₜ-SO₂-;
W is: -CR^{b}R^{c}-; -O-; -S-; -SO₂-; or -NR^{d}-;
one of X¹, X², X³ and X⁴ is N and the others are CR^{e}; or two of X¹, X², X³ and X⁴ are N and the others are CR^{e}; or three of X¹, X², X³ and X⁴ are N and the other is CR^{e}; or each of X¹, X², X³ and X⁴ is CR^{e};
R¹, R², R³, R⁴, R⁵, R⁶, R⁷ and R⁸ each independently is: hydrogen; or C₁₋₆alkyl which may be unsubstituted or substituted one or more times with halo;
or R³ and R⁴ together with the atom to which they are attached may form an ethylene group;
or R³ and R⁴ together with the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
or R⁵ and R⁶ together with the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
or R⁷ and R⁸ together with the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
or one of R³ and R⁴ together with one of R⁵ and R⁶ and the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
or one of R⁵ and R⁶ together with one of R⁷ and R⁸ and the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
each R⁹ is independently: C₁₋₆alkyl; halo; C₁₋₆alkoxy; or cyano; wherein the C₁₋₆alkyl moieties may be unsubstituted or substituted one or more times with halo;
R¹⁰ is: hydrogen; C₁₋₆alkyl; cyano; -(CH₂)ᵥ-NR^{f}R^{g}; -(CH₂)ᵥ-S(O)_{w}-R^{h}; -(CH₂)ᵥ-C(O)-NR^{f}R^{g}; -(CH₂)ᵥ-S(O)_{w}-NR^{f}R^{g}; -(CH₂)ᵥ-NR^{f}-C(O)-R^{h}; -(CH₂)ᵥ-NR^{f}-C(O)-NR^{f}R^{g}; or -(CH₂)ᵥ-NR^{f}- S(O)_{w}-R^{h};
each R¹¹ is independently: C₁₋₆alkyl; halo; C₁₋₆alkoxy; cyano; halo-C₁₋₆alkyl; hydroxy-C₁₋₆alkyl; halo-C₁₋₆alkoxy; or C₁₋₆alkylsulfonyl;
R^{a}, R^{b}, R^{c}, R^{d} each independent is: hydrogen; or C₁₋₆alkyl which may be unsubstituted or substituted one or more times with halo;
or R^{b} and R^{c} together with the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
or one of R^{b} and R^{c} together with one of R⁷ and R⁸ and the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
or one of R^{b} and R^{c} together with one of R⁵ and R⁶ and the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
each R^{e} is independently: hydrogen; C₁₋₆alkyl; halo; C₁₋₆alkoxy; or cyano; wherein the C₁₋₆alkyl moieties may be unsubstituted or substituted one or more times with halo;
R^{f} and R^{g} each independently is: hydrogen; or C₁₋₆alkyl which may be unsubstituted or substituted one or more times with halo;
R^{h} is: C₁₋₆alkyl; C₃₋₆cycloalkyl; or C₃₋₆cycloalkyl-C₁₋₆alkyl, each of which may be unsubstituted or substituted one or more times with halo;
Rⁱ is: C₁₋₆alkyl; halo; oxo; hydroxy; acetyl; or C₁₋₆alkoxy; wherein the C₁₋₆alkyl moieties may be unsubstituted or substituted one or more times with halo; and
R^{j} and R^{k} each independent is: hydrogen; or C₁₋₆alkyl which may be unsubstituted or substituted one or more times with halo;
provided that when Ar is imidazolyl substituted with methyl, A is a bond, and each of X¹, X², X³ and X⁴ is CR^{e}, then R^{e} is not methoxy.

In certain embodiments of formula I, when Ar is 4-methyl-imidazol-1-yl, A is a bond, and each of X¹, X², X³ and X⁴ is CR^{e}, then R^{e} is not methoxy.

In certain embodiments of formula I, when Ar is imidazolyl, A is a bond, R¹⁰ is methyl; q is 0 and each of X¹, X², X³ and X⁴ is CR^{e}, then R^{e} is not methoxy.

In certain embodiments of formula I, when Ar is imidazolyl substituted with methyl, A is a bond, and each of X¹, X², X³ and X⁴ is CR^{e}, then R^{e} is not methoxy.

In certain embodiments of formula I, when Ar is 4-methyl-imidazol-1-yl, then R^{e} is not methoxy. In certain embodiments of formula I, when Ar is imidazolyl substituted with methyl, then R^{e} is not methoxy.

In certain embodiments of formula I, m is 0.

In certain embodiments of formula I, m is 1.

In certain embodiments of formula I, n is 0.

In certain embodiments of formula I, n is 1.

In certain embodiments of formula I, p is from 0 to 2.

In certain embodiments of formula I, p is 0 or 1.

In certain embodiments of formula I, p is 0.

In certain embodiments of formula I, p is 1.

In certain embodiments of formula I, p is 2.

In certain embodiments of formula I, p is 3.

In certain embodiments of formula I, q is 0.

In certain embodiments of formula I, q is 1.

In certain embodiments of formula I, q is 2.

In certain embodiments of formula I, t is from 0 to 3.

In certain embodiments of formula I, t is 0.

In certain embodiments of formula I, t is 1.

In certain embodiments of formula I, t is 2.

In certain embodiments of formula I, t is 3.

In certain embodiments of formula I, t is 4.

In certain embodiments of formula I, Ar is: phenyl; imidazolyl; pyrazolyl; isoxazolyl; oxazolyl; thiazolyl; isothizaolyl; oxadiazolyl; thiadiazolyl; triazolyl; tetrazolyl; pyridinyl; pyrimidinyl; pyridazinyl; pyrazinyl; indolyl; indazolyl; or [1,2,4]triazolo[4,3-a]pyridinyl.

In certain embodiments of formula I, Ar is: phenyl; imidazolyl; pyrazolyl; isoxazolyl; oxazolyl; thiazolyl; oxadiazolyl; triazolyl; pyridinyl; pyrimidinyl; pyridazinyl; or [1,2,4]triazolo[4,3-a]pyridinyl.

In certain embodiments of formula I, Ar is phenyl.

In certain embodiments of formula I, Ar is imidazolyl.

In certain embodiments of formula I, Ar is pyrazolyl.

In certain embodiments of formula I, Ar is isoxazolyl.

In certain embodiments of formula I, Ar is oxazolyl.

In certain embodiments of formula I, Ar is thiazolyl.

In certain embodiments of formula I, Ar is oxadiazolyl.

In certain embodiments of formula I, Ar is triazolyl.

In certain embodiments of formula I, Ar is pyridinyl.

In certain embodiments of formula I, Ar is pyrimidinyl.

In certain embodiments of formula I, Ar is pyridazinyl.

In certain embodiments of formula I, Ar is [1,2,4]triazolo[4,3-a]pyridinyl.

In certain embodiments of formula I, Ar is: phenyl; imidazol-1-yl; imidazol-2-yl; imidazol-4-yl; pyrazol-3-yl; pyrazol-4-yl; isoxazol-3-yl; isoxazol-4-yl; isoxazol-5-yl; oxazol-2-yl; thiazol-5-yl; [1,2,4]oxadiazol-3-yl; [1,2,4]triazol-3-yl; pyridin-2-yl; pyridin-3-yl; pyridin-4-yl; pyrimidin-5-yl; pyridazinyl; or [1,2,4]triazolo[4,3-a]pyridin-5-yl.

In certain embodiments of formula I, A is: a bond; -CH₂-; -C(O)-; -NR^{a}-; -O-;
-S-; or -SO₂-.

In certain embodiments of formula I, A is: a bond; -(CRⱼRₖ)ₜ-; -C(O)-(CRⱼRₖ)ₜ-; -(CRⱼRₖ)ₜ-C(O)-; -(CRⱼRₖ)ₜ-NR^{a}-; -C(O)NR^{a}-(CRⱼRₖ)ₜ-; (CRⱼRₖ)ₜ-NR^{a}C(O)-; -(CRⱼRₖ)ₜ-O-; -(CRⱼRₖ)ₜ-S-; - or -(CRⱼRₖ)ₜ-SO₂-.

In certain embodiments of formula I, A is: a bond; -C(O)-(CRⱼRₖ)ₜ-; -(CRⱼRₖ)ₜ-C(O)-; -(CRⱼRₖ)ₜ-NR^{a}-; -C(O)NR^{a}-(CRⱼRₖ)ₜ-; (CRⱼRₖ)ₜ-NR^{a}C(O)-; or -(CRⱼRₖ)ₜ-O.

In certain embodiments of formula I, A is: A is: a bond; -(CR^{j}R^{k})ₜ-NR^{a}C(O)-; -(CR^{j}R^{k})ₜ-O-; or-C(O)NR^{a}-(CR^{j}R^{k})ₜ-.

In certain embodiments of formula I, A is: a bond; -NR^{a}-; -O-; or -S-.

In certain embodiments of formula I, A is: a bond; -NR^{a}-; or -O-.

In certain embodiments of formula I, A is a bond.

In certain embodiments of formula I, A is -CH₂-.

In certain embodiments of formula I, A is -C(O)-.

In certain embodiments of formula I, A is -NR^{a}-.

In certain embodiments of formula I, A is -O-.

In certain embodiments of formula I, A is -S-.

In certain embodiments of formula I, A is -SO₂-.

In certain embodiments of formula I, A is -C(O)NR^{a}-(CH₂)ₜ-.

In certain embodiments of formula I, A is -(CH₂)ₜ-NR^{a}C(O)-.

In certain embodiments of formula I, A is -(CRⱼRₖ)ₜ.

In certain embodiments of formula I, A is -CRⱼRₖ-.

In certain embodiments of formula I, A is - C(O)-(CRⱼRₖ)ₜ-.

In certain embodiments of formula I, A is -(CRⱼRₖ)ₜ-C(O)-.

In certain embodiments of formula I, A is -NR^{a}-(CRⱼRₖ)ₜ-.

In certain embodiments of formula I, A is -(CRⱼRₖ)ₜ-NR^{a}-.

In certain embodiments of formula I, A is -C(O)-NR^{a}-(CRⱼRₖ)ₜ-.

In certain embodiments of formula I, A is (CRⱼRₖ)ₜ-NR^{a}-C(O)-.

In certain embodiments of formula I, A is -O-(CRⱼRₖ)ₜ-.

In certain embodiments of formula I, A is -(CRⱼRₖ)ₜ-O-.

In certain embodiments of formula I, A is -S-(CRⱼRₖ)ₜ.

In certain embodiments of formula I, A is -(CRⱼRₖ)ₜ-S-.

In certain embodiments of formula I, A is -SO₂-(CRⱼRₖ)ₜ-.

In certain embodiments of formula I, A is -(CRⱼRₖ)ₜ-SO₂-.

In certain embodiments of formula I, A is -(CH₂)₂-O-.

In certain embodiments of formula I, A is -(CH₂)-O-.

In certain embodiments of formula I, A is -O-(CH₂)₂-.

In certain embodiments of formula I, A is -O-(CH₂)-.

In certain embodiments of formula I, A is -(CH₂)₂-C(O)-.

In certain embodiments of formula I, A is -(CH₂)-C(O)-.

In certain embodiments of formula I, A is -C(O)-(CH₂)₂-.

In certain embodiments of formula I, A is -C(O)-(CH₂)-.

In certain embodiments of formula I, A is -C(O)-NH-.

In certain embodiments of formula I, A is -CH₂-C(O)-NH-.

In certain embodiments of formula I, A is -NH-.

In certain embodiments of formula I, A is -(CH₂)₂-NH-.

In certain embodiments of formula I, A is -CH₂-NH-.

In certain embodiments of formula I, A is -NH-(CH₂)₂-.

In certain embodiments of formula I, A is -NH-CH₂-.

In certain embodiments of formula I, A is -NH-C(O)-.

In certain embodiments of formula I, t is from 0 to 3.

In certain embodiments of formula I, t is from 1 to 3.

In certain embodiments of formula I, t is from 0 to 2.

In certain embodiments of formula I, W is -CR^{b}R^{c}- or -O-.

In certain embodiments of formula I, W is -CR^{b}R^{c}-.

In certain embodiments of formula I, W is -O-.

In certain embodiments of formula I, W is -NR^{d}-.

In certain embodiments of formula I, W is -S-.

In certain embodiments of formula I, W is -SO₂-.

In certain embodiments of formula I, W is -CH₂-.

In certain embodiments of formula I, one or two of X¹, X², X³ and X⁴ is N and the others are CR^{e}.

In certain embodiments of formula I, three of X¹, X², X³ and X⁴ are CR^{e} and the other is N.

In certain embodiments of formula I, X¹, X², X³ and X⁴ are CR^{e}.

In certain embodiments of formula I, X¹ is N and X², X³ and X⁴ are CR^{e}.

In certain embodiments of formula I, X² is N and X¹, X³ and X⁴ are CR^{e}.

In certain embodiments of formula I, X¹ and X⁴ are N, and X² and X³ are CR^{a}.

In certain embodiments of formula I, X² and X³ are N, and X¹ and X⁴ are CR^{e}.

In certain embodiments of formula I, X¹ and X² are N, and X³ and X⁴ are CR^{e}.

In certain embodiments of formula I, R¹ is hydrogen.

In certain embodiments of formula I, R¹ is C₁₋₆alkyl.

In certain embodiments of formula I, R² is hydrogen.

In certain embodiments of formula I, R² is C₁₋₆alkyl.

In certain embodiments of formula I, R³ is hydrogen.

In certain embodiments of formula I, R³ is C₁₋₆alkyl.

In certain embodiments of formula I, R⁴ is hydrogen.

In certain embodiments of formula I, R⁴ is C₁₋₆alkyl.

In certain embodiments of formula I, R⁵ is hydrogen.

In certain embodiments of formula I, R⁵ is C₁₋₆alkyl.

In certain embodiments of formula I, R⁶ is hydrogen.

In certain embodiments of formula I, R⁶ is C₁₋₆alkyl.

In certain embodiments of formula I, R⁷ is hydrogen.

In certain embodiments of formula I, R⁷ is C₁₋₆alkyl.

In certain embodiments of formula I, R⁸ is hydrogen.

In certain embodiments of formula I, R⁸ is C₁₋₆alkyl.

In certain embodiments of formula I, R³ and R⁴ together with the atoms to which they are attached form a three, four, five, six or seven membered saturated or partially saturated ring that may optionally include one or two heteroatoms selected from -O-, -NR^{a}- or -S-, and which may be optionally substituted one or more times with Rⁱ.

In certain embodiments of formula I, R³ and R⁴ together with the atoms to which they are attached form a three, four or five membered saturated ring.

In certain embodiments of formula I, R⁵ and R⁶ together with the atoms to which they are attached form a three, four, five, six or seven membered saturated or partially saturated ring that may optionally include one or two heteroatoms selected from -O-, -NR^{a}- or -S-, and which may be optionally substituted one or more times with Rⁱ.

In certain embodiments of formula I, R⁵ and R⁶ together with the atoms to which they are attached form a three, four or five membered saturated ring.

In certain embodiments of formula I, R⁷ and R⁸ together with the atoms to which they are attached form a three, four, five, six or seven membered saturated or partially saturated ring that may optionally include one or two heteroatoms selected from -O-, -NR^{a}- or -S-, and which may be optionally substituted one or more times with Rⁱ.

In certain embodiments of formula I, R⁷ and R⁸ together with the atoms to which they are attached form a three, four or five membered saturated ring.

In certain embodiments of formula I, one of R³ and R⁴ together with one of R⁵ and R⁶ and the atoms to which they are attached form a three, four, five, six or seven membered ring that may optionally include one or two heteroatoms selected from -O-, -NR^{a}- or -S-, and which may be optionally substituted one or more times with Rⁱ.

In certain embodiments of formula I, one of R⁵ and R⁶ together with one of R⁷ and R⁸ and the atoms to which they are attached form a three, four, five, six or seven membered saturated or partially saturated ring that may optionally include one or two heteroatoms selected from -O-,-NR^{a}- or -S-, and which may be optionally substituted one or more times with Rⁱ.

In certain embodiments of formula I, each R⁹ is independently: C₁₋₆alkyl; halo; or halo-C₁₋₆alkyl. In certain embodiments of formula I, R⁹ is C₁₋₆alkyl.

In certain embodiments of formula I, R⁹ is halo.

In certain embodiments of formula I, R⁹ is C₁₋₆alkoxy.

In certain embodiments of formula I, R⁹ is cyano.

In certain embodiments of formula I, R⁹ is halo-C₁₋₆alkyl.

In certain embodiments of formula I, each R⁹ is independently: fluoro; chloro; or trifluoromethyl.

In certain embodiments of formula I, R¹⁰ is hydrogen.

In certain embodiments of formula I, R¹⁰ is C₁₋₆alkyl.

In certain embodiments of formula I, R¹⁰ is cyano.

In certain embodiments of formula I, R¹⁰ is -(CH₂)ᵥ-NR^{f}R^{g}.

In certain embodiments of formula I, R¹⁰ is -(CH₂)ᵥ-S(O)_{w}-R^{h}.

In certain embodiments of formula I, R¹⁰ is -(CH₂)ᵥ-C(O)-NR^{f}R^{g}.

In certain embodiments of formula I, R¹⁰ is -(CH₂)ᵥ-S(O)_{w}-NR^{f}R^{g}.

In certain embodiments of formula I, R¹⁰ is -(CH₂)ᵥ-NR^{f}-C(O)-R^{h}.

In certain embodiments of formula I, R¹⁰ is -(CH₂)ᵥ-NR^{f}-C(O)-NR^{f}R^{g}.

In certain embodiments of formula I, R¹⁰ is -(CH₂)ᵥ-NR^{f}-S(O)_{w}-R^{h}.

In certain embodiments of formula I, R¹⁰ is: hydrogen; C₁₋₆alkyl; -SO₂-NH₂; -SO₂-CH₃; cyano;-C(O)-NH₂; -CH₂-C(O)-NH₂; -CH₂-NH-C(O)-CH₃; -C(O)-NH-CH₃; -C(O)-N(CH₃)₂; or -NH-SO₂-CH₃.

In certain embodiments of formula I, R¹⁰ is -SO₂-CH₃.

In certain embodiments of formula I, R¹¹ is C₁₋₆alkyl.

In certain embodiments of formula I, R¹⁰ is halo.

In certain embodiments of formula I, R¹⁰ is C₁₋₆alkoxy.

In certain embodiments of formula I, R¹⁰ is cyano.

In certain embodiments of formula I, R¹⁰ is halo-C₁₋₆alkyl.

In certain embodiments of formula I, R¹⁰ is hydroxy-C₁₋₆alkyl.

In certain embodiments of formula I, R¹⁰ is halo-C₁₋₆alkoxy.

In certain embodiments of formula I, R¹⁰ is C₁₋₆alkylsulfonyl.

In certain embodiments of formula I, R¹¹ is C₁₋₆alkyl; halo.

In certain embodiments of formula I, R¹¹ is C₁₋₆alkoxy.

In certain embodiments of formula I, R¹¹ is cyano.

In certain embodiments of formula I, R¹¹ is halo-C₁₋₆alkyl.

In certain embodiments of formula I, R¹¹ is hydroxy-C₁₋₆alkyl.

In certain embodiments of formula I, R¹¹ is halo-C₁₋₆alkoxy.

In certain embodiments of formula I, R¹¹ is C₁₋₆alkylsulfonyl.

In certain embodiments of formula I, R^{a} is hydrogen.

In certain embodiments of formula I, R^{a} is C₁₋₆alkyl.

In certain embodiments of formula I, R^{b} is hydrogen.

In certain embodiments of formula I, R^{b} is C₁₋₆alkyl.

In certain embodiments of formula I, R^{c} is hydrogen.

In certain embodiments of formula I, R^{c} is C₁₋₆alkyl.

In certain embodiments of formula I, R^{b} and R^{c} together with the atoms to which they are attached form a three, four, five, six or seven membered saturated or partially saturated ring that may optionally include one or two heteroatoms selected from -O-, -NR^{a}- or -S-, and which may be optionally substituted one or more times with Rⁱ.

In certain embodiments of formula I, one of R^{b} and R^{c} together with one of R⁷ and R⁸ and the atoms to which they are attached form a three, four, five, six or seven membered saturated or partially saturated ring that may optionally include one or two heteroatoms selected from -O-, -NR^{a}- or-S-, and which may be optionally substituted one or more times with Rⁱ.

In certain embodiments of formula I, one of R^{b} and R^{c} together with one of R⁵ and R⁶ and the atoms to which they are attached form a three, four, five, six or seven membered saturated or partially saturated ring that may optionally include one or two heteroatoms selected from -O-,-NR^{a}- or -S-, and which may be optionally substituted one or more times with Rⁱ.

In certain embodiments of formula I, R^{d} is hydrogen.

In certain embodiments of formula I, R^{d} is C₁₋₆alkyl.

In certain embodiments of formula I, each R^{e} is independently: hydrogen; C₁₋₆alkyl; halo; or cyano; wherein the C₁₋₆alkyl moieties may be unsubstituted or substituted one or more times with halo;

In certain embodiments of formula I, each R^{e} is independently: hydrogen; C₁₋₆alkyl; halo; or halo-C₁₋₆alkyl.

In certain embodiments of formula I, each R^{e} is independently: hydrogen; C₁₋₆alkyl; or halo.

In certain embodiments of formula I, each R^{e} is independently: hydrogen; or halo.

In certain embodiments of formula I, each R^{e} is independently: hydrogen; or fluoro.

In certain embodiments of formula I, R^{e} is hydrogen.

In certain embodiments of formula I, R^{e} is C₁₋₆alkyl.

In certain embodiments of formula I, R^{e} is halo.

In certain embodiments of formula I, R^{e} is C₁₋₆alkoxy.

In certain embodiments of formula I, R^{e} is cyano.

In certain embodiments of formula I, R^{e} is halo-C₁₋₆alkyl.

In certain embodiments of formula I, R^{f} is hydrogen.

In certain embodiments of formula I, R^{f} is C₁₋₆alkyl.

In certain embodiments of formula I, R^{g} is hydrogen.

In certain embodiments of formula I, R^{g} is C₁₋₆alkyl.

In certain embodiments of formula I, R^{h} is C₁₋₆alkyl.

In certain embodiments of formula I, R^{h} is C₃₋₆cycloalkyl.

In certain embodiments of formula I, R^{h} is C₃₋₆cycloalkyl-C₁₋₆alky.

In certain embodiments of formula I, Rⁱ is: C₁₋₆alkyl; halo; oxo; hydroxy; acetyl; or C₁₋₆alkoxy.

In certain embodiments of formula I, Rⁱ is C₁₋₆alkyl.

In certain embodiments of formula I, Rⁱ is halo.

In certain embodiments of formula I, Rⁱ is C₁₋₆alkoxy.

In certain embodiments of formula I, Rⁱ is halo-C₁₋₆alkyl.

In certain embodiments of formula I, Rⁱ is oxo.

In certain embodiments of formula I, Rⁱ is hydroxy.

In certain embodiments of formula I, Rⁱ is acetyl.

In certain embodiments of formula I, R^{j} and R^{k} each independent is: hydrogen; or methyl.

In certain embodiments of formula I, R^{j} is hydrogen.

In certain embodiments of formula I, R^{k} is hydrogen.

In certain embodiments of formula I, the subject compounds may be of formula la or Ib: 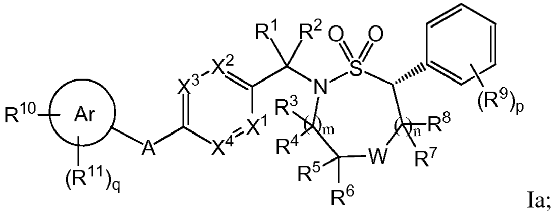 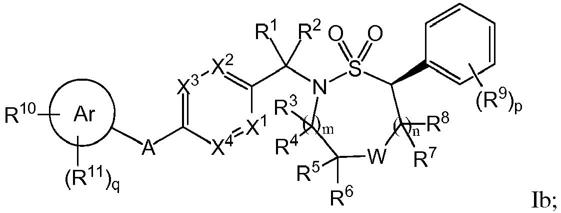 wherein m, n, p, q, Ar, A, W, X¹, X², X³, X⁴, R¹, R², R³, R⁴, R⁵, R⁶, R⁷, R⁸, R⁹, R¹⁰ and R¹¹ are as defined herein.

In certain embodiments, the subject compounds are of formula Ia.

In certain embodiments, the subject compounds are of formula lb.

In certain embodiments of formula I, the subject compounds may be of formula IIa or IIb 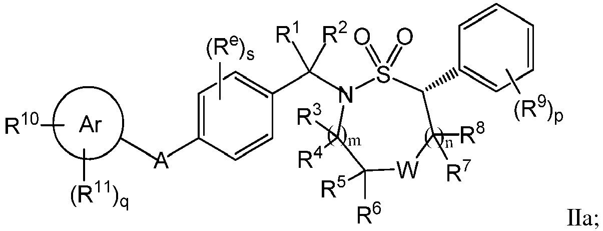 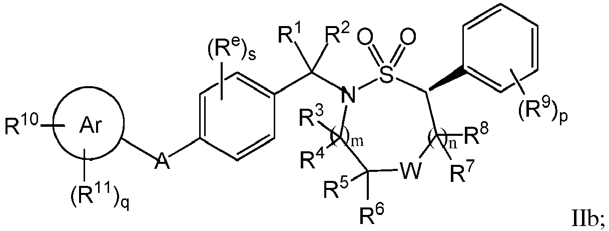
wherein s is from 0 to 3,
and m, n, p, q, Ar, A, W, R¹, R², R³, R⁴, R⁵, R⁶, R⁷, R⁸, R⁹, R¹⁰, R¹¹ and R^{e} are as defined herein. In certain embodiments, the subject compounds are of formula IIa.

In certain embodiments, the subject compounds are of formula IIb.

In certain embodiments of formula IIa or IIb, R^{e} is halo.

In certain embodiments of formula IIa or IIb, R^{e} is fluoro.

In certain embodiments of formula IIa or IIb, s is 0 or 1.

In certain embodiments of formula IIa or IIb, s is 0.

In certain embodiments of formula IIa or IIb, s is 1.

In certain embodiments of formula IIa or IIb, s is 1 or 2.

In certain embodiments of formula IIa or IIb, s is 2.

In certain embodiments of formula IIa or IIb, s is 1, 2 or 3.

In certain embodiments of formula IIa or IIb, s is 2 or 3.

In certain embodiments of formula IIa or IIb, s is 3.

In certain embodiments of formula I, the subject compounds may be of formula IIIa or IIIb: 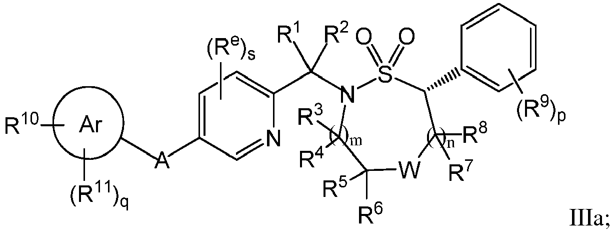 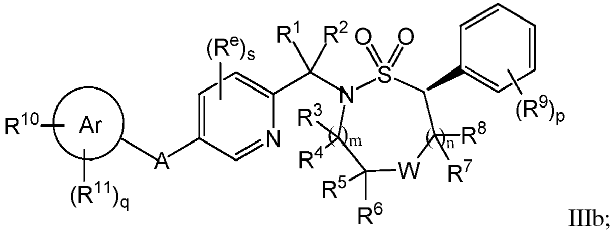 wherein m, n, p, q, s, Ar, A, W, R¹, R², R³, R⁴, R⁵, R⁶, R⁷, R⁸, R⁹, R¹⁰, R¹¹ and R^{e} are as defined herein.

In certain embodiments, the subject compounds are of formula IIIa.

In certain embodiments, the subject compounds are of formula IIIb.

In certain embodiments of formula I, the subject compounds may be of formula IVa or IVb 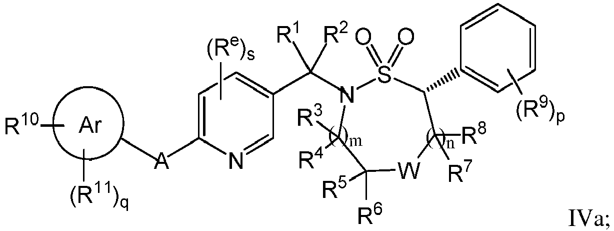 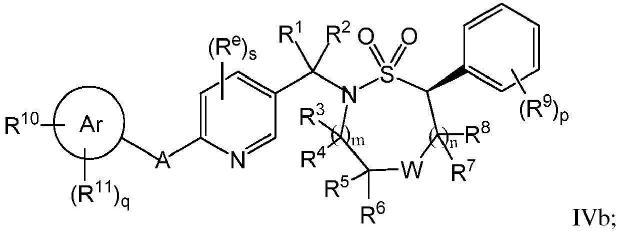 wherein m, n, p, q, s, Ar, A, W, R¹, R², R³, R⁴, R⁵, R⁶, R⁷, R⁸, R⁹, R¹⁰, R¹¹ and R^{e} are as defined herein.

In certain embodiments, the subject compounds are of formula IVa.

In certain embodiments, the subject compounds are of formula IVb.

In certain embodiments of formula I, the subject compounds may be of formula Va or Vb: 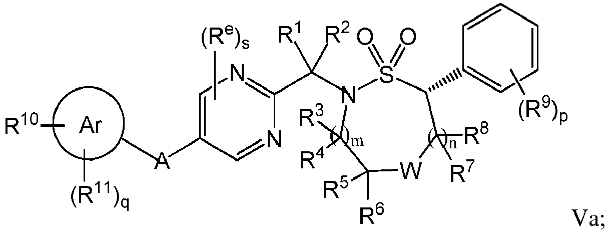 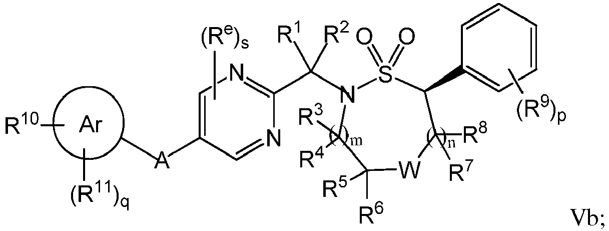 wherein m, n, p, q, s, Ar, A, W, R¹, R², R³, R⁴, R⁵, R⁶, R⁷, R⁸, R⁹, R¹⁰, R¹¹ and R^{e} are as defined herein.

In certain embodiments, the subject compounds are of formula Va.

In certain embodiments, the subject compounds are of formula Vb.

In certain embodiments of formula I, the subject compounds may be of formula VIa or VIb: 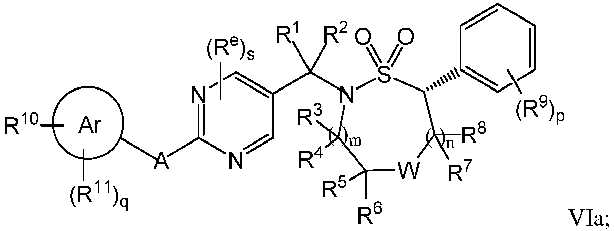 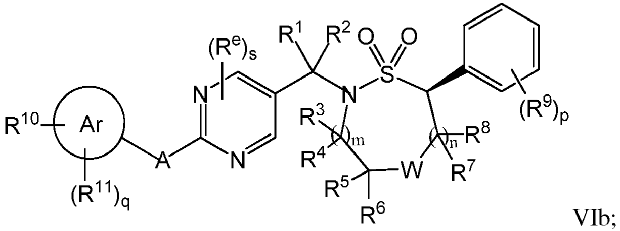 wherein m, n, p, q, s, Ar, A, W, R¹, R², R³, R⁴, R⁵, R⁶, R⁷, R⁸, R⁹, R¹⁰, R¹¹ and R^{e} are as defined herein.

In certain embodiments, the subject compounds are of formula VIa.

In certain embodiments, the subject compounds are of formula VIb.

In certain embodiments of formula I, the subject compounds may be of formula VIIa or VIIb: 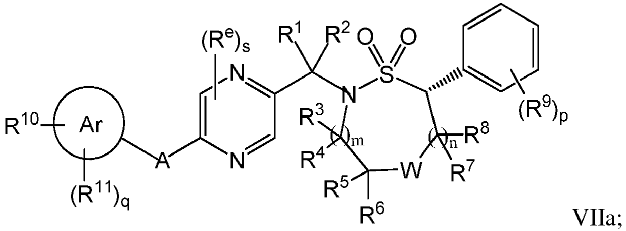 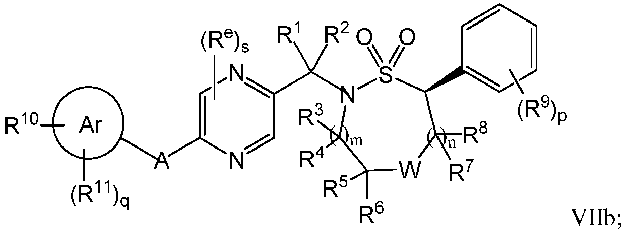 wherein m, n, p, q, s, Ar, A, W, R¹, R², R³, R⁴, R⁵, R⁶, R⁷, R⁸, R⁹, R¹⁰, R¹¹ and R^{e} are as defined herein.

In certain embodiments, the subject compounds are of formula VIIa.

In certain embodiments, the subject compounds are of formula VIIb.

In certain embodiments of formula I, the subject compounds may be of formula VIIIa or VIIIb: 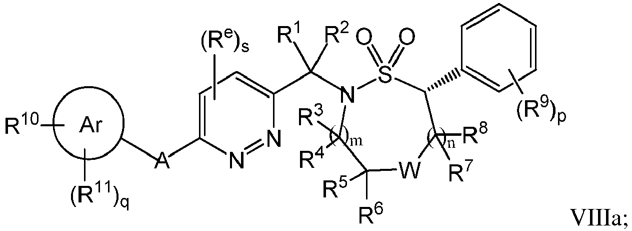 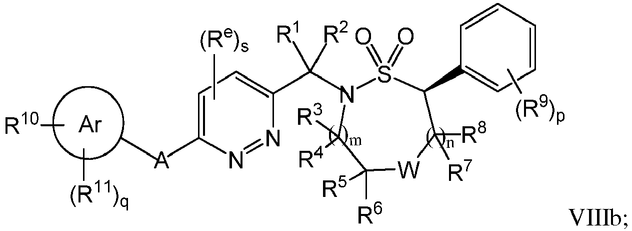 wherein m, n, p, q, s, Ar, A, W, R¹, R², R³, R⁴, R⁵, R⁶, R⁷, R⁸, R⁹, R¹⁰, R¹¹ and R^{e} are as defined herein.

In certain embodiments, the subject compounds are of formula VIIIa.

In certain embodiments, the subject compounds are of formula VIIIb.

In certain embodiments of formula I, , the subject compounds may be of formula IXa or IXb: 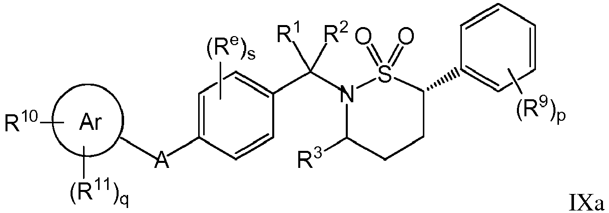 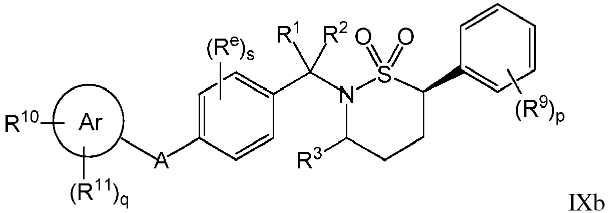 wherein p, q, s, Ar, A, Y, Z, R¹, R², R³, R⁹, R¹⁰, R¹¹ and R^{e} are as defined herein.

### Methods

The invention also provides a method for treating a disease or condition mediated by or otherwise associated with the RORc receptor, the method comprising administering to a subject in need thereof an effective amount of a compound of the invention.

The disease may be arthritis such as rheumatoid arthritis or osteoarthritis.

The disease may be asthma or COPD.

The invention also provides the use of a compound according to formula (I) for the treatment or prophylaxis of arthritis.

The invention also provides a compound according to to formula (I) for the treatment or prophylaxis of arthritis.

The invention also provides the use of a compound according to formula (I) for the preparation of a medicament for the treatment or prophylaxis of arthritis.

Representative compounds in accordance with the methods of the invention are shown in the experimental examples below.

### Synthesis

Compounds of the present invention can be made by a variety of methods depicted in the illustrative synthetic reaction schemes shown and described below.

The starting materials and reagents used in preparing these compounds generally are either available from commercial suppliers, such as Aldrich Chemical Co., or are prepared by methods known to those skilled in the art following procedures set forth in references such as Fieser and Fieser's Reagents for Organic Synthesis; Wiley & Sons: New York, 1991, Volumes 1-15; Rodd's Chemistry of Carbon Compounds, Elsevier Science Publishers, 1989, Volumes 1-5 and Supplementals; and Organic Reactions, Wiley & Sons: New York, 1991, Volumes 1-40. The following synthetic reaction schemes are merely illustrative of some methods by which the compounds of the present invention can be synthesized, and various modifications to these synthetic reaction schemes can be made and will be suggested to one skilled in the art having referred to the disclosure contained in this Application.

The starting materials and the intermediates of the synthetic reaction schemes can be isolated and purified if desired using conventional techniques, including but not limited to, filtration, distillation, crystallization, chromatography, and the like. Such materials can be characterized using conventional means, including physical constants and spectral data.

Unless specified to the contrary, the reactions described herein may be conducted under an inert atmosphere at atmospheric pressure at a reaction temperature range of from about -78 °C to about 150 °C, for example, from about 0 °C to about 125 °C, or conveniently at about room (or ambient) temperature, e.g., about 20 °C.

Scheme A below illustrates one synthetic procedure usable to prepare specific compounds of formula I, wherein LG is a leaving group such as halo, sulfonate, or the like, and m, n, p, q, Ar, A, X¹, X², X³, X⁴, Y, R¹, R², R³, R⁴, R⁵, R⁶, R⁷, R⁸, R⁹, R¹⁰, R^{b} and R^{c} are as defined herein.

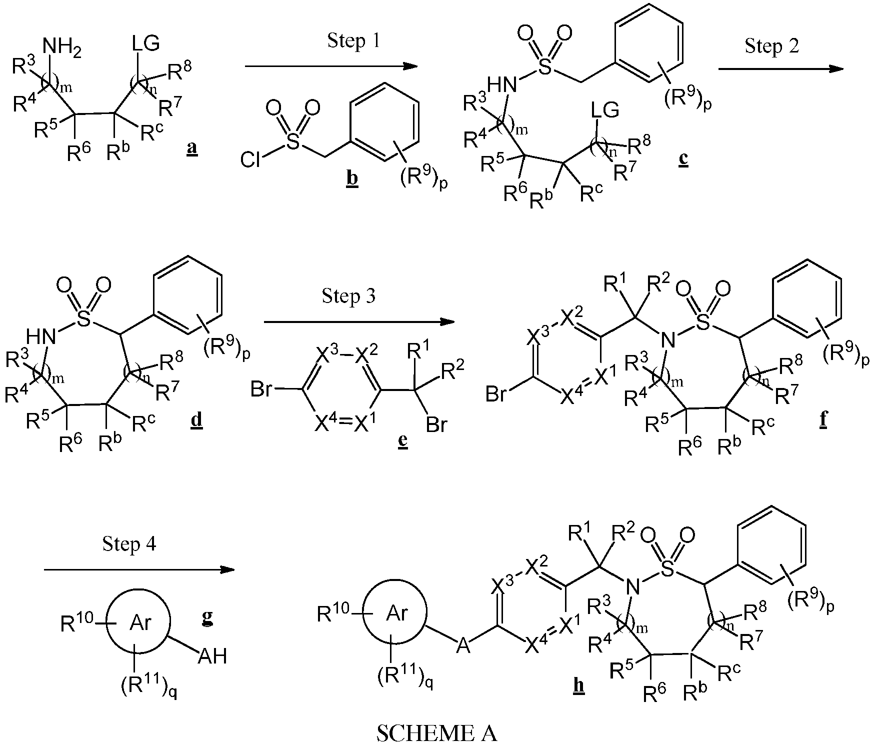

In step 1 of Scheme A, alkyl amine a is reacted with benzyl sulfonyl chloride b to form sulfonamide compound c. The reaction of step 1 may be carried out in a polar aprotic solvent such as THF or methylene chloride, and in the presence of a tertiary amine base or weak base such as potassium carbonate. The leaving group of compound a may be bromo in certain embodiments. Similarly, the chloro group of compound b may in certain embodiments be replaced by other halo or leaving group.

A cyclization reaction is carried out in step 2 to afford thiazinane compound d. The cyclization may be achieved in the presence of a strong base such as an alkyl lithium reagent, using polar aprotic solvent under anhydrous conditions.

In step 3, thiazinane compound c is reacted with aryalkyl halide compound e to yield aralkyl thiazinane f. The reaction of step 3 may be carried out in the presence of a strong base such as sodium hydride under anhydrous polar aprotic solvent conditions. The bromo groups of compound e may be replaced by other suitable leaving groups used in the art.

Thiazinane compound f may then be treated with reagent g in step 4 to provide sultam compound h, which is a compound of formula I in accordance with the invention. In embodiments wherein A is oxygen or includes oxygen such that reagent g is an alcohol, the reaction of step 4 may utilize a copper catalyst with hydrophobic solvent, in the presence of cesium carbonate or like base.

Scheme B below shows another synthetic procedure usable to prepare specific compounds of formula I, wherein TBS is tri-(tert-butyl)-silyl, and m, n, p, q, A, X¹, X², X³, X⁴, Y, R¹, R², R³, R⁴, R⁵, R⁶, R⁹ and R¹⁰ are as defined herein.

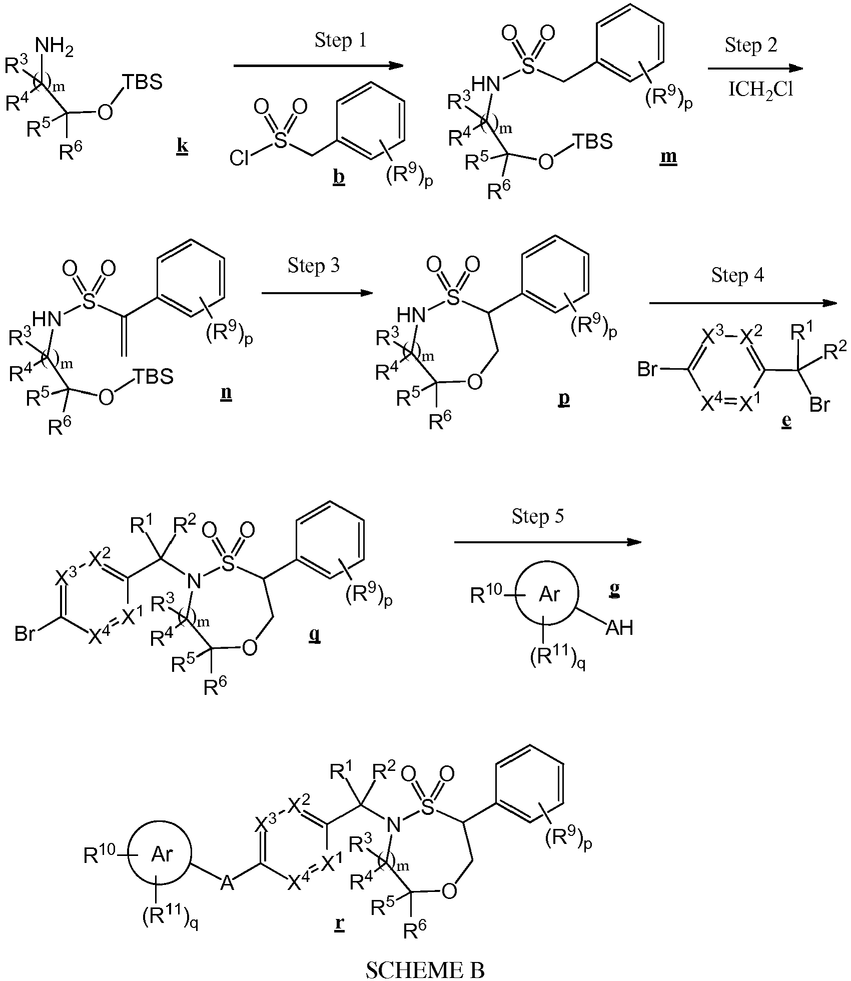

In step 1 of Scheme B, tri-(tert-butyl)-slilyloxy amine k is reacted with benzyl sulfonyl chloride b, as described above with reference to Scheme A, to form sulfonamide compound m. In certain embodiments the tri-(tert-butyl)-slilyloxy group may be replaced with other leaving groups.

In step 2, sulfonamide compound m is reacted with iodochloromethane to provide an alkenylsulfonamide compound n. This reaction may be achieved in the presence of a strong base such as an alkyl lithium reagent, using polar aprotic solvent such as THF under anhydrous conditions. In certain embodiments iodochloromethane may be replaced with other methylene reagents.

In step 3, a cyclization reaction is affected to provide oxathiazepane compound p. The cyclization may be carried out in the presence of an amine base under polar aprotic solvent conditions.

In step 4, oxathiazepane compound p is reacted with aryalkyl halide compound e to yield aralkyl oxathiazepane compound q, in the manner described above with reference to Scheme A.

Steps 5 may then be carried out by reaction of oxathiazepane compound q with reagent g in the manner described above with reference to Scheme A, to afford sultam compounds r and s respectively, which are compounds of formula I in accordance with the invention.

Many variations on the procedures of Scheme A and Scheme B are possible and will suggest themselves to those skilled in the art. Specific details for producing compounds of the invention are described in the Examples below.

### Administration and Pharmaceutical Composition

The invention includes pharmaceutical compositions comprising at least one compound of the present invention, or an individual isomer, racemic or non-racemic mixture of isomers or a pharmaceutically acceptable salt or solvate thereof, together with at least one pharmaceutically acceptable carrier, and optionally other therapeutic and/or prophylactic ingredients.

In general, the compounds of the invention will be administered in a therapeutically effective amount by any of the accepted modes of administration for agents that serve similar utilities. Suitable dosage ranges are typically 1-500 mg daily, for example 1-100 mg daily, and most preferably 1-30 mg daily, depending upon numerous factors such as the severity of the disease to be treated, the age and relative health of the subject, the potency of the compound used, the route and form of administration, the indication towards which the administration is directed, and the preferences and experience of the medical practitioner involved. One of ordinary skill in the art of treating such diseases will be able, without undue experimentation and in reliance upon personal knowledge and the disclosure of this Application, to ascertain a therapeutically effective amount of the compounds of the present invention for a given disease.

Compounds of the invention may be administered as pharmaceutical formulations including those suitable for oral (including buccal and sub-lingual), rectal, nasal, topical, pulmonary, vaginal, or parenteral (including intramuscular, intraarterial, intrathecal, subcutaneous and intravenous) administration or in a form suitable for administration by inhalation or insufflation. A particular manner of administration is generally oral using a convenient daily dosage regimen which can be adjusted according to the degree of affliction.

A compound or compounds of the invention, together with one or more conventional adjuvants, carriers, or diluents, may be placed into the form of pharmaceutical compositions and unit dosages. The pharmaceutical compositions and unit dosage forms may be comprised of conventional ingredients in conventional proportions, with or without additional active compounds or principles, and the unit dosage forms may contain any suitable effective amount of the active ingredient commensurate with the intended daily dosage range to be employed. The pharmaceutical compositions may be employed as solids, such as tablets or filled capsules, semisolids, powders, sustained release formulations, or liquids such as solutions, suspensions, emulsions, elixirs, or filled capsules for oral use; or in the form of suppositories for rectal or vaginal administration; or in the form of sterile injectable solutions for parenteral use. Formulations containing about one (1) milligram of active ingredient or, more broadly, about 0.01 to about one hundred (100) milligrams, per tablet, are accordingly suitable representative unit dosage forms.

The compounds of the invention may be formulated in a wide variety of oral administration dosage forms. The pharmaceutical compositions and dosage forms may comprise a compound or compounds of the present invention or pharmaceutically acceptable salts thereof as the active component. The pharmaceutically acceptable carriers may be either solid or liquid. Solid form preparations include powders, tablets, pills, capsules, cachets, suppositories, and dispersible granules. A solid carrier may be one or more substances which may also act as diluents, flavouring agents, solubilizers, lubricants, suspending agents, binders, preservatives, tablet disintegrating agents, or an encapsulating material. In powders, the carrier generally is a finely divided solid which is a mixture with the finely divided active component. In tablets, the active component generally is mixed with the carrier having the necessary binding capacity in suitable proportions and compacted in the shape and size desired. The powders and tablets may contain from about one (1) to about seventy (70) percent of the active compound. Suitable carriers include but are not limited to magnesium carbonate, magnesium stearate, talc, sugar, lactose, pectin, dextrin, starch, gelatine, tragacanth, methylcellulose, sodium carboxymethylcellulose, a low melting wax, cocoa butter, and the like. The term "preparation" is intended to include the formulation of the active compound with encapsulating material as carrier, providing a capsule in which the active component, with or without carriers, is surrounded by a carrier, which is in association with it. Similarly, cachets and lozenges are included. Tablets, powders, capsules, pills, cachets, and lozenges may be as solid forms suitable for oral administration.

Other forms suitable for oral administration include liquid form preparations including emulsions, syrups, elixirs, aqueous solutions, aqueous suspensions, or solid form preparations which are intended to be converted shortly before use to liquid form preparations. Emulsions may be prepared in solutions, for example, in aqueous propylene glycol solutions or may contain emulsifying agents, for example, such as lecithin, sorbitan monooleate, or acacia. Aqueous solutions can be prepared by dissolving the active component in water and adding suitable colorants, flavors, stabilizers, and thickening agents. Aqueous suspensions can be prepared by dispersing the finely divided active component in water with viscous material, such as natural or synthetic gums, resins, methylcellulose, sodium carboxymethylcellulose, and other well known suspending agents. Solid form preparations include solutions, suspensions, and emulsions, and may contain, in addition to the active component, colorants, flavors, stabilizers, buffers, artificial and natural sweeteners, dispersants, thickeners, solubilizing agents, and the like.

The compounds of the invention may be formulated for parenteral administration (e.g., by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, for example solutions in aqueous polyethylene glycol. Examples of oily or nonaqueous carriers, diluents, solvents or vehicles include propylene glycol, polyethylene glycol, vegetable oils (e.g., olive oil), and injectable organic esters (e.g., ethyl oleate), and may contain formulatory agents such as preserving, wetting, emulsifying or suspending, stabilizing and/or dispersing agents. Alternatively, the active ingredient may be in powder form, obtained by aseptic isolation of sterile solid or by lyophilization from solution for constitution before use with a suitable vehicle, e.g., sterile, pyrogen-free water.

The compounds of the invention may be formulated for topical administration to the epidermis as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also containing one or more emulsifying agents, stabilizing agents, dispersing agents, suspending agents, thickening agents, or coloring agents. Formulations suitable for topical administration in the mouth include lozenges comprising active agents in a flavored base, usually sucrose and acacia or tragacanth; pastilles comprising the active ingredient in an inert base such as gelatine and glycerine or sucrose and acacia; and mouthwashes comprising the active ingredient in a suitable liquid carrier.

The compounds of the invention may be formulated for administration as suppositories. A low melting wax, such as a mixture of fatty acid glycerides or cocoa butter is first melted and the active component is dispersed homogeneously, for example, by stirring. The molten homogeneous mixture is then poured into convenient sized molds, allowed to cool, and to solidify.

The compounds of the invention may be formulated for vaginal administration. Pessaries, tampons, creams, gels, pastes, foams or sprays containing in addition to the active ingredient such carriers as are known in the art to be appropriate.

The subject compounds may be formulated for nasal administration. The solutions or suspensions are applied directly to the nasal cavity by conventional means, for example, with a dropper, pipette or spray. The formulations may be provided in a single or multidose form. In the latter case of a dropper or pipette, this may be achieved by the patient administering an appropriate, predetermined volume of the solution or suspension. In the case of a spray, this may be achieved for example by means of a metering atomizing spray pump.

The compounds of the invention may be formulated for aerosol administration, particularly to the respiratory tract and including intranasal administration. The compound will generally have a small particle size for example of the order of five (5) microns or less. Such a particle size may be obtained by means known in the art, for example by micronization. The active ingredient is provided in a pressurized pack with a suitable propellant such as a chlorofluorocarbon (CFC), for example, dichlorodifluoromethane, trichlorofluoromethane, or dichlorotetrafluoroethane, or carbon dioxide or other suitable gas. The aerosol may conveniently also contain a surfactant such as lecithin. The dose of drug may be controlled by a metered valve. Alternatively the active ingredients may be provided in a form of a dry powder, for example a powder mix of the compound in a suitable powder base such as lactose, starch, starch derivatives such as hydroxypropylmethyl cellulose and polyvinylpyrrolidine (PVP). The powder carrier will form a gel in the nasal cavity. The powder composition may be presented in unit dose form for example in capsules or cartridges of e.g., gelatine or blister packs from which the powder may be administered by means of an inhaler.

When desired, formulations can be prepared with enteric coatings adapted for sustained or controlled release administration of the active ingredient. For example, the compounds of the present invention can be formulated in transdermal or subcutaneous drug delivery devices. These delivery systems are advantageous when sustained release of the compound is necessary and when patient compliance with a treatment regimen is crucial. Compounds in transdermal delivery systems are frequently attached to an skin-adhesive solid support. The compound of interest can also be combined with a penetration enhancer, e.g., Azone (1-dodecylazacycloheptan-2-one). Sustained release delivery systems are inserted subcutaneously into the subdermal layer by surgery or injection. The subdermal implants encapsulate the compound in a lipid soluble membrane, e.g., silicone rubber, or a biodegradable polymer, e.g., polylactic acid.

The pharmaceutical preparations may be in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

Other suitable pharmaceutical carriers and their formulations are described in Remington: The Science and Practice of Pharmacy 1995, edited by E. W. Martin, Mack Publishing Company, 19th edition, Easton, Pennsylvania. Representative pharmaceutical formulations containing a compound of the present invention are described below.

### Utility

The compounds of the invention are useful for treatment of immune disorders generally. The compounds may be used for treatment of arthritis, including rheumatoid arthritis, osteoarthritis, psoriatic arthritis, septic arthritis, spondyloarthropathies, gouty arthritis, systemic lupus erythematosus and juvenile arthritis, osteoarthritis, and other arthritic conditions.

The compounds may be used for treatment of respiratory disorders such as chronic obstructive pulmonary disease (COPD), asthma, bronchospasm, and the like.

The compounds may be used for treatment of gastrointestinal disorder ("GI disorder") such as Irritable Bowel Syndrome (IBS), Inflammatory Bowel Disease (IBD), biliary colic and other biliary disorders, renal colic, diarrhea-dominant IBS, pain associated with GI distension, and the like.

The compounds may be used for treatment of pain conditions such as inflammatory pain; arthritic pain, surgical pain; visceral pain; dental pain; premenstrual pain; central pain; pain due to burns; migraine or cluster headaches; nerve injury; neuritis; neuralgias; poisoning; ischemic injury; interstitial cystitis; cancer pain; viral, parasitic or bacterial infection; post-traumatic injury; or pain associated with irritable bowel syndrome.

### Examples

The following preparations and examples are given to enable those skilled in the art to more clearly understand and to practice the present invention. They should not be considered as limiting the scope of the invention, but merely as being illustrative and representative thereof. Unless otherwise stated, all temperatures including melting points (i.e., MP) are in degrees celsius (°C). It should be appreciated that the reaction which produces the indicated and/or the desired product may not necessarily result directly from the combination of two reagents which were initially added, i.e., there may be one or more intermediates which are produced in the mixture which ultimately leads to the formation of the indicated and/or the desired product. The following abbreviations may be used in the Preparations and Examples.

### LIST OF ABBREVIATIONS

- AcOH: Acetic acid
- AIBN: 2,2'-Azobis(2-methylpropionitrile)
- Atm.: Atmosphere
- (BOC)₂O: di-*tert*-Butyl dicarbonate
- DCM: Dichloromethane/Methylene chloride
- DIAD: Diisopropyl azodicarboxylate
- DIPEA: Diisopropylethylamine
- DMAP: 4-Dimethylaminopyridine
- DME: 1,2-Dimethoxyethane
- DMF: *N,N*-Dimethylformamide
- DMSO: Dimethyl sulfoxide
- DPPF: 1,1'-Bis(diphenylphosphino)ferrocene
- DTBA: Di-tert-butyl azodicarboxylate
- Et₂O: Diethyl ether
- EtOH: Ethanol/Ethyl alcohol
- EtOAc: Ethyl acetate
- HATU: 2-(1H-7-Azabenzotriazol-1-yl)--1,1,3,3-tetramethyl uronium hexafluorophosphate Methanaminium
- HBTU: *O*-Benzotriazol-1-yl-*N,N,N',N'*-tetramethyluronium hexafluorophosphate
- HOBT: 1-Hydroxybenzotriazole
- HPLC: High pressure liquid chromatography
- RP HPLC: Reverse phase high pressure liquid chromatography
- i-PrOH: Isopropanol/isopropyl alcohol
- LCMS: Liquid Chromatograph/Mass Spectroscopy
- MeOH: Methanol/Methyl alcohol
- MW: Microwaves
- NBS: *N*-Bromosuccinimide
- NMP: 1-Methyl-2-pyrrolidinone
- PSI: Pound per square inch
- r.t.: Room temperature
- TBDMS: *tert*-Butyldimethylsilyl
- TFA: Trifluoroacetic acid
- THF: Tetrahydrofuran
- TLC: Thin layer chromatography

### Preparations 1 and 2: (3R)-3-Aminobutan-1-ol and (3S)-3-Aminobutan-1-ol

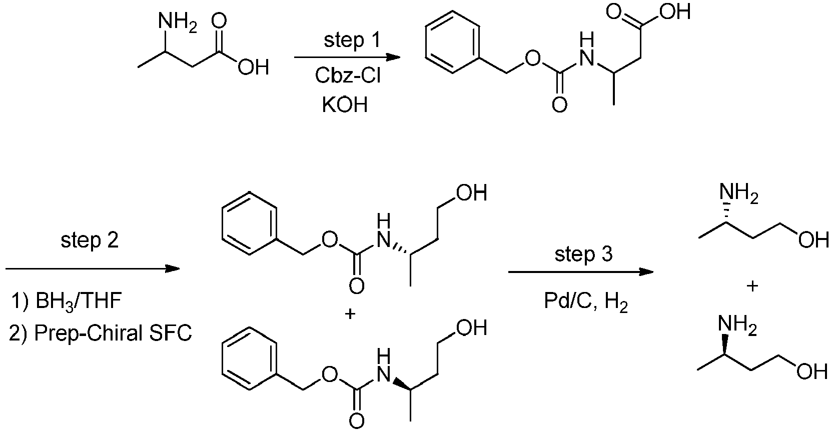

### Step 1 3-[[(Benzyloxy)carbonyl]amino]butanoic acid

Into a 2000-mL 4-necked round-bottom flask was placed a solution of 3-aminobutanoic acid (100 g, 969.75 mmol, 1.00 equiv) in water (1000 mL), followed by the addition of potassium hydroxide (136 g, 2.42 mol, 2.50 equiv) in several batches. To this was added benzyl chloroformate (247 g, 1.45 mol, 1.50 equiv) dropwise with stirring at 0-5°C. The resulting solution was stirred at 25°C for 5 h. The reaction progress was monitored by LCMS. The resulting solution was extracted with 3x250 mL of dichloromethane and the aqueous layers were combined. The pH value of the water phase was adjusted to 3 with hydrogen chloride (2 mol/L). The precipitates were collected by filtration and dried to afford 102 g (44%) of 3-[[(benzyloxy)carbonyl]amino]butanoic acid as a white solid.

### Step 2: Benzyl N-[(2S)-4-hydroxybutan-2-yl]carbamate and Benzyl N-[(2R)-4-hydroxybutan-2-yl]carbamate

Into a 2000-mL 3-necked round-bottom flask purged and maintained with an inert atmosphere of nitrogen was placed a solution of 3-[[(benzyloxy)carbonyl]amino]butanoic acid (102 g, 429.92 mmol, 1.00 equiv) in THF (300 mL), followed by the addition of BH₃/THF (1N) (645 mL, 1.50 equiv) dropwise with stirring at 0-5°C. The resulting solution was stirred at 40°C for 2 h, quenched by the addition of 200 mL of methanol and concentrated under vacuum. The residue was purified on a silica gel column eluting with ethyl acetate: petroleum ether (1:2). The crude product (70 g) was purified by Prep-SFC with the following conditions (prep SFC): Column, Phenomenex Lux 5u Cellulose-4, 2.12*25,5um; mobile phase, CO₂ (85%), ethanol (15%); Detector, UV 254nm. This resulted in 30 g (31.5%) of benzyl N-[(2R)-4-hydroxybutan-2-yl]carbamate as an off-white solid and 30 g (31.5%) of benzyl N-[(2S)-4-hydroxybutan-2-yl]carbamate as an off-white solid.

### Step 3: (3R)-3-Aminobutan-1-ol and (3S)-3-Aminobutan-1-ol

Into a 1000-mL round-bottom flask was placed a solution of benzyl *N*-[(2*S*)-4-hydroxybutan-2-yl]carbamate (30 g, 134.4 mmol, 1.00 equiv) in methanol (500 mL) and palladium carbon (3 g, 0.10 equiv). The resulting solution was stirred at 25°C for 12 h under an atmosphere of hydrogen. The solids were filtered out and the filtrate was concentrated under vacuum to afford 11.7 g (92%) of (3*S*)-3-aminobutan-1-ol as an oil. ¹H NMR (300MHz, *DMSO*, *ppm*): δ 4.48 (3H, s), 3.47 (2H, s), 2.96 (1H, s), 1.47-1.41 (2H, q), 1.02-0.99 (3H, d); LCMS (ESI), m/z, 90 [M+H] ⁺; measured [α]_{D}^{20.2} +11.65° (C=1.22g/100mL in EtOH), lit. [α]_{D}²⁰ +16.3° (c=4.5 in EtOH) (J. Org. Chem. 1996, 61, 2293-2304.).

Using the above procedure, 12.0 g 12 g (94%) of (3*R*)-3-aminobutan-1-ol was isolated as an oil. ¹H NMR (300MHz, DMSO, *ppm*): δ 4.48 (3H, s), 3.47 (2H, s), 2.96 (1H, s), 1.47-1.41 (2H, q), 1.02-0.99 (3H, d); LCMS (ESI), m/z, 90 [M+H] ⁺; measured [α]_{D}^{20.2} -11.1° (C = 0.32g/100mL in EtOH), lit. [α]_{D}²⁵ -25° (c=1.25 in EtOH) (Tetrahedron: Asymmetry 1999, 10, 2213-2224.).

### Preparation 3: (R)-N-(4-Chlorobutan-2-yl)-1-phenylmethanesulfonamide

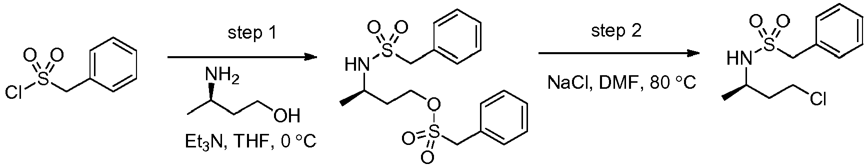

### Step 1: (R)-3-(Phenylmethylsulfonamido)butyl phenylmethanesulfonate

To a solution of (3*R*)-3-aminobutan-1-ol (1.0 g, 11.2 mmol) and triethylamine (3.3 mL, 23.6 mmol) in tetrahydrofuran (37 mL) at 0 °C was slowly added phenylmethanesulfonyl chloride (4.49 g, 23.6 mmol) and the reaction was stirred at room temperature for 16 hours. MTBE (100 mL) was then added and the Et₃N·HCl salt was removed by filtration. The filtrate was then concentrated to give crude (R)-3-(phenylmethylsulfonamido)butyl phenylmethanesulfonate which was used without purification. LCMS (ESI), m/z, 398 [M+H] +.

### Step 2: (R)-N-(4-Chlorobutan-2-yl)-1-phenylmethanesulfonamide

To the crude (*R*)-3-(phenylmethylsulfonamido)butyl phenylmethanesulfonate (23.6 mmol) was added sodium chloride (984 mg, 16.8 mmol) and dimethylformamide (37 mL) and the reaction was stirred at 80 °C for 16 hours. The reaction was then diluted with EtOAc, washed with water (x2) and brine, dried with MgSO₄, concentrated and purified by silica gel column chromatography (0-50% Acetone in Heptane, 216 nM) to give (*R*)-*N*-(4-chlorobutan-2-yl)-1-phenylmethanesulfonamide (1.71 g, 6.53 mmol, 58% yield over 2 steps). LCMS (ESI), m/z, 261 [M+H] +.

Additional compounds made using the above procedure are shown in Table 1.

**Table 1**

| | Structure | Name | LCMS (ESI), m/z, [M+H]+ |
|---|---|---|---|
| 4 | 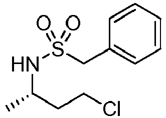 | (S)-N-(4-chlorobutan-2-yl)-1-phenylmethanesulfonamide | 261 |
| 5 | 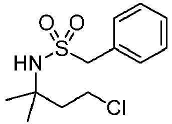 | N-(4-chloro-2-methylbutan-2-yl)-1-phenylmethanesulfonamide | 275 |
| 6 | 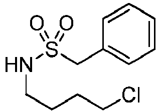 | N-(4-chlorobutyl)-1-phenylmethanesulfonamide | 261 |

### Preparation 7: N-(2-bromoethyl)(phenyl)methanesulfonamide

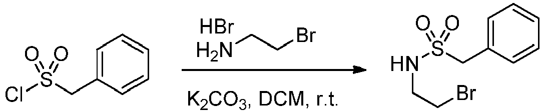

K₂CO₃ (8.7 g, 62 mmol) was added into a mixture of phenylmethanesulfonyl chloride (6 g, 31 mmol) and 2-bromoethanamine hydrobromide (6.4 g, 31 mmol) in DCM (100 mL) at 0°C. And the resulting mixture was stirred at r.t. for 4 hours and left standing overnight. Upon the completion of reaction, water (100 mL) was added in and DCM phase was separated. The aqueous phase was extracted with DCM. The combined organic phase was dried over Na₂SO₄, filetered and concentrated in vacuo to provide a crude which was separated with column chromatography (silica gel with 200 - 300 mesh, 0 to 50% of EtOAc in petroleum ether) to provide compound *N*-(2-bromoethyl)(phenyl)methanesulfonamide (7.0 g, 80%) as a pale yellow solid. ¹H NMR (300 MHz, CDCl₃) δ 7.40 (m, 5H), 4.58 (m, 1H), 4.29 (s, 2H), 3.34-3.29 (m, 4H). LCMS (ESI), 300, 302 [M+Na]⁺, Br pattern found.

### Preparation 8 N-(2-bromoethyl)(4-fluorophenyl)methanesulfonamide

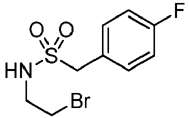

*N*-(2-bromoethyl)(4-fluorophenyl)methanesulfonamide was also made using the above procedure, replacing phenylmethanesulfonyl chloride with 4-fluoro-phenylmethanesulfonyl chloride. ¹H NMR (300 MHz, CDCl₃) δ 7.43-7.38 (m, 2H), 7.13-7.07 (m, 2H), 4.62 (br s, 1H), 4.26 (s, 2H), 3.41-3.32 (m, 4H).

### Preparation 9: N-(3-bromopropyl)(phenyl)methanesulfonamide

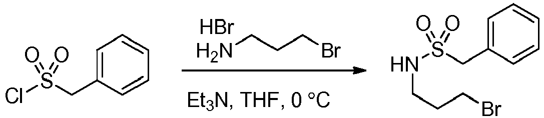

A solution of phenylmethanesulfonyl chloride (2.19 g, 10 mmol) was added into a suspension of 3-bromopropan-1-amine hydrobromide (2.19 g, 10 mmol) and Et₃N (2.02 g, 20 mmol) in THF (50 mL) at 0 °C. The mixture was stirred at 0 °C for 5 min. TLC confirmed the completion of reaction. Solid was filtered out with suction, and the filtrate was concentrated to provide compound N-(3-bromopropyl)(phenyl)methanesulfonamide (2.7 g, quant.) as a pale yellow solid which was used in the next step without further purification. ¹H NMR (300 MHz, CDCl₃) δ 7.40 (m, 5H), 4.48 (m, 1H), 4.27 (s, 2H), 3.41 (t, J = 6.6 Hz, 2H), 3.16 (q, 2H), 2.01 (m, 2H). LCMS (ESI), m/z, 314 and 316 [M+Na]⁺, Br pattern found.

### Preparation 10: N-(3-bromopropyl)(4-fluorophenyl)methanesulfonamide

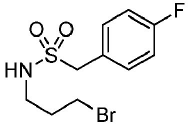

*N*-(3-bromopropyl)(4-fluorophenyl)methanesulfonamide was prepared using the above procedure. ¹H NMR (300 MHz, CDCl₃) δ 7.42-7.37 (m, 2H), 7.13-7.07 (m, 2H), 4.26 (m, 1H), 4.24 (s, 2H), 3.46-3.42 (m, 2H), 3.20-3.16 (m, 2H), 2.05-2.00 (m, 2H).

### Preparation 11: 6-Phenyl-1,2-thiazinane 1,1-dioxide

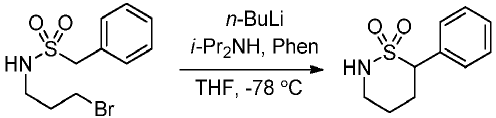

To a solution of *N*-(3-bromopropyl)-1-phenylmethanesulfonamide (2.3 g, 7.9 mmol), diisopropylamine (0.28 mL, 2.0 mmol) and 1,10-phenanthroline (3.6 mg, 0.02 mmol) in tetrahydrofuran (26 mL) at -78 °C was added *n*-BuLi (6.8 mL, 2.5 M in hexanes) dropwise and the reaction was stirred for 16 hours. Saturated NH₄Cl was then added and the reaction was diluted with EtOAc, washed with water and brine, dried with MgSO₄, concentrated and purified by silica gel column chromatography (0-50% EtOAc/heptane) to 6-Phenyl-1,2-thiazinane 1,1-dioxide (1.3 g, 80% yield). ¹H NMR (300 MHz, DMSO-*d₆*) δ 7.40-7.35 (m, 5H), 6.98 (m, 1H), 4.12 (dd, 1H), 3.26-3.20 (m, 2H), 2.40-2.30 (m, 1H), 2.16-2.12 (m, 1H), 1.77-1.65 (m, 2H). LCMS (ESI), m/z, 234 [M+Na]⁺. (Reference: D. Askin, et al. Org. Lett. 2003, 4175.) Additional compounds made using the above procedure are shown in Table 2.

**Table 2**

| | Structure | Name | LCMS (ESI), m/z, [M+H]⁺ |
|---|---|---|---|
| 12 | 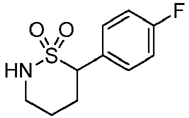 | 6-(4-fluorophenyl)-1,2-thiazinane 1,1-dioxide | 230 |
| 13 | 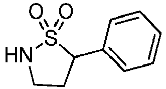 | 5-phenylisothiazolidine 1,1-dioxide | 198 |
| 14 | 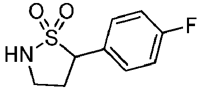 | 5-(4-fluorophenyl)isothiazolidine 1,1-dioxide | 216 |
| 15 | 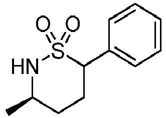 | (3*R*)-3-methyl-6-phenyl-1,2-thiazinane 1,1-dioxide | 226 |
| 16 | 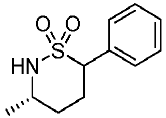 | (3*S*)-3-methyl-6-phenyl-1,2-thiazinane 1,1-dioxide | 226 |
| 17 | 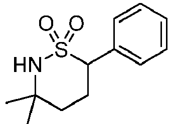 | 3,3-dimethyl-6-phenyl-1,2-thiazinane 1,1-dioxide | 240 |
| 18 | 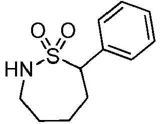 | 7-phenyl-1,2-thiazepane 1,1-dioxide | 226 |

### Preparation 19: 3-Phenyl-1,4,5-oxathiazepane 4,4-dioxide

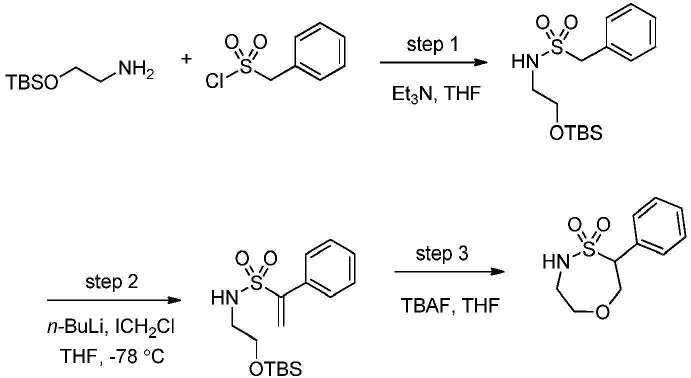

### Step 1: N-(2-((Tert-butyldimethylsilyl)oxy)ethyl)-1-phenylmethanesulfonamide

To a solution of 2-((tert-butyldimethylsilyl)oxy)ethanamine (11.7 g, 66.6 mmol) and triethylamine (11.2 mL, 79.9 mmol) in tetrahydrofuran (222 mL) at 0 °C was slowly added phenylmethanesulfonyl chloride (12.7 g, 66.6 mmol) portion wise and the reaction was stirred at room temperature for 16 hours. MTBE was then added and the Et₃N·HCl salt was removed by filtration. The filtrate was then concentrated and purified by silica gel solumn chromatography (0-30% Acetone in heptane, 216 nM) to *N*-(2-((tert-butyldimethylsilyl)oxy)ethyl)-1-phenylmethanesulfonamide (17.8 g, 81 % yield). LCMS (ESI), m/z, 330. [M+H] +.

### Step 2: N-(2-((Tert-butyldimethylsilyl)oxy)ethyl)-1-phenylethenesulfonamid

To a solution of *N*-[2-[tert-butyl(dimethyl)silyl]oxyethyl]-1-phenyl-methanesulfonamide (33 g, 100.2 mmol) in tetrahydrofuran (334 mL) at -78 °C was slowly added *n*-BuLi (2.5 M in hexanes) (100 mL, 250 mmol) via cannula and the reaction was stirred at -78 °C was 2 hours. Chloroiodomethane (8.3 mL, 110 mmol) was then slowly added and the reaction was stirred at-78 °C for one hour, then allowed to warm to room temperature and aged for 16 hours. The reaction was then quenched with saturated NH₄Cl and extracted with dichloromethane, dried with MgSO₄, concentrated and purified by silica gel column chromatography (0-60% EtOAc in heptane) to give *N*-[2-[tert-butyl(dimethyl)silyl]oxyethyl]-1-phenyl-ethenesulfonamide (24 g, 70 % yield). LCMS (ESI), m/z, 342. [M+H] +.

### Step 3: 3-Phenyl-1,4,5-oxathiazepane 4,4-dioxide

To a solution of *N*-(2-((Tert-butyldimethylsilyl)oxy)ethyl)-1-phenylethenesulfonamide (717 mg, 2.1 mmol) in tetrahydrofuran (7 mL) at 0 °C was added tetrabutylammonium fluoride (1.0 M in THF) (2.2 mL, 2.2 mmol) dropwise and the reaction was stirred at room temperature for 16 hours. Saturated NH₄Cl was then added and the product was extracted with dichloromethane (x2), dried with MgSO₄, concentrated and purified by silica gel column chromatography (0-100% EtOAc in heptane) to give 3-phenyl-1,4,5-oxathiazepane 4,4-dioxide (401 mg, 84 % yield). (24 g, 70 % yield). LCMS (ESI), m/z, 228. [M+H] +. (Reference: P. Hansen, et al. Org. Lett. 2008, 2951).

Additional compounds made using the above procedure are shown in Table 3.

**Table 3**

| | Structure | Name | LCMS (ESI), m/z, [M+H]⁺ |
|---|---|---|---|
| 20 | 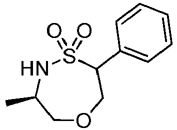 | (6*R*)-6-methyl-3-phenyl-1,4,5-oxathiazepane 4,4-dioxide | 242 |
| 21 | 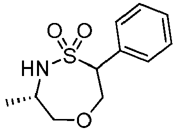 | (6*S*)-6-methyl-3-phenyl-1,4,5-oxathiazepane 4,4-dioxide | 242 |
| 22 | 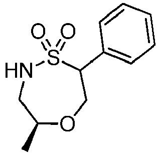 | (7*S*)-7-methyl-3-phenyl-1,4,5-oxathiazepane 4,4-dioxide | 242 |
| 23 | 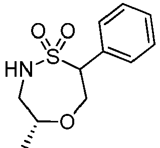 | (7*R*)-7-methyl-3-phenyl-1,4,5-oxathiazepane 4,4-dioxide | 242 |

### Preparation 20 2-(4-Bromo-2-fluorobenzyl)-6-phenyl-1,2-thiazinane 1,1-dioxide

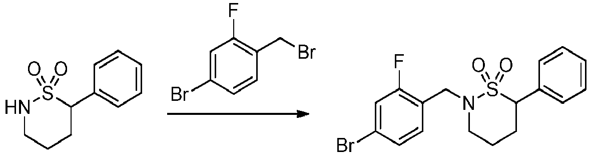

To a solution of 6-phenyl-1,2-thiazinane 1,1-dioxide (300 mg, 1.42 mmol) and 4-bromo-1-(bromomethyl)-2-fluorobenzene (456 mg, 1.7 mmol) in *N,N*-dimethylacetamide (5 mL) at 0 °C was added sodium hydride (60% in mineral oil) (68 mg, 1.85 mmol) and the reaction was stirred at room temperature for 2 hours. Water was added and the reaction was diluted with EtOAc, washed with brine, dried with MgSO₄, filtered and purified by silica gel column chromatography (0-60% EtOAc/heptane) to give 2-(4-bromo-2-fluorobenzyl)-6-phenyl-1,2-thiazinane 1,1-dioxide as a mixture of diastereomers (396 mg, 70% yield). LCMS (ESI), m/z, 398 [M+H] +.

Similarly prepared was (3S)-2-(4-bromo-2-fluorobenzyl)-3-methyl-6-phenyl-1,2-thiazinane 1,1-dioxide.

### Example 1 (3S)-2-[[4-(3,5-dimethyl-1H-pyrazol-4-yl)-2-fluoro-phenyl]methyl]-3-methyl-6-phenyl-thiazinane 1,1-dioxide

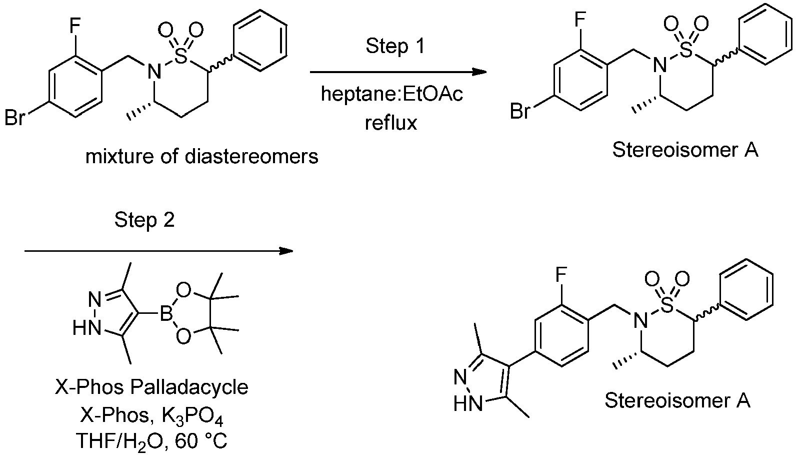

Step 1 (3S)-2-[(4-Bromo-2-fluoro-phenyl)methyl]-3-methyl-6-phenyl-thiazinane 1,1-dioxide (3S)-3-methyl-6-phenyl-thiazinane 1,1-dioxide (40 g, 97 mmol, 85:15 *trans*:*cis* mixture) was suspended in heptane (750 mL) and the suspension was heated to reflux. Ethyl acetate was slowly added until complete dissolution of the material occurred (250 mL). The solution was then subjected to a hot filtration, cooled to room temperature and store at 4 °C for 16 hours. Crystals were collected by filtration to give enantomerically pure (3S)-2-[(4-bromo-2-fluoro-phenyl)methyl]-3-methyl-6-phenyl-thiazinane 1,1-dioxide Stereoisomer A (30 g, 73 mmol, 72% yield). ¹H NMR (300 MHz, DMSO) δ 7.55 - 7.31 (m, 8H), 4.61 - 4.43 (m, 2H), 4.41 - 4.29 (m, 1H), 4.23 - 4.00 (m, 1H), 2.48 - 2.34 (m, 1H), 2.18 - 2.03 (m, 1H), 1.92 - 1.72 (m, 1H), 1.72 - 1.58 (m, 1H), 1.12 - 1.03 (d, *J* = 6.8 Hz, 3H); LCMS [M+1]+ = 412.1.
Step 2 (3*S*)-2-[[4-(3,5-dimethyl-1*H*-pyrazol-4-yl)-2-fluoro-phenyl]methyl]-3-methyl-6-phenyl-thiazinane 1,1-dioxide
   A vial was charged with (3*S*)-2-[(4-bromo-2-fluoro-phenyl)methyl]-3-methyl-6-phenyl-thiazinane 1,1-dioxide (50 mg, 0.12 mmol), 3,5-dimethyl-4-(4,4,5,5-tetramethyl-1,3,2-dioxaborolan-2-yl)-1*H*-pyrazole (40 mg, 0.18 mmol), (2-dicyclohexylphosphino-2',4',6'-triisopropyl-1,1'-biphenyl)[2-(2 aminoethyl)-phenyl]palladium(II) chloride (9.1 mg, 0.012 mmol), 2-(dicyclohexylphosphino)-2',4',6'-triisopropylbiphenyl (5.9 mg, 0.012 mmol) and potassium phosphate tribasic (101 mg, 0.42 mmol) and the vial was purged with nitrogen. Tetrahydrofuran (1 mL) and water (0.3 mL) were then added and the reaction was stirred at 60 °C for 2 hours. The reaction was then partitioned between dichloromethane and saturated sodium bicarbonate in water and the organic layer was isolated, concentrated and purified by preparative HPLC to give (3*S*)-2-[[4-(3,5-dimethyl-1*H*-pyrazol-4-yl)-2-fluoro-phenyl]methyl]-3-methyl-6-phenyl-thiazinane 1,1-dioxide Stereoisomer A (28.9 mg, 0.068 mmol, 56% yield) as a white solid. ¹H NMR (400 MHz, DMSO) δ 12.40 - 12.23 (s, 1H), 7.58 - 7.50 (m, 1H), 7.50 - 7.44 (m, 2H), 7.44 - 7.32 (m, 3H), 7.19 - 7.12 (m, 1H), 7.12 - 7.04 (m, 1H), 4.60 - 4.49 (m, 2H), 4.47 - 4.35 (m, 1H), 4.22 - 4.07 (m, 1H), 2.48 - 2.37 (m, 1H), 2.26 - 2.17 (s, 6H), 2.15 - 2.04 (m, 1H), 1.91 - 1.76 (m, 1H), 1.73 - 1.62 (m, 1H), 1.18 - 1.10 (d, J= 6.9 Hz, 3H); LCMS [M+l]⁺ = 428.2.

### Example 2 (3S)-3-methyl-2-[[4-[(1-methylpyrazol-4-yl)methoxy]phenyl]methyl]-6-phenyl-thiazinane

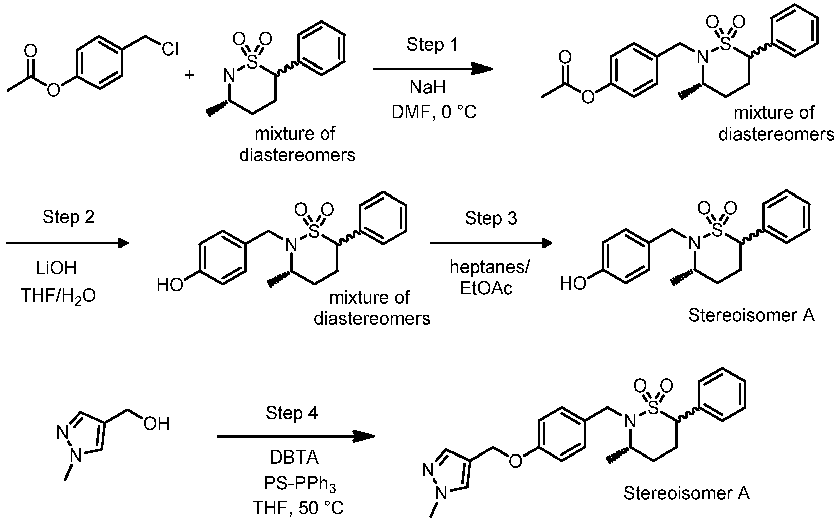

Step 1 (S)-4-((3-methyl-1,1-dioxido-6-phenyl-1,2-thiazinan-2-yl)methyl)phenyl acetate
   To a solution of (3*S*)-3-methyl-6-phenyl-thiazinane 1,1-dioxide (3.85 g, 17.1 mmol) and [4-(chloromethyl)phenyl] acetate (3.47 g, 18.8 mmol) in *N,N*-dimethylformamide (85 mL) at 0 °C was added sodium hydride (60% in mineral oil, 820 mg, 20.5 mmol) and the reaction was warmed to room temperature and stirred at that temperature for 3 hours. Water was then added and the reaction was diluted with ethyl acetate and washed with water and brine, dried with MgSO₄, concentrated to give crude (S)-4-((3-methyl-1,1-dioxido-6-phenyl-1,2-thiazinan-2-yl)methyl)phenyl acetate as a mixture of diastereomers. LCMS [M+1]⁺ = 374.
Step 2 4-[[(3*S*)-3-methyl-1,1-dioxo-6-phenyl-thiazinan-2-yl]methyl]phenol
   To the crude (S)-4-((3-methyl-1,1-dioxido-6-phenyl-1,2-thiazinan-2-yl)methyl)phenyl acetate of Step 1 was added lithium hydroxide (4.1 g, 171 mmol), tetrahydrofuran (50 mL) and water (15 mL) and the reaction was stirred at room temperature for 3 hours. The reaction was acidified to pH = 1 with 1N HCl and extracted dichloromethane and purified by silica gel column chromatography (0-100% EtOAc/Heptane) to give 4-[[(3*S*)-3-methyl-1,1-dioxo-6-phenyl-thiazinan-2-yl]methyl]phenol as a mixture of diastereomers.
Step 3 Enantomerically pure 4-[[(3*S*)-3-methyl-1,1-dioxo-6-phenyl-thiazinan-2-yl]methyl]phenol
   The 4-[[(3*S*)-3-methyl-1,1-dioxo-6-phenyl-thiazinan-2-yl]methyl]phenol of Step 2 was suspended in boiling heptane (100 mL) and ethyl acetate was added until complete dissolution occurred. The solution was cooled to room temperature and then stored at -4 °C for 16 hours. The crystals were collected by filtration to give 4-[[(3*S*)-3-methyl-1,1-dioxo-6-phenyl-thiazinan-2-yl]methyl]phenol Stereoisomer A (3.63 g, 11.0 mmol, 64% yield). ¹H NMR (300 MHz, DMSO) δ 9.28 - 9.25 (s, 1H), 7.49 - 7.33 (m, 5H), 7.22 - 7.14 (m, 2H), 6.76 - 6.68 (m, 2H), 4.48 - 4.31 (m, 2H), 4.21 - 4.12 (m, 1H), 4.12 - 3.98 (m, 1H), 2.47 - 2.33 (m, 1H), 2.15 - 2.02 (m, 1H), 1.89 - 1.71 (m, 1H), 1.70 - 1.55 (m, 1H), 1.11 - 1.03 (d, J = 7.0 Hz, 3H); LCMS [M+1]+ = 332.
Step 4 (3*S*)-3-methyl-2-[[4-[(1-methylpyrazol-4-yl)methoxy]phenyl]methyl]-6-phenyl-thiazinane
   A vial was charged with 4-[[(3*S*)-3-methyl-1,1-dioxo-6-phenyl-thiazinan-2-yl]methyl]phenol (30 mg, 0.09 mmol), (1-methyl-1*H*-pyrazol-4-yl)methanol (30 mg, 0.27 mmol), polystyrene-triphenylphosphine (2.06 mmol/g, 131 mg, 0.27 mmol), di-tert-butyl azodicarboxylate (125 mg, 0.54 mmol) and tetrahydrofuran (2 mL). The vial was then heated to 50 °C and stirred at that temperature for 16 hours. The PS-PPh₃ resin was filtered off and the reaction was concentrated and purified by preparative HPLC to give (3*S*)-3-methyl-2-[[4-[(1-methylpyrazol-4-yl)methoxy]phenyl]methyl]-6-phenyl-thiazinane 1,1-dioxide Stereoisomer A (13.7 mg, 0.032 mmol, 36% yield) as a white solid. ¹H NMR (400 MHz, DMSO-*d6*) δ 7.78 (s, 1H), 7.51 - 7.43 (m, 3H), 7.43 - 7.32 (m, 3H), 7.32 - 7.25 (m, 2H), 6.99 - 6.90 (m, 2H), 4.91 (s, 2H), 4.46 (d, *J* = 16.5 Hz, 1H), 4.38 (dd, *J =* 12.8, 3.6 Hz, 1H), 4.22 (d, *J* = 16.6 Hz, 1H), 4.11 - 4.01 (m, 1H), 3.81 (s, 3H), 2.50 - 2.35 (m, 1H), 2.14 - 2.04 (m, 1H), 1.89 - 1.73 (m, 1H), 1.68 - 1.59 (m, 1H), 1.07 (d, *J* = 6.9 Hz, 3H); LCMS [M+1]⁺ = 426.2.

### Example 3 N-((1H-pyrazol-4-yl)methyl)-4-(((3S)-3-methyl-1,1-dioxido-6-phenyl-1,2-thiazinan-2-yl)methyl)benzamide

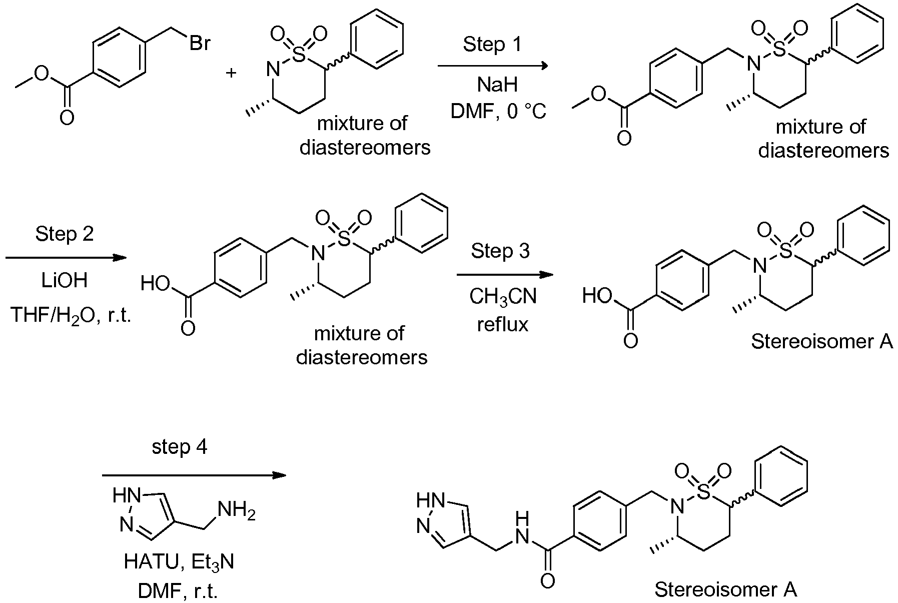

Step 1 Methyl (S)-methyl 4-((3-methyl-1,1-dioxido-6-phenyl-1,2-thiazinan-2-yl)methyl)benzoate
   To a solution of (S)-3-methyl-6-phenyl-1,2-thiazinane 1,1-dioxide (20 g, 88.9 mmol) and methyl 4-(bromomethyl)benzoate (22.4 g, 97.6 mmol) in *N,N*-dimethylformamide (295 mL) at 0 °C was added sodium hydride (60% in mineral oil, 4.6 g, 115 mmol) in small portions and the reaction was stirred at room temperature for 3 hours. Water (500 mL) was then added and the precipitate was collected by filtration to give crude methyl (S)-methyl 4-((3-methyl-1,1-dioxido-6-phenyl-1,2-thiazinan-2-yl)methyl)benzoate.
Step 2 (S)-4-((3-Methyl-1,1-dioxido-6-phenyl-1,2-thiazinan-2-yl)methyl)benzoic acid
   To a solution of crude methyl (S)-methyl 4-((3-methyl-1,1-dioxido-6-phenyl-1,2-thiazinan-2-yl)methyl)benzoate in tetrahydrofuran (300 mL) and water (100 mL) was added lithium hydroxide (21.3 g, 890 mmol) and the reaction was stirred at room temperature for 16 hours. Sodium hydroxide (1N in water, 100 mL) and water (200 mL) were then added to the reaction and the solution was washed with ethyl acetate. The aqueous layer was then acidified to pH=1 with concentrated hydrochloric acid and the precipitate was collected by filtration and dried under vacuum to give 33 grams of crude (S)-4-((3-methyl-1,1-dioxido-6-phenyl-1,2-thiazinan-2-yl)methyl)benzoic acid (85:15 *trans*:*cis*).
Step 3 Enantomerically pure 4-[[(3S)-3-methyl-1,1-dioxo-6-phenyl-thiazinan-2-yl]methyl]benzoic acid
   The crude mixture of diastereomers of (S)-4-((3-methyl-1,1-dioxido-6-phenyl-1,2-thiazinan-2-yl)methyl)benzoic acid from Step 2 was dissolved in boiling acetonitrile (500 mL), subjected to a hot filtration and then cooled to room temperature and stored at 4 °C for 16 hours. Crystals were collected by filtration to give 4-[[(3S)-3-methyl-1,1-dioxo-6-phenyl-thiazinan-2-yl]methyl]benzoic acid Stereoisomer A (6g, 16.6 mmol, 19% yield). ¹H NMR (400 MHz, DMSO) δ 12.99 - 12.69 (s, 1H), 7.98 - 7.87 (m, 2H), 7.55 - 7.43 (m, 4H), 7.43 - 7.33 (m, 3H), 4.67 - 4.55 (m, 1H), 4.50 - 4.36 (m, 2H), 4.20 - 4.05 (m, 1H), 2.47 - 2.37 (m, 2H), 2.19 - 2.06 (m, 1H), 1.91 - 1.76 (m, 1H), 1.72 - 1.61 (m, 1H), 1.11 - 1.01 (d, *J* = 6.8 Hz, 3H); LCMS [M+1]⁺ = 360.1.
Step 4 *N*-((1H-pyrazol-4-yl)methyl)-4-(((3S)-3-methyl-1,1-dioxido-6-phenyl-1,2-thiazinan-2-yl)methyl)benzamide
   To a solution of 4-[[(3*S*,6*R*)-3-methyl-1,1-dioxo-6-phenyl-thiazinan-2-yl]methyl]benzoic acid (75 mg, 0.21 mmol), (1*H*-pyrazol-4-yl)methanamine (61 mg, 0.63 mmol) and triethylamine (0.12 mL, 0.83 mmol) in *N,N*-dimethylformamide (1.5 mL) was added *O*-(7-azabenzotriazol-1-yl)-*N*,*N*,*N'*,*N'*-tetramethyluronium hexafluorophosphate (121 mg, 0.31 mmol) and the reaction was stirred at room temperature for 2 hours. The reaction was then partitioned between dichloromethane and saturated sodium bicarbonate in water. The organic layer was separated, concentrated and purified by preparative HPLC to give *N*-((1H-pyrazol-4-yl)methyl)-4-(((3*S*)-3-methyl-1,1-dioxido-6-phenyl-1,2-thiazinan-2-yl)methyl)benzamide Stereoisomer A (41.2 mg, 0.094 mmol, 45% yield). ¹H NMR (400 MHz, DMSO) δ 12.71 - 12.51 (br s, 1H), 8.78 - 8.67 (m, 1H), 7.87 - 7.79 (m, 2H), 7.68 - 7.55 (br s, 1H), 7.51 - 7.32 (m, 8H), 4.65 - 4.53 (m, 1H), 4.48 - 4.29 (m, 4H), 4.19 - 4.03 (m, 1H), 2.47 - 2.35 (m, 1H), 2.19 - 2.05 (m, 1H), 1.90 - 1.74 (m, 1H), 1.71 - 1.59 (m, 1H), 1.10 - 1.02 (d, *J* = 6.8 Hz, 3H); LCMS [M+1]⁺ = 439.2.

### Example 4 N-[3-fluoro-4-[[(3S)-3-methyl-1,1-dioxo-6-phenyl-thiazinan-2-yl]methyl]phenyl]-3,5-dimethyl-isoxazole-4-carboxamide

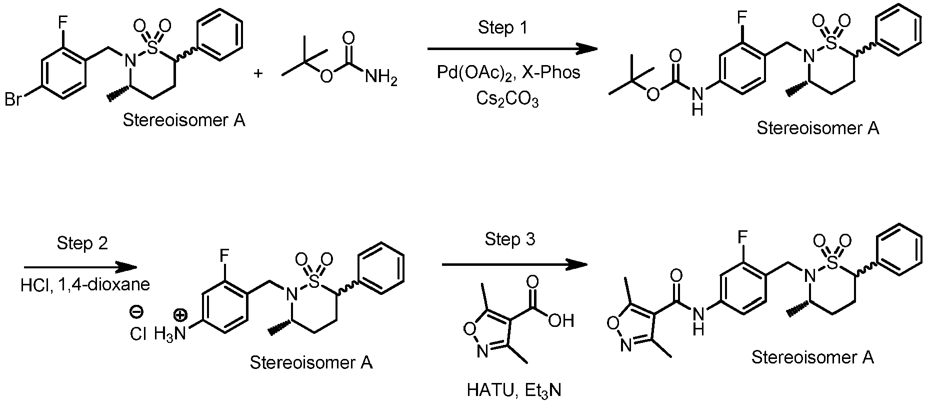

Step 1 *N*-[3-fluoro-4-[[(3*S*)-3-methyl-1,1-dioxo-6-phenyl-thiazinan-2-yl]methyl]phenyl]carbamate
A 250-mL round-bottom flask was charged with (3*S*)-2-[(4-bromo-2-fluoro-phenyl)methyl]-3-methyl-6-phenyl-thiazinane 1,1-dioxide (2.0 g, 4.8 mmol), *tert*-butyl carbamate (739 mg, 6.30 mmol), palladium(II) acetate (54 mg, 0.24 mmol) 2-(dicyclohexylphosphino)-2',4',6'-triisopropylbiphenyl (354 mg, 0.73 mmol) and cesium carbonate (2.4 g, 7.3 mmol) and the flask was purged with nitrogen. 1,4-dioxane (35 mL) was then added and the reaction was stirred at 100 °C for 4 hours. The reaction was cooled down to room temperature and filtered through celite, concentrated and purified by silica gel column chromatography (0-100% EtOAc/Heptane) to give tert-butyl *N*-[3-fluoro-4-[[(3*S*)-3-methyl-1,1-dioxo-6-phenyl-thiazinan-2-yl]methyl]phenyl]carbamate Stereoisomer A (2.26 g, 5.04 mmol, >99% yield). LCMS [M+1]+ = 449.
Step 2 3-fluoro-4-[[(3*S*)-3-methyl-1,1-dioxo-6-phenyl-thiazinan-2-yl]methyl]aniline hydrochloride
To a solution of tert-butyl N-[3-fluoro-4-[[(3*S*)-3-methyl-1,1-dioxo-6-phenyl-thiazinan-2-yl]methyl]phenyl]carbamate (2.26 g, 5.04 mmol) in 1,4-dioxane (30 mL) was added hydrochloric acid (4N in 1,4-dioxane, 30 mL) and the reaction was stirred at room temperature for 3 hours. The precipitate was collected by filtration to give 3-fluoro-4-[[(3S)-3-methyl-1,1-dioxo-6-phenyl-thiazinan-2-yl]methyl]aniline hydrochloride Stereoisomer A (1.7 g, 4.4 mmol, 88% yield) as a white solid. ¹H NMR (400 MHz, DMSO) δ 7.48 - 7.43 (m, 2H), 7.42 - 7.33 (m, 4H), 6.90 - 6.79 (m, 2H), 4.53 - 4.47 (m, 1H), 4.47 - 4.42 (m, 1H), 4.35 - 4.28 (m, 1H), 4.13 - 4.07 (m, 1H), 3.94 - 3.77 (br s, 3H), 2.47 - 2.36 (m, 1H), 2.15 - 2.05 (m, 1H), 1.88 - 1.73 (m, 1H), 1.70 - 1.61 (m, 1H), 1.13 - 1.04 (d, *J* = 6.8 Hz, 3H); LCMS [M+1]⁺ = 349.
Step 3 *N*-[3-fluoro-4-[[(3*S*)-3-methyl-1,1-dioxo-6-phenyl-thiazinan-2-yl]methyl]phenyl]-3,5-dimethyl-isoxazole-4-carboxamide
A vial was charged with 3-fluoro-4-[[(3*S*)-3-methyl-1,1-dioxo-6-phenyl-thiazinan-2-yl]methyl]aniline hydrochloride (75 mg, 0.19 mmol), 3,5-dimethylisoxazole-4-carboxylic acid (41 mg, 0.29 mmol), triethylamine (0.14 mL, 0.97 mmol) and *N,N*-dimethylformamide (1 mL), followed by *O*-(7-azabenzotriazol-1-yl)-*N,N,N',N'*-tetramethyluronium hexafluorophosphate (92 mg, 0.23 mmol) and the reaction was stirred at room temperature for 2 hours. The reaction was then partitioned between dichloromethane and saturated sodium bicarbonate in water. The organic layer was separated, concentrated and purified by preparative HPLC to give *N*-[3-fluoro-4-[[(3*S*)-3-methyl-1,1-dioxo-6-phenyl-thiazinan-2-yl]methyl]phenyl]-3,5-dimethyl-isoxazole-4-carboxamide Stereoisomer A (23.6 mg, 0.050 mmol, 26% yield). ¹H NMR (400 MHz, DMSO) δ 10.22 - 10.18 (s, 1H), 7.67 - 7.60 (m, 1H), 7.51 - 7.44 (m, 3H), 7.44 - 7.34 (m, 4H), 4.54 - 4.46 (m, 2H), 4.39 - 4.33 (m, 1H), 4.17 - 4.07 (m, 1H), 2.57 - 2.52 (s, 3H), 2.47 - 2.39 (m, 1H), 2.34 - 2.29 (s, 3H), 2.16 - 2.06 (m, 1H), 1.90 - 1.75 (m, 1H), 1.71 - 1.61 (m, 1H), 1.13 - 1.07 (d, J = 6.8 Hz, 3H); LCMS [M+1]⁺ = 472.2.
The above compounds of Examples 1-4, together with additional compounds made using the above procedures, are shown inTable 4 below, together with RORc IC₅₀ (micromolar), together with proton NMR data for selected compounds. Structures shown in table 4 with a 'waved line" bond ( 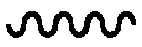 ) associated with a chiral center represent compounds for which stereoisomers of the compound have been isolated, but for which specific stereochemistry of the chiral center has not been identified.

**Table 4**

| | **Structure** | Name | **¹HNMR 400 MHz** | IC₅₀ |
|---|---|---|---|---|
| 1 | 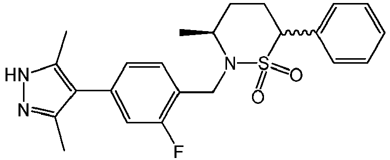 | (3S)-2-[4-(3,5-Dimethyl-1H-pyrazol-4-yl)-2-fluoro-benzyl]-3-methyl-6-phenyl-[1,2]thiazinane 1,1-dioxide | (DMSO) δ 12.40 - 12.23 (s, 1H), 7.58 - 7.50 (m, 1H), 7.50 - 7.44 (m, 2H), 7.44 - 7.32 (m, 3H), 7.19 - 7.12 (m, 1H), 7.12 - 7.04 (m, 1H), 4.60 - 4.49 (m, 2H), 4.47 - 4.35 (m, 1H), 4.22 - 4.07 (m, 1H), 2.48 - 2.37 (m, 1H), 2.26 - 2.17 (s, 6H), 2.15 - 2.04 (m, 1H), 1.91 - 1.76 (m, 1H), 1.73 - 1.62 (m, 1H), 1.18 - 1.10 (d, *J* = 6.9 Hz, 3H) | 0.527 |
| | Stereoisomer A | | | |
| 2 | 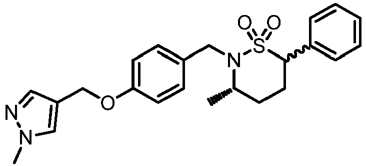 | (3*S*)-3-methyl-2-[[4-[(1-methylpyrazol-4-yl)methoxy]phenyl] methyl]-6-phenyl-thiazinane | (DMSO-*d*6) δ 7.78 (s, 1H), 7.51 - 7.43 (m, 3H), 7.43 - 7.32 (m, 3H), 7.32 - 7.25 (m, 2H), 6.99 - 6.90 (m, 2H), 4.91 (s, 2H), 4.46 (d, *J* = 16.5 Hz, 1H), 4.38 (dd, *J* = 12.8, 3.6 Hz, 1H), 4.22 (d, *J* = 16.6 Hz, 1H), 4.11 - 4.01 (m, 1H), 3.81 (s, 3H), 2.50 - 2.35 (m, 1H), 2.14 - 2.04 (m, 1H), 1.89 - 1.73 (m, 1H), 1.68 - 1.59 (m, 1H), 1.07 (d, *J* = 6.9 Hz, 3H) | |
| | Stereoisomer A | | | |
| 3 | 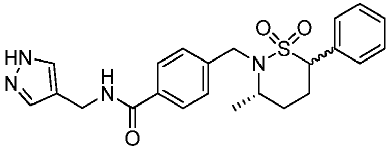 | N-((1H-pyrazol-4-yl)methyl)-4-(((3*S*)-3-methyl-1,1-dioxido-6-phenyl-[1,2]thiazinan-2-yl)methyl)benzami de | (DMSO) δ 12.71 - 12.51 (br s, 1H), 8.78 - 8.67 (m, 1H), 7.87 - 7.79 (m, 2H), 7.68 - 7.55 (br s, 1H), 7.51-7.32 (m, 8H), 4.65 - 4.53 (m, 1H), 4.48 - 4.29 (m, 4H), 4.19 - 4.03 (m, 1H), 2.47 - 2.35 (m, 1H), 2.19 - 2.05 (m, 1H), 1.90 - 1.74 (m, 1H), 1.71 - 1.59 (m, 1H), 1.10 - 1.02 (d, *J* = 6.8 Hz, 3H) | |
| | Stereoisomer A | | | |
| 4 | 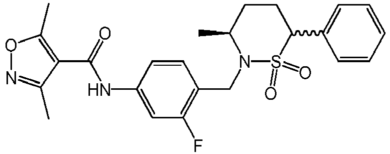 | 3,5-Dimethylisoxazole-4-carboxylic acid [3-fluoro-4-((3S)-3-methyl-1,1-dioxo-6-phenyl-[1,2]thiazinan-2-ylmethyl)-phenyl]-amide | (DMSO) δ 10.22 - 10.18 (s, 1H), 7.67 - 7.60 (m, 1H), 7.51 - 7.44 (m, 3H), 7.44 - 7.34 (m, 4H), 4.54 - 4.46 (m, 2H), 4.39 - 4.33 (m, 1H), 4.17 - 4.07 (m, 1H), 2.57 - 2.52 (s, 3H), 2.47 - 2.39 (m, 1H), 2.34 - 2.29 (s, 3H), 2.16 - 2.06 (m, 1H), 1.90 - 1.75 (m, 1H), 1.71- 1.61 (m, 1H), 1.13 - 1.07 (d, *J* = 6.8 Hz, 3H). | 0.027 |
| | Stereoisomer A | | | |
| 5 | 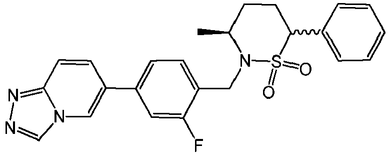 | 6-[3-Fluoro-4-((3S)-3-methyl-1,1-dioxo-6-phenyl-[1,2]thiazinan-2-ylmethyl)-phenyl]-[1,2,4]triazolo[4,3-a]pyridine | | 0.454 |
| 6 | 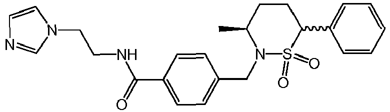 | N-(2-Imidazol-1-yl-ethyl)-4-((3S)-3-methyl-1,1-dioxo-6-phenyl-[1,2]thiazinan-2-ylmethyl)-benzamide | | 3.92 |
| 7 | 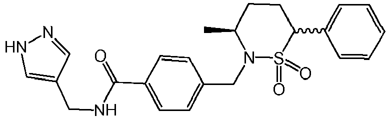 | 4-((3S)-3-Methyl-1,1-dioxo-6-phenyl-[1,2]thiazinan-2-ylmethyl)-N-(1H-pyrazol-4-ylmethyl)-benzamide | | 0.502 |
| 8 | 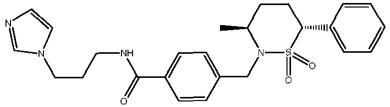 | N-(3-Imidazol-1-yl-propyl)-4-((3S)-3-methyl-1,1-dioxo-6-phenyl-[1,2]thiazinan-2-ylmethyl)-benzamide | | 4.98 |
| 9 | 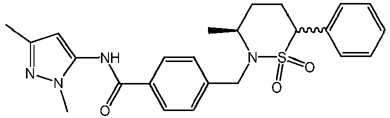 | N-(2,5-Dimethyl-2H-pyrazol-3-yl)-4-((3S)-3-methyl-1,1-dioxo-6-phenyl-[1,2]thiazinan-2-ylmethyl)-benzamide | | 0.969 |
| 10 | 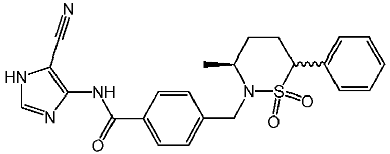 | N-(5-Cyano-1H-imidazol-4-yl)-4-((3S)-3-methyl-1,1-dioxo-6-phenyl-[1,2]thiazinan-2-ylmethyl)-benzamide | | 2.9 |
| 11 | 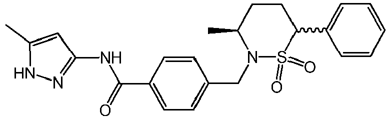 | 4-((3S)-3-Methyl-1,1-dioxo-6-phenyl-[1,2]thiazinan-2-ylmethyl)-N-(5-methyl-1H-pyrazol-3-yl)-benzamide | | 1.22 |
| 12 | 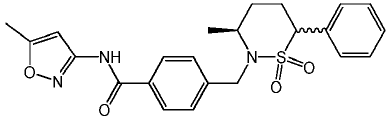 | 4-((3S)-3-Methyl-1,1-dioxo-6-phenyl-[1,2]thiazinan-2-ylmethyl)-N-(5-methyl-isoxazol-3-yl)-benzamide | | 0.404 |
| 13 | 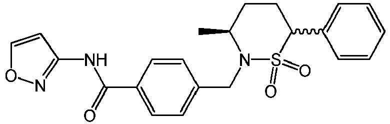 | N-Isoxazol-3-yl-4-((3S)-3-methyl-1,1-dioxo-6-phenyl-[1,2]thiazinan-2-ylmethyl)-benzamide | | 0.166 |
| 14 | 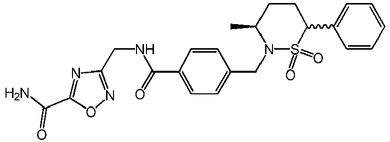 | 3-{[4-((3S)-3-Methyl-1,1-dioxo-6-phenyl-[1,2]thiazinan-2-ylmethyl)-benzoylamino]-methyl}-[1,2,4]oxadiazole-5-carboxylic acid amide | | 1.39 |
| 15 | 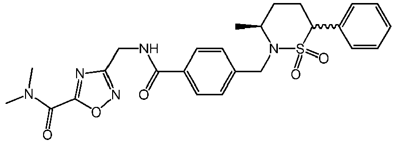 | 3-{[4-((3S)-3-Methyl-1,1-dioxo-6-phenyl-[1,2]thiazinan-2-ylmethyl)-benzoylamino]-methyl}-[1,2,4]oxadiazole-5-carboxylic acid dimethylamide | | 5.19 |
| 16 | 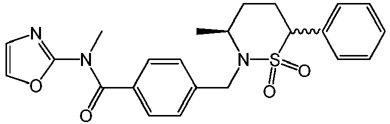 | N-Methyl-4-((3S)-3-methyl-1,1-dioxo-6-phenyl-[1,2]thiazinan-2-ylmethyl)-N-oxazol-2-yl-benzamide | | 4.84 |
| 17 | 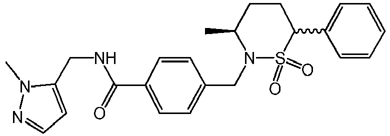 | 4-((3S)-3-Methyl-1,1-dioxo-6-phenyl-[1,2]thiazinan-2-ylmethyl)-N-(2-methyl-2H-pyrazol-3-ylmethyl)-benzamide | | 1.98 |
| 18 | 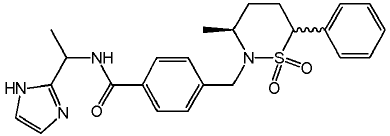 | N-[1-(1H-Imidazol-2-yl)-ethyl]-4-((3S)-3-methyl-1,1-dioxo-6-phenyl-[1,2]thiazinan-2-ylmethyl)-benzamide | | 1.92 |
| 19 | 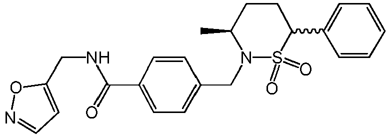 | N-Isoxazol-5-ylmethyl-4-((3S)-3-methyl-1,1-dioxo-6-phenyl-[1,2]thiazinan-2-ylmethyl)-benzamide | | 0.985 |
| 20 | 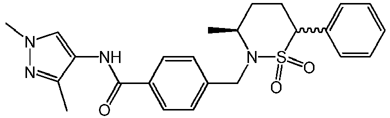 | N-(1,3-Dimethyl-1H-pyrazol-4-yl)-4-((3S)-3-methyl-1,1-dioxo-6-phenyl-[1,2]thiazinan-2-ylmethyl)-benzamide | | 2.11 |
| 21 | 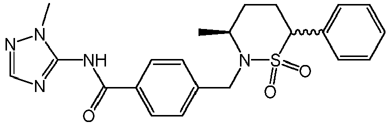 | 4-((3S)-3-Methyl-1,1-dioxo-6-phenyl-[1,2]thiazinan-2-ylmethyl)-N-(2-methyl-2H-[1,2,4]triazol-3-yl)-benzamide | | 3.28 |
| 22 | 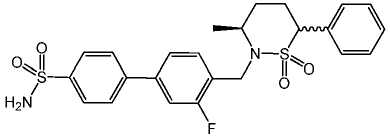 | 3'-Fluoro-4'-((3S)-3-methyl-1,1-dioxo-6-phenyl-[1,2]thiazinan-2-ylmethyl)-biphenyl-4-sulfonic acid amide | (DMSO) δ 7.93 - 7.86 (m, 4H), 7.69 - 7.57 (m, 3H), 7.50 - 7.45 (m, 2H), 7.44 - 7.33 (m, 5H), 4.65 - 4.51 (m, 2H), 4.49 - 4.40 (m, 1H), 4.23-4.09 (m, 1H), 2.47 - 2.38 (m, 1H), 2.19 - 2.08 (m, 1H), 1.93 - 1.78 (m, 1H), 1.75 - 1.62 (m, 1H), 1.23 - 1.04 (d, *J* = 6.8 Hz, 3H). | 0.087 |
| 23 | 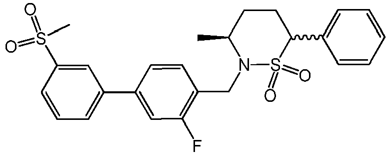 | (3S)-2-(3-Fluoro-3'-methanesulfonyl-biphenyl-4-ylmethyl)-3-methyl-6-phenyl-[1,2]thiazinane 1,1-dioxide | | 0.212 |
| 24 | 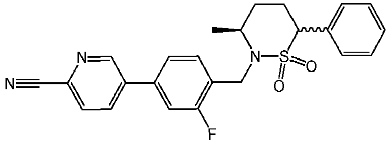 | 5-[3-Fluoro-4-((3S)-3-methyl-1,1-dioxo-6-phenyl-[1,2]thiazinan-2-ylmethyl)-phenyl]-pyridine-2-carbonitrile | | 0.11 |
| 25 | 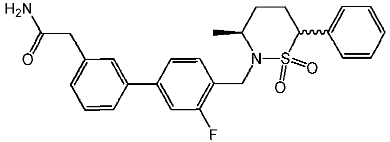 | 2-[3'-Fluoro-4'-((3S)-3-methyl-1,1-dioxo-6-phenyl-[1,2]thiazinan-2-ylmethyl)-biphenyl-3-yl]-acetamide | | 0.208 |
| 26 | 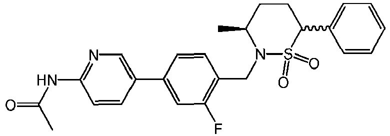 | N-{5-[3-Fluoro-4-((3S)-3-methyl-1,1-dioxo-6-phenyl-[1,2]thiazinan-2-ylmethyl)-phenyl]-pyridin-2-yl}-acetamide | | 1.91 |
| 27 | 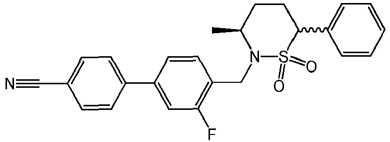 | 3'-Fluoro-4'-((3S)-3-methyl-1,1-dioxo-6-phenyl-[1,2]thiazinan-2-ylmethyl)-biphenyl-4-carbonitrile | | 0.102 |
| 28 | 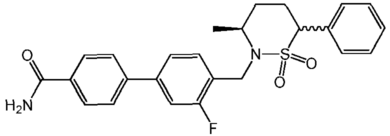 | 3'-Fluoro-4'-((3S)-3-methyl-1,1-dioxo-6-phenyl-[1,2]thiazinan-2-ylmethyl)-biphenyl-4-carboxylic acid amide | (DMSO) δ 8.06 - 7.99 (br s, 1H), 7.99-7.93 (m, 2H), 7.83 - 7.77 (m, 2H), 7.67 - 7.55 (m, 3H), 7.52 - 7.45 (m, 2H), 7.44 - 7.34 (m, 4H), 4.65 - 4.51 (m, 2H), 4.50 - 4.40 (m, 1H), 4.23 - 4.07 (m, 1H), 2.46 - 2.38 (m, 1H), 2.19 - 2.07 (m, 1H), 1.94-1.78 (m, 1H), 1.75 - 1.63 (m, 1H), 1.19 - 1.07 (d, *J* = 6.8 Hz, 3H). | 0.037 |
| 29 | 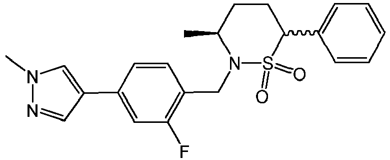 | (3S)-2-[2-Fluoro-4-(1-methyl-1H-pyrazol-4-yl)-benzyl]-3-methyl-6-phenyl-[1,2]thiazinane 1,1-dioxide | | 0.727 |
| 30 | 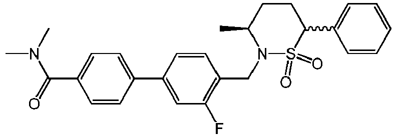 | 3'-Fluoro-4'-((3S)-3-methyl-1,1-dioxo-6-phenyl-[1,2]thiazinan-2-ylmethyl)-biphenyl-4-carboxylic acid dimethylamide | | 0.16 |
| 31 | 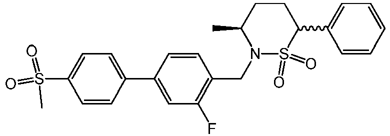 | (3S)-2-(3-Fluoro-4'-methanesulfonyl-biphenyl-4-ylmethyl)-3-methyl-6-phenyl-[1,2]thiazinane 1,1-dioxide | (DMSO) δ 8.06 - 7.94 (m, 3H), 7.71 - 7.59 (m, 2H), 7.52 - 7.44 (m, 2H), 7.44 - 7.32 (m, 2H), 4.67 - 4.52 (m, 2H), 4.51 - 4.41 (m, 1H), 4.24 - 4.08 (m, 1H), 3.27 - 3.24 (s, 3H), 2.46 - 2.40 (m, 1H), 2.19 - 2.05 (m, 1H), 1.94 - 1.78 (m, 1H), 1.76-1.63 (m, 1H), 1.18 - 1.09 (d, *J* = 6.8 Hz, 3H). | 0.028 |
| 32 | 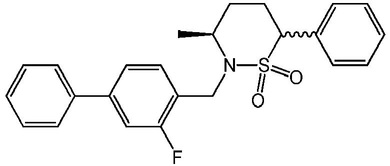 | (3S)-2-(3-Fluoro-biphenyl-4-ylmethyl)-3-methyl-6-phenyl-[1,2]thiazinane 1,1-dioxide | | 0.161 |
| 33 | 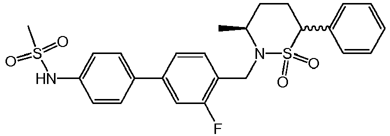 | N-[3'-Fluoro-4'-((3S)-3-methyl-1,1-dioxo-6-phenyl-[1,2]thiazinan-2-ylmethyl)-biphenyl-4-yl]-methanesulfonamid e | | 0.044 |
| 34 | 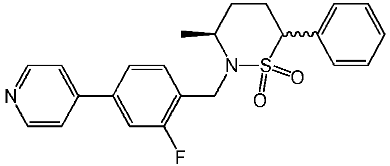 | (3S)-2-(2-Fluoro-4-pyridin-4-yl-benzyl)-3-methyl-6-phenyl-[1,2]thiazinane 1,1-dioxide | | 0.463 |
| 35 | 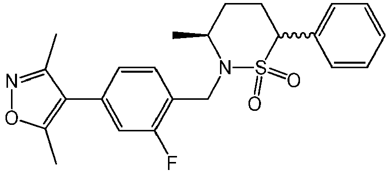 | (3S)-2-[4-(3,5-Dimethyl-isoxazol-4-yl)-2-fluoro-benzyl]-3-methyl-6-phenyl-[1,2]thiazinane 1,1-dioxide | | 0.534 |
| 36 | 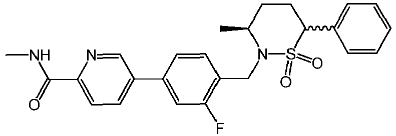 | 5-[3-Fluoro-4-((3S)-3-methyl-1,1-dioxo-6-phenyl-[1,2]thiazinan-2-ylmethyl)-phenyl]-pyridine-2-carboxylic acid methylamide | | 1.6 |
| 37 | 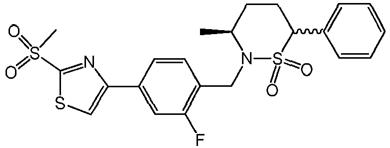 | (3S)-2-[2-Fluoro-4-(2-methanesulfonyl-thiazol-4-yl)-benzyl]-3-methyl-6-phenyl-[1,2]thiazinane 1,1 - dioxide | | 0.273 |
| 38 | 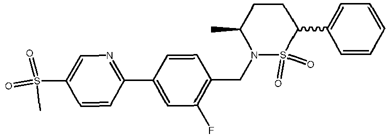 | (3S)-2-[2-Fluoro-4-(5-methanesulfonyl-pyridin-2-yl)-benzyl]-3-methyl-6-phenyl-[1,2]thiazinane 1,1-dioxide | (DMSO) δ 9.15 - 9.11 (m, 1H), 8.31- 8.27 (m, 1H), 2.47 - 2.38 (m, 1H), 8.09 - 8.04 (m, 1H), 8.02 - 7.96 (m, 1H), 7.74 - 7.66 (m, 1H), 3.40 - 3.34 (s, 3H), 2.18 - 2.07 (m, 1H), 7.50 - 7.45 (m, 2H), 7.44 - 7.36 (m, 3H), 4.68 - 4.53 (m, 2H), 4.52 - 4.44 (m, 1H), 8.43 - 8.37 (m, 1H), 4.23 - 4.11 (m, 1H), 1.94-1.79 (m, 1H), 1.74 - 1.63 (m, 1H), 1.18 - 1.08 (d, *J* = 6.9 Hz, 3H). | 0.087 |
| 39 | 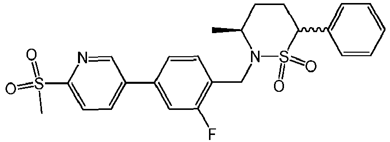 | (3S)-2-[2-Fluoro-4-(6-methanesulfonyl-pyridin-3-yl)-benzyl]-3-methyl-6-phenyl-[1,2]thiazinane 1,1-dioxide | | 0.151 |
| 40 | 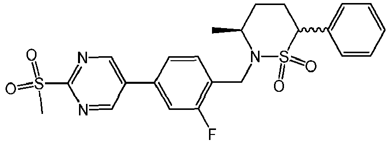 | (3S)-2-[2-Fluoro-4-(2-methanesulfonyl-pyrimidin-5-yl)-benzyl]-3-methyl-6-phenyl-[1,2]thiazinane 1,1-dioxide | | 0.989 |
| 41 | 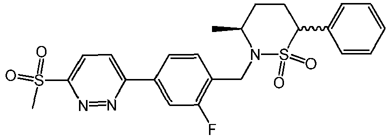 | (3S)-2-[2-Fluoro-4-(6-methanesulfonyl-pyridazin-3-yl)-benzyl]-3-methyl-6-phenyl-[1,2]thiazinane 1,1-dioxide | | 0.289 |
| 42 | 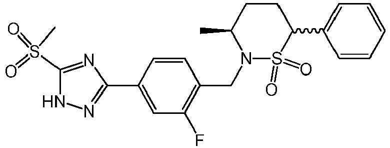 | (3S)-2-[2-Fluoro-4-(5-methanesulfonyl-1H-[1,2,4]triazol-3-yl)-benzyl]-3-methyl-6-phenyl-[1,2]thiazinane 1,1-dioxide | | 2.17 |
| 43 | 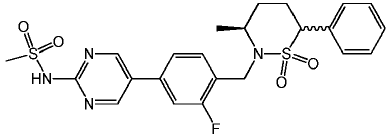 | N-{5-[3-Fluoro-4-((3S)-3-methyl-1,1-dioxo-6-phenyl-[1,2]thiazinan-2-ylmethyl)-phenyl]-pyrimidin-2-yl}-methanesulfonamid e | | 0.275 |
| 44 | 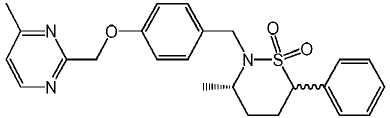 | (3S)-3-Methyl-2-[4-(4-methyl-pyrimidin-2-ylmethoxy)-benzyl]-6-phenyl-[1,2]thiazinane 1,1-dioxide | | 0.963 |
| 45 | 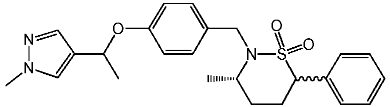 | (3S)-3-Methyl-2-{4-[1-(1-methyl-1H-pyrazol-4-yl)-ethoxy]-benzyl}-6-phenyl-[1,2]thiazinane 1,1-dioxide | | 0.693 |
| 46 | 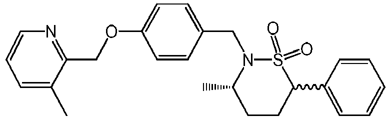 | (3S)-3-Methyl-2-[4-(3-methyl-pyridin-2-ylmethoxy)-benzyl]-6-phenyl-[1,2]thiazinane 1,1-dioxide | | 0.153 |
| 47 | 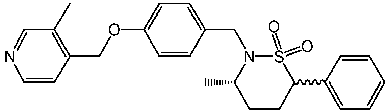 | (3S)-3-Methyl-2-[4-(3-methyl-pyridin-4-ylmethoxy)-benzyl]-6-phenyl-[1,2]thiazinane 1,1-dioxide | | 0.205 |
| 48 | 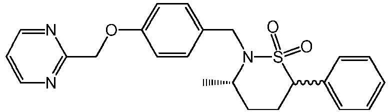 | (3S)-3-Methyl-6-phenyl-2-[4-(pyrimidin-2-ylmethoxy)-benzyl]-[1,2]thiazinane 1,1-dioxide | | 1.63 |
| 49 | 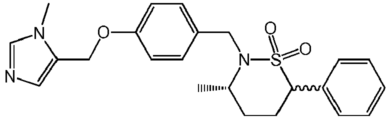 | (3S)-3-Methyl-2-[4-(3-methyl-3H-imidazol-4-ylmethoxy)-benzyl]-6-phenyl-[1,2]thiazinane 1,1-dioxide | | 0.295 |
| 50 | 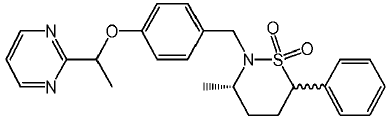 | (3S)-3-Methyl-6-phenyl-2-[4-(1-pyrimidin-2-yl-ethoxy)-benzyl]-[1,2]thiazinane 1,1-dioxide | | 1.35 |
| 51 | 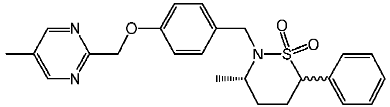 | (3S)-3-Methyl-2-[4-(5-methyl-pyrimidin-2-ylmethoxy)-benzyl]-6-phenyl-[1,2]thiazinane 1,1-dioxide | | 1.18 |
| 52 | 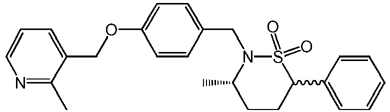 | (3S)-3-Methyl-2-[4-(2-methyl-pyridin-3-ylmethoxy)-benzyl]-6-phenyl-[1,2]thiazinane 1,1-dioxide | (DMSO-*d*6) δ 8.40 (dd, *J* = 4.9, 1.7 Hz, 1H), 7.78 (dd, *J* = 7.7, 1.7 Hz, 1H), 7.50 - 7.43 (m, 2H), 7.43 -7.35 (m, 3H), 7.35 - 7.28 (m, 2H), 7.27 - 7.19 (m, 1H), 7.07 - 6.96 (m, 2H), 5.11 (s, 2H), 4.48 (d, *J* = 16.5 Hz, 1H), 4.38 (dd, *J* = 12.8, 3.6 Hz, 1H), 4.25 (d, *J* = 16.6 Hz, 1H), 4.14 - 4.01 (m, 1H), 2.48 - 2.36 (m, 1H), 2.10 (dq, *J* = 13.9, 3.5 Hz, 1H), 1.89 - 1.73 (m, 1H), 1.70 - 1.59 (m, 1H), 1.09 (d, *J* = 6.9 Hz, 3H). | 0.057 |
| 53 | 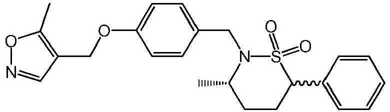 | (3S)-3-Methyl-2-[4-(5-methyl-isoxazol-4-ylmethoxy)-benzyl]-6-phenyl-[1,2]thiazinane 1,1-dioxide | | 0.208 |
| 54 | 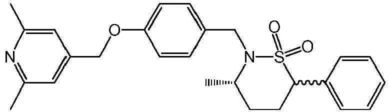 | (3S)-2-[4-(2,6-Dimethyl-pyridin-4-ylmethoxy)-benzyl]-3-methyl-6-phenyl-[1,2]thiazinane 1,1-dioxide | | 0.295 |
| 55 | 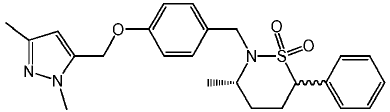 | (3S)-2-[4-(2,5-Dimethyl-2H-pyrazol-3-ylmethoxy)-benzyl]-3-methyl-6-phenyl-[1,2]thiazinane 1,1-dioxide | | 0.387 |
| 56 | 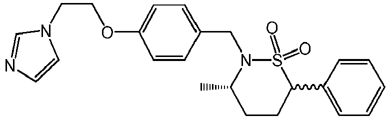 | (3S)-2-[4-(2-Imidazol-1-yl-ethoxy)-benzyl]-3-methyl-6-phenyl-[1,2]thiazinane 1,1-dioxide | | 0.316 |
| 57 | 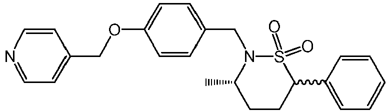 | (3S)-3-Methyl-6-phenyl-2-[4-(pyridin-4-ylmethoxy)-benzyl]-[1,2]thiazinane 1,1-dioxide | | 0.114 |
| 58 | 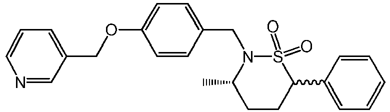 | (3S)-3-Methyl-6-phenyl-2-[4-(pyridin-3-ylmethoxy)-benzyl]-[1,2]thiazinane 1,1-dioxide | | 0.106 |
| 59 | 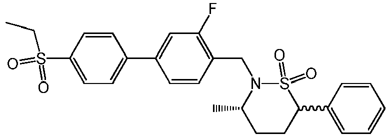 | (3S)-2-(4'-Ethanesulfonyl-3-fluoro-biphenyl-4-ylmethyl)-3-methyl-6-phenyl-[1,2]thiazinane 1,1-dioxide | (DMSO) δ 8.03 - 7.98 (m, 2H), 7.98 - 7.93 (m, 2H), 7.69 - 7.62 (m, 3H), 7.51 - 7.45 (m, 2H), 7.44 - 7.34 (m, 3H), 4.65 - 4.53 (m, 2H), 4.52 - 4.41 (m, 1H), 4.22 - 4.11 (m, 1H), 3.39 - 3.31 (m, 2H), 2.47 - 2.40 (m, 1H), 2.17 - 2.07 (m, 1H), 1.91 - 1.77 (m, 1H), 1.72 - 1.62 (m, 1H), 1.19 - 1.08 (m, 6H). | 0.069 |
| 60 | 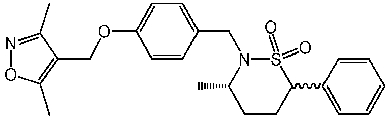 | (3S)-2-[4-(3,5-Dimethyl-isoxazol-4-ylmethoxy)-benzyl]-3-methyl-6-phenyl-[1,2]thiazinane 1,1-dioxide | | 0.151 |
| 61 | 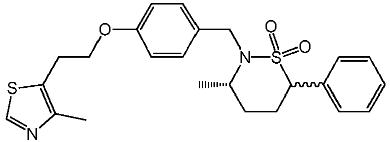 | (3S)-3-Methyl-2-{4-[2-(4-methyl-thiazol-5-yl)-ethoxy]-benzyl}-6-phenyl-[1,2]thiazinane 1,1-dioxide | | 0.151 |

### Example 68 In Vitro RORc Ligand Binding Assay

This assay was used to determine a compound's potency in inhibiting activity of RORc by determining, Kiₐₚₚ, IC₅₀, or percent inhibition values. Consumables used in this Example are shown in Table 5 below.

**Table 5**

| **Consumable** | **Supplier and product code** |
|---|---|
| GFB Unifilter plates | Perkin Elmer 6005177 |
| 3-[(3-Cholamidopropyl)dimethylamm onio] -1 -propanesulfonate (CHAPS) | Sigma C5070 |
| 96-well polypropylene U-bottom assay plate | Nunc 267245 |
| HEPES buffer, 1 M | Sigma H3375 |
| Magnesium chloride (MgCl₂) | Sigma M8266 |
| D,L-Dithiothreitol (DTT) | Sigma D0632 |
| Sodium chloride (NaCl) | Sigma 71382 |
| Bovine serum albumin (BSA) | Sigma A7030 [lyophilized powder, ≥98% (agarose gel electrophoresis), Essentially fatty acid free, essentially globulin free] |
| 25-hydroxycholesterol | Sigma H1015 |
| 25-[26,27-³H]hydroxycholesterol | Perkin Elmer NET674250UC |
| | American Radiolabeled Chemicals ART0766 |
| RORc ligand binding domain | Genentech (e.g., PUR 28048), expressed in *E. coli* |
| Plate seals | Perkin Elmer 6005185 |
| Microscint 0 | Perkin Elmer 6013611 |

### Filter Plate Preparation

On day of the assay, 100 uL of 0.05% CHAPS (in deionized H₂O) was added to all wells of the GFB Unifilter plate and allowed soak for 1 h. A wash buffer of 50 mM HEPES (pH 7.4), 150 mM NaCl, and 5 mM MgCl₂ was prepared to wash the filter plate. To prepare an assay buffer, BSA was added to the wash buffer to reach 0.01% and DTT was added to reach 1 mM.

### Compounds

For IC₅₀ mode, 10 mM compound stocks were serially diluted in DMSO with DMSO to give 20x required final concentration in DMSO (15 uL compound + 30 uL DMSO). The 20x compound stocks were diluted in DMSO with Assay Buffer 4-fold to reach 5x the final test concentration in 25% DMSO (10 uL compound + 30 uL Assay Buffer). Solutions were mixed by aspiration several times with a pipette set on 50 uL volume. For the assay, 10 uL of 5x compound stock solutions in 25% DMSO were added to the assay plate in duplicate.

For two point screening, 10 mM stock compound solutions were diluted in DMSO to obtain 200 uM (20x the high test concentration) and then diluted 10-fold further to reach 20 uM (20x the low test concentration). The 20x stocks were diluted 4-fold with Assay Buffer (10 uL compound + 30 uL Assay Buffer) to reach 5x the test concentrations (50 uM and 5 uM) and 10 uL were added to two assay plates for the duplicate wells. With each concentration tested on 2 plates, each set of 80 compounds used 4 assay plates (1 uM and 10 uM, with n=2).

### Nonspecific binding (NSB) samples, Total Binding (TB) samples and No Receptor (No R) samples

25-hydroxycholesterol (1 uM) was used to determine the level of NSB signal is prepared in DMSO as for compounds above, then diluted in Assay Buffer to give a final concentration of 5 uM. For 25-hydroxycholesterol in 25% DMSO/75% Assay Buffer; 10 uL per well was used for NSB samples. Wells for Total Binding and No Receptor sample determination contained 10 uL of 25% DMSO/75% Assay Buffer per well.

### Radioligand (25-[³H]hydroxycholesterol) Preparation

25-[³H]hydroxycholesterol was dilute in Assay Buffer to obtain 15 nM and vortex to mix. Add 20 uL to all wells to reach 6 nM final in the assay.

### Receptor Preparation

The optimal concentration for RORc receptor was found to be 0.6 ug/mL. Stock receptor solution was diluted in assay buffer to obtain 1.5 ug/mL in Assay Buffer. 20 uL was added to all wells. For No R samples, 20 uL Assay Buffer was substituted for receptor solution.

### Sample addition to Plates and Incubation

Assay plates were 96-well polypropylene V-bottom plates. 10 uL of 5x compound in 25% DMSO/75% Assay Buffer was added to Test wells. 10 uL of 25% DMSO/75% Assay Buffer was added to Total Binding or No Receptor wells. 10 uL of 5 uM 25-hydroxycholesterol in 25% DMSO/75% Assay Buffer was added to NSB wells. 20 uL of 15 nM 25-[³H]hydroxycholesterol prepared in Assay Buffer was added to all wells. 20 uL of 1.5 ug/mL RORc receptor was added to wells (or 40 uL Assay Buffer to No R wells). Following addition to the wells, the plates were incubated 3 h at 25°C.

### Filtration

Using a Packard Filtermate Harvester, the filter plate were washed 4 times following transfer of the incubated samples. Plates were dry-filtered completely (2 h at 50 °C or overnight at room temperature). 50 uL Microscint 0 was added to all wells and read on Topcount protocol Inverted.

### Final concentrations

Final concentrations were as follows: 50 mM HEPES buffer (pH 7.4); 150 mM NaCl; 1 mM DTT; 5 mM MgCl_{2;} 0.01% BSA; 5% DMSO; 0.6 ug/mL RORc receptor; 6 nM 25-[³H]hydroxycholesterol. For NSB wells, 1 uM 25-hydroxycholesterol was also present.

### Example 9 Arthritis Mouse Model

8 to 10-week old male DBA/1 (DBA/1OlaHsd, Harlan Laboratories) mice are housed in a specific pathogen free (SPF) animal facility. Arthritis is induced by two injections of collagen subcutaneously in the base of the tail. The initial injection (on day 0) uses bovine type II collagen (2 mg/ml from Chondrex, Redmond, Wash.) emulsified in equal volume of CFA containing 4 mg/ml of M. tuberculosis (Chondrex). The CII booster injection on Day 29 is emulsified in incomplete Freund's adjuvant (IFA). Each animal receives 0.1 ml of emulsion by subcutaneous/intradermal injection in the tail 2 to 3 cm from the body of the mouse. The booster injection site is in the vicinity of but different from the initial injection site and closer to the body of the animal. OR-1050 was formulated in HRC-6 as above. On weekdays, the animals received two doses (a.m. and p.m.) of HRC-6 or 50 mg/kg OR-1050 p.o. (2.5 mls/kg). On weekends, a single dose of 100 mg/kg was administered (5 mls/kg).

The mice were observed daily for clinical symptoms of CIA based on the following qualitative scale. Each paw was examined individually and scored. Grade 0, normal; grade 1, mild but definite redness and swelling of the ankle or wrist, or apparent redness and swelling limited to individual digits, regardless of the number of affected digits; grade 2, moderate redness and swelling of ankle or wrist; grade 3, severe redness and swelling of the entire paw including digits; grade 4, maximally inflamed limb with involvement of multiple joints. To estimate cumulative disease severity for each animal, an area under the curve score was calculated for each animal by totaling the sum of the daily hind paw measurements betweens days 24 and 48. While the present invention has been described with reference to the specific embodiments thereof, it should be understood by those skilled in the art that various changes may be made and equivalents may be substituted without departing from the true scope of the invention which is defined by the claims appended hereto.

## Claims

1. A compound of formula I: 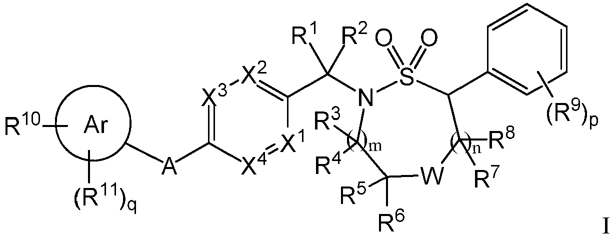 or a pharmaceutically acceptable salt thereof,
wherein:
m is 0 or 1;
n is 0 or 1;
p is from 0 to 3;
q is from 0 to 2;
t is from 0 to 4;
v is 0 or 1,
w is from 0 to 2;
Ar is mono- or bicyclic aryl or heteroaryl;
A is: a bond; -(CR^{j}R^{k})ₜ-; -C(O)-(CR^{j}R^{k})ₜ-; -(CR^{j}R^{k})ₜ-C(O)-; -NR^{a}-(CR^{j}R^{k})ₜ-; -(CR^{j}R^{k})ₜ-NR^{a}-; -C(O)NR^{a}-(CR^{j}R^{k})ₜ-; -(CR^{j}R^{k})ₜ-NR^{a}C(O)-; -O-(CR^{j}R^{k})ₜ; -(CR^{j}R^{k})ₜ-O-; -S-(CR^{j}R^{k})ₜ; -(CR^{j}R^{k})ₜ-S-; -SO₂-(CR^{j}R^{k})ₜ-; or -(CR^{j}R^{k})ₜ-SO₂-,
W is: -CR^{b}R^{c}-; -O-; -S-; -SO₂-; or -NR^{d}-;
one of X¹, X², X³ and X⁴ is N and the others are CR^{e}; or two of X¹, X², X³ and X⁴ are N and the others are CR^{e}; or three of X¹, X², X³ and X⁴ are N and the other is CR^{e}; or each of X¹, X², X³ and X⁴ is CR^{e};
R¹, R², R³, R⁴, R⁵, R⁶, R⁷ and R⁸ each independently is: hydrogen; or C₁₋₆alkyl which may be unsubstituted or substituted one or more times with halo;
or R³ and R⁴ together with the atom to which they are attached may form an ethylene group;
or R³ and R⁴ together with the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
or R⁵ and R⁶ together with the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
or R⁷ and R⁸ together with the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
or one of R³ and R⁴ together with one of R⁵ and R⁶ and the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
or one of R⁵ and R⁶ together with one of R⁷ and R⁸ and the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
each R⁹ is independently: C₁₋₆alkyl; halo; C₁₋₆alkoxy; or cyano; wherein the C₁₋₆alkyl moieties may be unsubstituted or substituted one or more times with halo;
R¹⁰ is: hydrogen; C₁₋₆alkyl; cyano; -(CH₂)ᵥ-NR^{f}R^{g}; -(CH₂)ᵥ-S(O)_{w}-R^{h}; -(CH₂)ᵥ-C(O)-NR^{f}R^{g}; -(CH₂)ᵥ-S(O0)_{w}-NR^{f}R^{g}; -(CH₂)ᵥ-NR^{f}-C(O)-R^{h}; -(CH₂)ᵥ-NR^{f}-C(O)-NR^{f}R^{g}; or -(CH₂)ᵥ-NR^{f}- S(O)_{w}-R^{h};
each R¹¹ is independently: C₁₋₆alkyl; halo; C₁₋₆alkoxy; cyano; halo-C₁₋₆alkyl; hydroxy-C₁₋₆alkyl; halo-C₁₋₆alkoxy; or C₁₋₆alkylsulfonyl;
R^{a}, R^{b}, R^{c}, R^{d} each independent is: hydrogen; or C₁₋₆alkyl which may be unsubstituted or substituted one or more times with halo;
or R^{b} and R^{c} together with the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
or one of R^{b} and R^{c} together with one of R⁷ and R⁸ and the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
or one of R^{b} and R^{c} together with one of R⁵ and R⁶ and the atoms to which they are attached may form a three, four, five, six or seven membered saturated or partially saturated ring that may optionally include one or two heteroatoms selected from -O-, -NR^{a}- or -S-, and which may be optionally substituted one or more times with Rⁱ;
each R^{e} is independently: hydrogen; C₁₋₆alkyl; halo; C₁₋₆alkoxy; or cyano; wherein the C₁₋₆alkyl moieties may be unsubstituted or substituted one or more times with halo;
R^{f} and R^{g} each independently is: hydrogen; or C₁₋₆alkyl which may be unsubstituted or substituted one or more times with halo;
R^{h} is: C₁₋₆alkyl; C₃₋₆cycloalkyl; or C₃₋₆cycloalkyl-C₁₋₆alkyl, each of which may be unsubstituted or substituted one or more times with halo;
Rⁱ is: C₁₋₆alkyl; halo; oxo; hydroxy; acetyl; or C₁₋₆alkoxy; wherein the C₁₋₆alkyl moieties may be unsubstituted or substituted one or more times with halo; and
R^{j} and R^{k} each independent is: hydrogen; or C₁₋₆alkyl which may be unsubstituted or substituted one or more times with halo;
provided that when Ar is imidazolyl substituted with methyl, A is a bond, and each of X¹, X², X³ and X⁴ is CR^{e}, then R^{e} is not methoxy.

2. The compound of claim 1, wherein m is 1.

3. The compound according to any one of claims 1 to 2, wherein n is 0.

4. The compound according to any one of claims 1 to 3, wherein Ar is: phenyl; imidazolyl; pyrazolyl; isoxazolyl; oxazolyl; thiazolyl; isothizaolyl; oxadiazolyl; thiadiazolyl; triazolyl; tetrazolyl; pyridinyl; pyrimidinyl; pyridazinyl; pyrazinyl; indolyl; indazolyl; or [1,2,4]triazolo[4,3-a]pyridinyl.

5. The compound according to any one of claims 1 to 4, wherein Ar is: phenyl; imidazol-1-yl; imidazol-2-yl; imidazol-4-yl; pyrazol-3-yl; pyrazol-4-yl; isoxazol-3-yl; isoxazol-4-yl; isoxazol-5-yl; oxazol-2-yl; thiazol-5-yl; [1,2,4]oxadiazol-3-yl; [1,2,4]triazol-3-yl; pyridin-2-yl; pyridin-3-yl; pyridin-4-yl; pyrimidin-5-yl; pyridazinyl; or [1,2,4]triazolo[4,3-a]pyridin-5-yl.

6. The compound according to any one of claims 1 to 3, wherein A is: a bond; -(CR^{j}R^{k})ₜ-NR^{a}C(O)-; -(CR^{j}R^{k})ₜ-O-; or -C(O)NR^{a}-(CR^{j}R^{k})ₜ.

7. The compound according to any one of claims 1 to 6, wherein W is -CR^{b}R^{c}-.

8. The compound according to any one of claims 1 to 7, wherein X¹, X², X³ and X⁴ are CR^{e}.

9. The compound according to any one of claims 1 to 8, wherein R¹, R², R³, R⁴, R⁵, R⁶, R⁷, and R⁸ are hydrogen.

10. The compoundaccording to any one of claims 1 to 9, wherein R³ is methyl, and R¹, R², R⁴, R⁵, R⁶, R⁷, and R⁸ are hydrogen.

11. The compound according to any one of claims 1 to 10, wherein R¹⁰ is: hydrogen; C₁₋₆alkyl; -SO₂-NH₂; -SO₂-CH₃; cyano; -C(O)-NH₂; -CH₂-C(O)-NH₂; -CH₂-NH-C(O)-CH₃; -C(O)-NH-CH₃; -C(O)-N(CH₃)₂; or -NH-SO₂-CH₃.

12. The compound of claim 1, wherein the compound is of formula IXa or IXb: 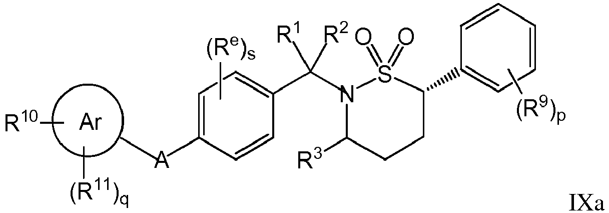 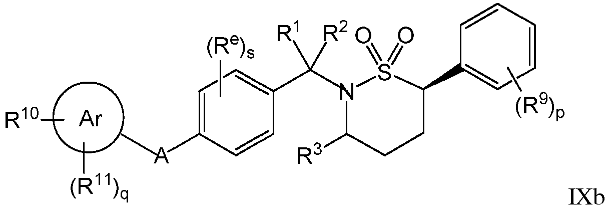 wherein s is from 0 to 3.

13. A composition comprising:
(a) a pharmaceutically acceptable carrier; and
(b) a compound according to any one of claims 1 to 12.

14. A compound according to any one of claims 1 to 12 for use as therapeutically active substance.

15. A compound according to any one of claims 1 to 12 for the treatment or prophylaxis of arthritis.

## Patentansprüche

1. Eine Verbindung der Formel I: 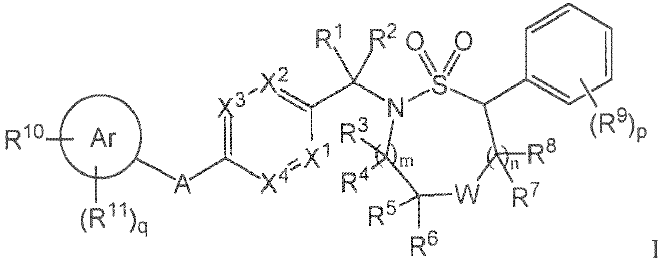 oder ein pharmazeutisch verträgliches Salz davon,
wobei:
m 0 oder 1 ist;
n 0 oder 1 ist;
p von 0 bis 3 ist;
q von 0 bis 2 ist;
t von 0 bis 4 ist;
v 0 oder 1 ist;
w von 0 bis 2 ist;
Ar mono- oder bicyclisches Aryl oder Heteroaryl ist;
A: eine Bindung; -(CR^{j}R^{k})ₜ-; -C(O)-(CR^{j}R^{k})ₜ-; -(CR^{j}R^{k})ₜ-C(O)-; -NR^{a}-(CR^{j}R^{k})ₜ-; -(CR^{j}R^{k}NR^{a}-; -C(O)NR^{a}-(CR^{j}R^{k})ₜ-; -(CR^{j}R^{k})ₜ-NR^{a}C(O)-; -O-(CR^{j}R^{k})ₜ-; -(CR^{j}R^{k})ₜ-O-; -S-(CR^{j}R^{k})ₜ-, -(CR^{j}R)ₜ-S-; -SO₂-(CR^{j}R^{k})ₜ-; oder -(CR^{j}R^{k})ₜ-SO₂- ist;
W: -CR^{b}R^{c}-; -O-; -S-; -SO₂-; oder -NR^{d}- ist;
eines von X¹, X², X³ und X⁴ gleich N ist und die anderen CR^{c} sind; oder zwei von X¹, X², X³ und X⁴ gleich N sind und die anderen CR^{e} sind; oder drei von X¹, X², X³ und X⁴ gleich N sind und das andere CR^{e} ist; oder jedes von X¹, X², X³ und X⁴ gleich CR^{e} ist;
R¹, R², R³, R⁴, R⁵, R⁶, R⁷ und R⁸ jeweils unabhängig: Wasserstoff; oder C₁₋₆Alkyl ist, welches unsubstituiert oder ein- oder mehrmals mit einem Halogen substituiert sein kann;
oder R³ und R⁴ zusammen mit dem Atom, an welches sie gebunden sind, eine Ethylengruppe bilden können;
oder R³ und R⁴ zusammen mit dem Atom, an welches sie gebunden sind, einen drei-, vier-, fünf-, sechs- oder sieben-gliedrigen gesättigten oder teilweise gesättigten Ring bilden können, der gegebenenfalls ein oder zwei Heteroatome ausgewählt aus -O-, -NR^{a}- oder -S- beinhalten kann, und welcher gegebenenfalls ein- oder mehrmals mit Rⁱ substituiert sein kann;
oder R⁵ und R⁶ zusammen mit dem Atom, an welches sie gebunden sind, einen drei-, vier-, fünf-, sechs- oder sieben-gliedrigen gesättigten oder teilweise gesättigten Ring bilden können, der gegebenenfalls ein oder zwei Heteroatome ausgewählt aus -O-, -NR^{a}- oder -S- beinhalten kann, und welcher gegebenenfalls ein- oder mehrmals mit Rⁱ substituiert sein kann;
oder R⁷ und R⁸ zusammen mit dem Atom, an welches sie gebunden sind, einen drei-, vier-, fünf-, sechs- oder sieben-gliedrigen gesättigten oder teilweise gesättigten Ring bilden können, der gegebenenfalls ein oder zwei Heteroatome ausgewählt aus -O-, -NR^{a}- oder -S- beinhalten kann, und welcher gegebenenfalls ein- oder mehrmals mit Rⁱ substituiert sein kann;
oder eines von R³ und R⁴ zusammen mit einem von R⁵ und R⁶ und den Atomen, an welche sie gebunden sind, einen drei-, vier-, fünf-, sechs- oder sieben-gliedrigen gesättigten oder teilweise gesättigten Ring bilden kann, der gegebenenfalls ein oder zwei Heteroatome ausgewählt aus -O-, -NR^{a}- oder -S- beinhalten kann, und welcher gegebenenfalls ein- oder mehrmals mit Rⁱ substituiert sein kann;
oder eines von R⁵ und R⁶ zusammen mit einem von R⁷ und R⁸ und den Atomen, an welche sie gebunden sind, einen drei-, vier-, fünf-, sechs- oder sieben-gliedrigen gesättigten oder teilweise gesättigten Ring bilden kann, der gegebenenfalls ein oder zwei Heteroatome ausgewählt aus -O-, -NR^{a}- oder -S- beinhalten kann, und welcher gegebenenfalls ein- oder mehrmals mit Rⁱ substituiert sein kann;
jedes R⁹ unabhängig: C₁₋₆Alkyl; Halogen; C₁₋₆Alkoxy; oder Cyano ist; wobei die C₁₋₆Alkyl-Einheiten unsubstituiert oder ein- oder mehrmals mit einem Halogen substituiert sein können;
R¹⁰: Wasserstoff; C₁₋₆Alkyl; Cyano; -(CH₂)ᵥ-NR^{f}R^{g}; -(CH₂)ᵥ-S(O)_{w}-R^{h}; -(CH₂)ᵥ-C(O)-NR^{f}R^{g}; -(CH₂)ᵥ-S(O)_{w}-NR^{f}R^{g}; -(CH₂)ᵥ-NR^{f}-C(O)-R^{h}; -(CH₂)ᵥ-NR^{f}-C(O)-NR^{f}R^{g} oder -(CH₂)ᵥ-NR^{f}-S(O)_{w}-R^{h} ist;
jedes R¹¹ unabhängig: C₁₋₆Alkyl; Halogen; C₁₋₆Alkoxy; Cyano; Halogen-C₁₋₆Alkyl; Hydroxy-C₁₋₆Alkyl; Halogen-C₁₋₆Alkoxy; oder C₁₋₆Alkylsulfonyl ist;
R^{a}, R^{b}, R^{c}, R^{d} jeweils unabhängig: Wasserstoff; oder C₁₋₆Alkyl, welches unsubstituiert oder ein- oder mehrmals mit einem Halogen substituiert sein kann, ist;
oder R^{b} und R^{c} zusammen mit dem Atom, an welches sie gebunden sind, einen drei-, vier-, fünf-, sechs- oder sieben-gliedrigen gesättigten oder teilweise gesättigten Ring bilden können, der gegebenenfalls ein oder zwei Heteroatome ausgewählt aus -O-, -NR^{a}- oder -S- beinhalten kann, und welcher gegebenenfalls ein- oder mehrmals mit Rⁱ substituiert sein kann;
oder eines von R^{b} und R^{c} zusammen mit einem von R⁷ und R⁸ und den Atomen, an welche sie gebunden sind, einen drei-, vier-, fünf-, sechs- oder sieben-gliedrigen gesättigten oder teilweise gesättigten Ring bilden kann, der gegebenenfalls ein oder zwei Heteroatome ausgewählt aus -O-, -NR^{a}- oder -S- beinhalten kann, und welcher gegebenenfalls ein- oder mehrmals mit Rⁱ substituiert sein kann;
oder eines von R^{b} und R^{c} zusammen mit einem von R⁵ und R⁶ und den Atomen, an welche sie gebunden sind, einen drei-, vier-, fünf-, sechs- oder sieben-gliedrigen gesättigten oder teilweise gesättigten Ring bilden kann, der gegebenenfalls ein oder zwei Heteroatome ausgewählt aus -O-, -NR^{a}- oder -S- beinhalten kann, und welcher gegebenenfalls ein- oder mehrmals mit Rⁱ substituiert sein kann;
jedes R^{e} unabhängig: Wasserstoff; C₁₋₆Alkyl; Halogen; C₁₋₆Alkoxy; oder Cyano ist; wobei die C₁₋₆Alkyl-Einheiten unsubstituiert oder ein- oder mehrmals mit einem Halogen substituiert sein können;
R^{f} und R^{g} jeweils unabhängig: Wasserstoff; oder C₁₋₆Alkyl ist, welches unsubstituiert oder ein- oder mehrmals mit einem Halogen substituiert sein kann;
R^{h}: C₁₋₆Alkyl; C₃₋₆Cycloalkyl; oder C₃₋₆Cycloalkyl-C₁₋₆alkyl ist, die jeweils unsubstituiert oder ein- oder mehrmals mit einem Halogen substituiert sein können;
Rⁱ: C₁₋₆Alkyl; Halogen; Oxo; Hydroxy; Acetyl; oder C₁₋₆Alkoxy ist; wobei die C₁₋₆Alkyl-Einheiten unsubstituiert oder ein- oder mehrmals mit einem Halogen substituiert sein können; und
R^{j} und R^{k} jeweils unabhängig: Wasserstoff; oder C₁₋₆Alkyl ist, welches unsubstituiert oder ein- oder mehrmals mit einem Halogen substituiert sein kann;
mit der Maßgabe, dass wenn Ar Imidazolyl substituiert mit Methyl ist, A eine Bindung ist, und jedes von X¹, X², X³ und X⁴ gleich CR^{e} ist, dann R^{e} kein Methoxy ist.

2. Die Verbindung nach Anspruch 1, wobei m 1 ist.

3. Die Verbindung nach einem der Ansprüche 1 bis 2, wobei n 0 ist.

4. Die Verbindung nach einem der Ansprüche 1 bis 3, wobei Ar: Phenyl; Imidazolyl; Pyrazolyl; Isoxazolyl; Oxazolyl; Thiazolyl; Isothiazolyl; Oxadiazolyl; Thiadiazolyl; Triazolyl; Tetrazolyl; Pyridinyl; Pyrimidinyl; Pyridazinyl; Pyrazinyl; Indolyl; Indazolyl; oder [1,2,4]Triazolo[4,3-a]pyridinyl ist.

5. Die Verbindung nach einem der Ansprüche 1 bis 4, wobei Ar: Phenyl; Imidazol-1-yl; Imidazol-2-yl; Imidazol-4-yl; Pyrazol-3-yl; Pyrazol-4-yl; Isoxazol-3-yl; Isoxazol-4-yl; Isoxazol-5-yl; Oxazol-2-yl; Thiazol-5-yl; [1,2,4]Oxadiazol-3-yl; [1,2,4]Triazol-3-yl; Pyridin-2-yl; Pyridin-3-yl; Pyridin-4-yl; Pyrimidin-5-yl; Pyridazinyl; oder [1,2,4]Triazolo[4,3-a]pyridin-5-yl ist.

6. Die Verbindung nach einem der Ansprüche 1 bis 3, wobei A: eine Bindung; -(CR^{j}R^{k})ₜ-NR^{a}C(O)-; -(CRⁱR^{k})ₜ-O-; oder -C(O)NR^{a}-(CR^{j}R^{k})ₜ- ist.

7. Die Verbindung nach einem der Ansprüche 1 bis 6, wobei W gleich -CR^{b}R^{c}- ist.

8. Die Verbindung nach einem der Ansprüche 1 bis 7, wobei X¹, X², X³ und X⁴ gleich CR^{e} sind.

9. Die Verbindung nach einem der Ansprüche 1 bis 8, wobei R¹, R², R³, R⁴, R⁵, R⁶, R⁷ und R⁸ Wasserstoff sind.

10. Die Verbindung nach einem der Ansprüche 1 bis 9, wobei R³ Methyl ist, und R¹, R², R⁴, R⁵, R⁶, R⁷ und R⁸ Wasserstoff sind.

11. Die Verbindung nach einem der Ansprüche 1 bis 10, wobei R¹⁰: Wasserstoff; C₁₋₆Alkyl; -SO₂-NH₂; -SO₂-CH₃; Cyano; -C(O)-NH₂; -CH₂-C(O)-NH₂; -CH₂-NH-C(O)-CH₃; -C(O)-NH-CH₃; -C(O)-N(CH₃)₂; oder -NH-SO₂-CH₃ ist.

12. Die Verbindung nach Anspruch 1, wobei die Verbindung der Formel IXa oder IXb entspricht: 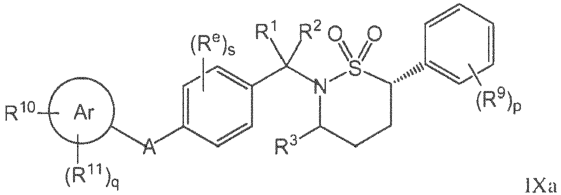 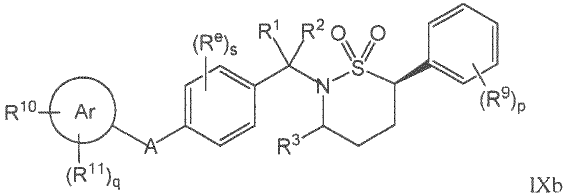 wobei s von 0 bis 3 ist.

13. Eine Zusammensetzung umfassend:
(a) einen pharmazeutisch verträglichen Träger; und
(b) eine Verbindung nach einem der Ansprüche 1 bis 12.

14. Eine Verbindung nach einem der Ansprüche 1 bis 12 zur Verwendung als therapeutisch wirksame Substanz.

15. Eine Verbindung nach einem der Ansprüche 1 bis 12 zur Behandlung oder Prophylaxe von Arthritis.

## Revendications

1. Composé de formule I : 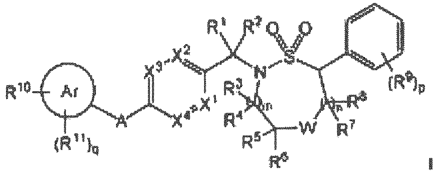 ou un de ses sels pharmaceutiquement acceptables,
dans laquelle :
m vaut 0 ou 1 ;
n vaut 0 ou 1 ;
p vaut de 0 à 3 ;
q vaut de 0 à 2 ;
t vaut de 0 à 4 ;
v vaut 0 ou 1 ;
w vaut de 0 à 2 ;
Ar est un groupe aryle ou hétéroaryle mono- ou bicyclique ;
A est : une liaison ; -(CR^{j}R^{k})ₜ- ; -C(O)(CR^{j}R^{k})ₜ- ; -(CR^{j}R^{k})ₜ-C(O)- ; -NR^{a}-(CR^{j}R^{k})ₜ- ; -(CR^{j}R^{k})ₜ-NR^{a} ; -C(O)NR^{a}-(CR^{j}R^{k})ₜ- ; -(CR^{j}R^{k})ₜ-NR^{a}C(O)- ; -O-(CR^{j}R^{k})ₜ- ; -(CR^{j}R^{k})ₜ-O- ; -S-(CR^{j}R^{k})ₜ- ; -(CR^{j}R^{k})ₜ-S- ; -SO₂-(CR^{j}R^{k})ₜ- ; (CR^{j}R^{k})ₜ-SO₂- ;
W est : -CR^{b}R^{c} ; -O- ; -S- ; -SO₂- ; ou -NR^{d}- ;
un de X¹, X², X³ et X⁴ est N et les autres sont CR^{e} ; ou deux de X¹, X², X³ et X⁴ sont N et les autres sont CR^{e} ; ou trois de X¹, X², X³ et X⁴ sont N et l'autre est CR^{e} ; ou chacun de X¹, X², X³ et X⁴ est CR^{e} ;
R¹, R², R³, R⁴, R⁵, R⁶, R⁷ et R⁸ sont chacun indépendamment : un hydrogène ; ou un groupe alkyle en C₁ à C₆ qui peut être non substitué ou substitué une ou plusieurs fois avec un halogéne ;
ou R³ et R⁴ conjointement avec l'atome auquel ils sont attachés, peuvent former un groupe éthylène ;
ou R³ et R⁴ conjointement avec les atomes auxquels ils sont attachés, peuvent former un noyau saturé ou partiellement saturé tri-, tétra-, penta-, hexa- ou heptagonal qui peut éventuellement comprendre un ou deux hétéroatomes choisis parmi -O-, -NR^{a}- ou -S-, et qui peut être éventuellement substitué une ou plusieurs fois avec Rⁱ ;
ou R⁵ et R⁶ conjointement avec les atomes auxquels ils sont attachés, peuvent former un noyau saturé ou partiellement saturé tri-, tétra-, penta-, hexa- ou heptagonal qui peut éventuellement comprendre un ou deux hétéroatomes choisis parmi -O-, -NR^{a}- ou -S-, et qui peut être éventuellement substitué une ou plusieurs fois avec Rⁱ ;
ou R⁷ et R⁸ conjointement avec les atomes auxquels ils sont attachés, peuvent former un noyau saturé ou partiellement saturé tri-, tétra-, penta-, hexa- ou heptagonal qui peut éventuellement comprendre un ou deux hétéroatomes choisis parmi -O-, -NR^{a}- ou -S-, et qui peut être éventuellement substitué une ou plusieurs fois avec Rⁱ;
ou un de R³ et R⁴ conjointement avec un de R⁵ et R⁶ et les atomes auxquels ils sont attachés, peuvent former un noyau saturé ou partiellement saturé tri-, tétra, penta-, hexa- ou heptagonal qui peut éventuellement comprendre un ou deux hétéroatomes choisis parmi -O-, -NR^{a}- ou -S-, et qui peut être éventuellement substitué une ou plusieurs fois avec Rⁱ ;
ou un de R⁵ et R⁶ conjointement avec un de R⁷ et R⁸ et les atomes auxquels ils sont attachés, peuvent former un noyau saturé ou partiellement saturé tri-, tétra-, penta-, hexa- ou heptagonal qui peut éventuellement comprendre un ou deux hétéroatomes choisis parmi -O-, -NR^{a}- ou -S-, et qui peut être éventuellement substitué une ou plusieurs fois avec Rⁱ ;
chaque R⁹ est indépendamment : un groupe alkyle en C₁ à C₆ ; halogéno ; groupe alkoxy en C₁ à C₆ ; ou groupe cyano ; où les groupements alkyle en C₁ à C₆ peuvent être non substitués ou substitués une ou plusieurs fois avec un halogéne ;
R¹⁰ est : un hydrogène ; un groupe alkyle en C₁ à C₆ ; cyano; -(CH₂)ᵥ-NR^{f}R^{g}; -(CH₂)ᵥ-S(O)_{w}-R^{h}; -(CH2)ᵥ-C(O)-NR^{f}R^{g} ; -(CH₂)ᵥ-S(O)_{w}-VR^{f}R^{g} ; -(CH₂)ᵥ-NR^{f}-C(O)-R^{h}- . -(CH₂)ᵥ-NR^{f}-C(O)-NR^{f}R^{g} ; ou -(CH₂)ᵥ-NR^{f}-S(O)_{w}-R^{h} ;
chaque R¹¹ est indépendamment : un groupe alkyle en C₁ à C₆ ; halogéne ; alkoxy en C₁ à C₆ ; cyano ; halogéno-alkyle en C₁ à C₆ ; hydroxyalkyl en C₁ à C₆ ; halogéno-alkoxy en C₁ à C₆ ; ou alkylsulfonyle en C₁ à C₆ ;
R^{a}, R^{b}, R^{c} et R^{d} sont chacun indépendamment : un hydrogène ; ou un groupe alkyle en C₁ à C₆ qui peut être non substitué ou substitué une ou plusieurs fois avec un halogéne ;
ou R^{b} et R^{c} conjointement avec les atomes auxquels ils sont attachés, peuvent former un noyau saturé ou partiellement saturé tri-, tétra-, penta-, hexa- ou heptagonal qui peut éventuellement comprendre un ou deux hétéroatomes choisis parmi -O-, -NR^{a}- ou -S-, et qui peut être éventuellement substitué une ou plusieurs fois avec Rⁱ ;
ou un de R^{b} et R^{c} conjointement avec un de R⁷ et R⁸ et les atomes auxquels ils sont attachés, peuvent former un noyau saturé ou partiellement saturé tri-, téta-, penta-, hexa- ou heptagonal qui peut éventuellement comprendre un ou deux hétéroatomes choisis parmi -O-, -NR^{a}- ou -S-, et qui peut être éventuellement substitué une ou plusieurs fois avec Rⁱ ;
ou un de R^{b} et R^{c} conjointement avec un de R⁵ et R⁶ et les atomes auxquels ils sont attachés, peuvent former un noyau saturé ou partiellement saturé tri-, tétra-, penta-, hexa- ou heptagonal qui peut éventuellement comprendre un ou deux hétéroatomes choisis parmi -O-, -NR^{a}- ou -S-, et qui peut être éventuellement substitué une ou plusieurs fois avec Rⁱ ;
chaque R^{e} est indépendamment : un hydrogène ; un groupe alkyle en C₁ à C₆ ; halogéno ; alkoxy en C₁ à C₆ ; ou cyano ; où les groupements alkyle en C₁ à C₆ peuvent être non substitués ou substitués une ou plusieurs fois avec un halogéno ;
R^{f} et R^{g} sont chacun indépendamment : un hydrogène ; ou un groupe alkyle en C₁ à C₆ qui peut être non substitué ou substitué une ou plusieurs fois avec un halogéne ;
R^{h} est est : un groupe alkyle en C₁ à C₆ ; cycloalkyle en C₃ à C₆ ; ou cycloalkyle en C₃ à C₆-alkyle en C₁ à C₆, dont chacun peut être non substitué ou substitué une ou plusieurs fois avec un halogéno ;
R¹ est : un groupe alkyle en C₁ à C₆ ; halogéno ; oxo ; hydroxy ; acétyle ; ou alkoxy en C₁ à C₆ ; où les groupements alkyle en C₁ à C₆ peuvent être non substitués ou substitués une ou plusieurs fois avec un halogéno ; et
R^{j} et R^{k} sont indépendamment : un hydrogène ; ou un groupe alkyle en C₁, à C₆ qui peut être non substitué ou substitué une ou plusieurs fois avec un halogéne ;
pourvu que, quand Ar est un groupe imidazolyle substitué avec un groupe méthyle, A est une liaison, et chacun de X¹, X², X³ et X⁴ est CR^{e}, alors R^{e} n'est pas un groupe méthoxy.

2. Composé selon la revendication 1, dans lequel m. vaut 1.

3. Composé selon l'une quelconque des revendications 1 et 2, dans lequel n vaut 0.

4. Composé selon l'une quelconque des revendications 1 à 3, dans lequel Ar est un groupe : phényle ; imidazolyle ; pyrazolyle ; isoxazolyle ; oxazolyle ; thiazolyle ; isothiazolyle ; oxadiazolyle ; thiadiazolyle ; triazolyle ; tétrazolyle ; pyridinyle ; pyrimidinyle ; pyrïdazinyle ; pyrazinyle ; indolyle ; indazolyle ; ou [1,2,4]triazolo--[4,3-a]pyridinyle.

5. Composé selon l'une quelconque des revendications 1 à 4, dans lequel Ar est un groupe : phényle ; imidazole-1-yle ; imidazole-2--yle ; imidazole-4-yle ; pyrazole-3-yle ; pyrazole-4-yle ; isoxazole-3-yle ; isoxazole-4-yle ; isoxazole-5-yle ; oxazole-2-yle ; thiazole-5-yle ; [1,2,4]-oxadiazole-3-yle ; [1,2,4]triazole-3-yle ; pyridine-2-yle ; pyridine-3-yle ; pyridine-4-yle ; pyrimidine-5-yle ; pyridazinyle ; oui [1,2,4]triazolo[4,3-a]pyridine-5-yle.

6. Composé selon l'une quelconque des revendications 1 à 3, dans lequel A est : une liaison ; -(CR^{j}R^{k})ₜ-NR^{a}C(O)- ; -(CR^{j}R^{k})_{c}-O- ; ou -C(O)NR^{a}-(CR^{j}R^{k})ₜ.

7. Composé selon l'une quelconque des revendications 1 à 6, dans lequel W est -CR^{b}R^{c}-.

8. Composé selon l'une quelconque des revendications 1 à 7, dans lequel X¹, X², X³ et X⁴ sont CR^{e}.

9. Composé selon l'une quelconque des revendications 1 à 8, dans lequel R¹, R², R³, R⁴, R⁵, R⁶, R⁷ et R⁸ sont un hydrogène.

10. Composé selon l'une quelconque des revendications 1 à 9, dans lequel R³ est un groupe méthyle, et R¹, R², R⁴, R⁵, R⁶, R⁷ et R⁸ sont un hydrogène.

11. Composé selon l'une quelconque des revendications 1 à 10, dans lequel R¹⁰ est : un hydrogène ; un groupe allyle en C₁ à C₆ ; -SO₂-NH₂ ; -SO₂-CH₃ ; cyano ; -C(O)-NH₂ ; -CH₂-C(O)-NH₂ ; -CH₂-NH-C(O)-CH₃ ; -C(O)-NH-CH₃; -C(O)-N(CH₃)₂ ; ou -NH-SO₂-CH₃.

12. Composé selon la revendication 1, le composé étant de formule IXa ou IXb : 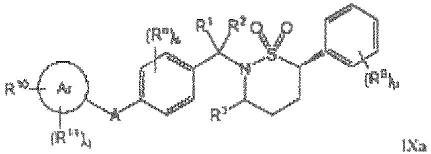 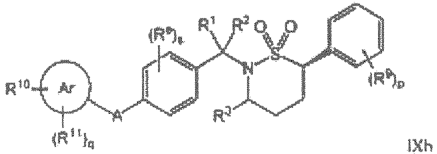 dans lesquelles s vaut de 0 à 3.

13. Composition comprenant :
(a) un véhicule pharmaceutiquement acceptable ; et
(b) un composé selon l'une quelconque des revendications 1 à 12.

14. Composé selon l'une quelconque des revendications 1 à 12 pour une utilisation comme substance thérapeutiquement active.

15. Composé selon l'une quelconque des revendications 1 à 12 pour le traitement ou à prophylaxie de l'arthrite.
